# EUROPEAN PATENT SPECIFICATION

(11) **EP 3 177 607 B1**
(45) Date of publication and mention of the grant of the patent: **26.09.2018**
(21) Application number: 15747158.2
(22) Date of filing: 05.08.2015
(51) Int. Cl.: C07D 401/14, C07D 231/12, C07D 403/04, A01N 43/56

(54) **HALOGEN-SUBSTITUTED BIPYRAZOLE COMPOUNDS FOR CONTROLLING ANIMAL PESTS**
HALOGENSUBSTITUIERTE VERBINDUNGEN UND DEREN VERWENDUNG ZUR KONTROLLE TIERISCHER PEST
COMPOSÉS À SUBSTITUTION HALOGÈNE ET LEUR UTILISATION POUR COMBATTRE DES ANIMAUX PARASITES

(30) Priority: 08.08.2014 EP 14180336
(43) Date of publication of application: 14.06.2017
(73) Proprietor: Bayer CropScience Aktiengesellschaft, 40789 Monheim am Rhein (DE)
(72) Inventor: MAUE, Michael, 40764 Langenfeld (DE); HARSCHNECK, Tobias, 40225 Düsseldorf (DE); FISCHER, Reiner, 40789 Monheim (DE); HAHN, Julia Johanna, 40589 Düsseldorf (DE); DÉCOR, Anne, 40764 Langenfeld (DE); HALLENBACH, Werner, 40789 Monheim (DE); SCHWARZ, Hans-Georg, 46282 Dorsten (DE); KÖBBERLING, Johannes, 41466 Neuss (DE); HÜBSCH, Walter, 42113 Wuppertal (DE); BRETSCHNEIDER, Manfred Thomas, Deceased (DE)
(74) Representative: BIP Patents
(86) International application number: PCT/EP2015/068061
(87) International publication number: WO 2016/020441

(56) References cited:
- WO-A1-2012/107434
- WO-A1-2012/126766
- WO-A1-2014/122083
- WO-A2-2010/051926

## Description

### Background

The present application relates to novel halogen-substituted compounds, to processes for their preparation and to their use for controlling animal pests, in particular arthropods and especially insects, arachnids and nematodes.

It is known that certain halogen-substituted compounds have herbicidal action (cf. J. Org. Chem. 1997, 62(17), 5908-5919, J. Heterocycl. Chem. 1998, 35(6), 1493-1499, WO 2004/035545, WO 2004/106324, US 2006/069132, WO 2008/029084).

Furthermore, it is known that certain halogen-substituted compounds have insecticidal action (EP 1 911 751, WO2012/069366, WO2012/080376, WO2012/107434, WO2012/126766 and WO2014/122083).

In addition, it is known that certain halogen-substituted compounds have cytokine-inhibitory activities (WO 2000/07980).

Modern crop protection compositions have to meet many demands, for example in relation to efficacy, persistence and spectrum of their action and possible use. Questions of toxicity, the combinability with other active compounds or formulation auxiliaries play a role, as well as the question of the expense that the synthesis of an active compound requires. Furthermore, resistances may occur. For all these reasons, the search for novel crop protection agents can never be considered as having been concluded, and there is a constant need for novel compounds having properties which, compared to the known compounds, are improved at least in respect of individual aspects.

It was an object of the present invention to provide compounds which widen the spectrum of the pesticides under various aspects and/or improve their activity.

Surprisingly, it has now been found that certain halogen-substituted compounds have biological properties and are particularly suitable for controlling animal pests, and can therefore be employed particularly well in the agrochemical field and in the animal health sector.

Similar compounds are already known from WO 2010/051926.

### Summary

One aspect of the present invention refers to a compounds of the general formula (I), 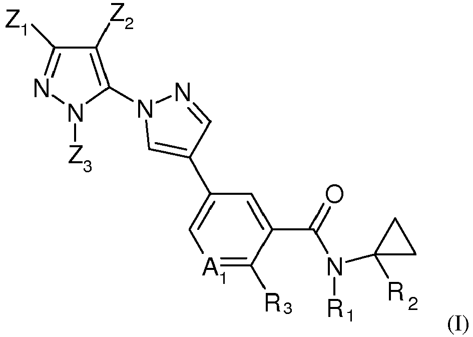 in which
- Z₁: represents 1-halogenated cyclopropyl;
- Z₂: represents -S(O)₀₋₂-(C₁-C₂)-alkyl, (C₁-C₂)-halogenalkyl, or nitro (NO₂);
- Z₃: represents (C₁-C₂)-alkyl;
- R₁: represents hydrogen (H) or (C₁-C₂)-alkyl;
- R₂: represents H or cyano (CN);
- R₃: represents CH₃ or chlorine (Cl);
- A₁: represents CH or nitrogen (N).

The skilled person will understand that all embodiments of the present invention can be combined. Parts of combinations which contradict natural laws are excluded.

In one preferred embodiment, in a compound of formula (I) A₁ is CH (while all other substituents are as defined above).

In another preferred embodiment, in a compound of formula (I) A₁ is CH and R₃ is Cl (while all other substituents are as defined above).

In yet another preferred embodiment, in a compound of formula (I)
Z₁ is 1-fluoro-cyclopropyl or 1-chloro-cyclopropyl;
Z₂ is -S-CH₃, -SO-CH₃, -SO₂-CH₃, CF₃, Cl, Br, I or NO₂;
Z₃ is CH₃ (while all other substituents are as defined above).

In yet another preferred embodiment, in a compound of formula (I)
Z₁ is 1-fluoro-cyclopropyl or 1-chloro-cyclopropyl;
Z₂ is -S-CH₃, Cl, Br, I, CF₃ or NO₂;
Z₃ is CH₃ (while all other substituents are as defined above).

In yet another preferred embodiment, in a compound of formula (I)
Z₁ is 1-fluoro-cyclopropyl or 1-chloro-cyclopropyl;
Z₂ is I, CF₃ or NO₂;
Z₃ is CH₃ (while all other substituents are as defined above).

In yet another preferred embodiment, in a compound of formula (I)
Z₁ is 1-fluoro-cyclopropyl or 1-chloro-cyclopropyl or 1-bromocyclopropyl;
Z₂ is fluorinated methyl (CF₃ CF₂H or CFH₂) or NO₂;
Z₃ is CH₃ (while all other substituents are as defined above).

In yet another preferred embodiment, in a compound of formula (I)
Z₁ is 1-fluoro-cyclopropyl or 1-chloro-cyclopropyl or 1-bromocyclopropyl;
Z₂ is CF₃, CF₂H or NO₂;
Z₃ is CH₃ (while all other substituents are as defined above).

In yet another preferred embodiment, in a compound of formula (I)
Z₁ is 1-fluoro-cyclopropyl or 1-chloro-cyclopropyl or 1-bromocyclopropyl;
Z₂ is CF₂H or NO₂;
Z₃ is CH₃ (while all other substituents are as defined above).

In yet another preferred embodiment, in a compound of formula (I)
Z₁ is 1-fluoro-cyclopropyl or 1-chloro-cyclopropyl or 1-bromocyclopropyl;
Z₂ is CF₃ or NO₂;
Z₃ is CH₃ (while all other substituents are as defined above).

In yet another preferred embodiment, in a compound of formula (I)
Z₁ is 1-fluoro-cyclopropyl or 1-chloro-cyclopropyl or 1-bromocyclopropyl;
Z₂ is S(O)_{0,1} or ₂-CH₃;
Z₃ is CH₃ (while all other substituents are as defined above).

In yet another preferred embodiment, in a compound of formula (I)
Z₁ is 1-fluoro-cyclopropyl or 1-chloro-cyclopropyl or 1-bromocyclopropyl;
Z₂ is S-CH₃;
Z₃ is CH₃ (while all other substituents are as defined above).

In yet another preferred embodiment, in a compound of formula (I)
Z₁ is 1-fluoro-cyclopropyl or 1-chloro-cyclopropyl or 1-bromocyclopropyl;
Z₂ is S(O)₁-CH₃;
Z₃ is CH₃ (while all other substituents are as defined above).

In yet another preferred embodiment, in a compound of formula (I)
Z₁ is 1-fluoro-cyclopropyl or 1-chloro-cyclopropyl or 1-bromocyclopropyl;
Z₂ is S(O)₂-CH₃;
Z₃ is CH₃ (while all other substituents are as defined above).

In yet another preferred embodiment, in a compound of formula (I)
Z₁ is 1-fluoro-cyclopropyl or 1-chloro-cyclopropyl or 1-bromocyclopropyl;
Z₂ is NO₂;
Z₃ is CH₃ (while all other substituents are as defined above).

In yet another preferred embodiment, in a compound of formula (I)
Z₁ is 1-fluoro-cyclopropyl or 1-chloro-cyclopropyl or 1-bromocyclopropyl;
Z₂ is CF₃;
Z₃ is CH₃ (while all other substituents are as defined above).

In yet another preferred embodiment, in a compound of formula (I)
Z₁ is 1-fluoro-cyclopropyl or 1-chloro-cyclopropyl or 1-bromocyclopropyl;
Z₂ is CF₂H;
Z₃ is CH₃ (while all other substituents are as defined above).

In yet another preferred embodiment, in a compound of formula (I) R₁ is H (while all other substituents are as defined above).

In yet another preferred embodiment, in a compound of formula (I) A₁ is N (while Z₁, Z₂, Z₃, R₁, R₂, and R₃ are as defined above).

In yet another preferred embodiment, in a compound of formula (I) R₃ is Cl (while all other substituents are as defined above).

Another aspect refers to the use of a compound of the present invention for controlling insects, arachnids and nematodes.

Yet another aspect refers to a pharmaceutical composition comprising at least one compound of the present invention.

Yet another aspect refers to a compound of te present invention for use as a medicament.

Yet another aspect refers to the use of a compound of the present invention for preparing a pharmaceutical composition for controlling parasites on animals.

Yet another aspect refers to a process for preparing a crop protection composition comprising a compound of the present invention and customary extenders and/or surfactants.

Yet another aspect refers to a compound selected from the group consisting of compounds (10), (11), (12), (13), (14) and (24) as described herein.

Yet another aspect refers to a compound of formula (10).

Yet another aspect refers to a compound of formula (11).

Yet another aspect refers to a compound of formula (12).

Yet another aspect refers to a compound of formula (13).

Yet another aspect refers to a compound of formula (14).

Yet another aspect refers to a compound of formula (24).

Yet another aspect refers to a process for htepreparation of a compound of formula (I) comprising the steps of preparing compounds of formula (10), (11), (12), (13) and (14) as described herein.

Yet another aspect refers to a method for controlling pests, characterized in that a compound of the present invention is allowed to act on the pests and/or their habitat.

Yet another aspect refers to the use of a compound of the present invention for protecting the propagation material of plants.

### Definitions

According to the invention, "alkyl" - on its own or as part of a chemical group - represents straight-chain or branched hydrocarbons preferably having 1 to 6 carbon atoms such as, methyl, ethyl, n-propyl, isopropyl, n-butyl, isobutyl, s-butyl, t-butyl, pentyl, 1-methylbutyl, 2-methylbutyl, 3-methylbutyl, 1,2-dimethylpropyl, 1,1-dimethylpropyl, 2,2-dimethylpropyl, 1-ethylpropyl, hexyl, 1-methylpentyl, 2-methylpentyl, 3-methylpentyl, 4-methylpentyl, 1,2-dimethylpropyl, 1,3-dimethylbutyl, 1,4-dimethylbutyl, 2,3-dimethylbutyl, 1,1-dimethylbutyl, 2,2-dimethylbutyl, 3,3-dimethylbutyl, 1,1,2-trimethylpropyl, 1,2,2-trimethylpropyl, 1-ethylbutyl and 2-ethylbutyl. Preference is furthermore given to alkyl groups having 1 to 2 carbon atoms.

According to the invention, "halogen" or "halo" represents fluorine, chlorine, bromine or iodine, in particular fluorine, chlorine or bromine.

The halogen-substituted chemical groups according to the invention such as, for example, haloalkyl are mono- or polysubstituted by halogen up to the maximum possible number of substituents (perhalogenated). In the case of polysubstitution by halogen, the halogen atoms can be identical or different, and can all be attached to one or to a plurality of carbon atoms. Here, halogen represents in particular fluorine, chlorine, bromine or iodine, preferably fluorine, chlorine or bromine and particularly preferably fluorine.

According to the invention, "cycloalkyl" - on its own or as part of a chemical group - represents monocyclic hydrocarbons preferably having 3 carbons (cyclopropyl). The cycloalkyl group according to the invention may be substituted by one or more identical or different radicals.

According to the invention, "halogenalkyl", "halogencycloalkyl", "halogenated alkyl" and "halogenated cycloalkyl" represents halogen-substituted alkyl/cycloalkyl having preferably 1 to 5 identical or different halogen atoms such as, for example, monohaloalkyl such as CH₂CH₂Cl, CH₂CH₂F, CHClCH₃, CHFCH₃, CH₂Cl, CH₂F; monohalocycloalkyl such as 1-fluoro-cycloalkyl or 1-chloro-cycloalkyl; perhaloalkyl such as CCl₃ or CF₃ or CF₂CF₃; polyhaloalkyl such as CHF₂, CH₂F, CH₂CHFCl, CHCl₂, CF₂CF₂H, CH₂CF₃.

The compounds according to the invention may, depending on the nature of the substituents, be in the form of geometric and/or optically active isomers or corresponding isomer mixtures in different compositions. These stereoisomers are, for example, enantiomers, diastereomers, atropisomers or geometric isomers. Accordingly, the invention encompasses both pure stereoisomers and any mixture of these isomers.

If appropriate, the compounds according to the invention may be present in various polymorphic forms or as a mixture of different polymorphic forms. Both the pure polymorphs and the polymorph mixtures are provided by the invention and can be used in accordance with the invention.

### Detailed description

The halogen-substituted compounds according to the invention are defined by the general formula (I) 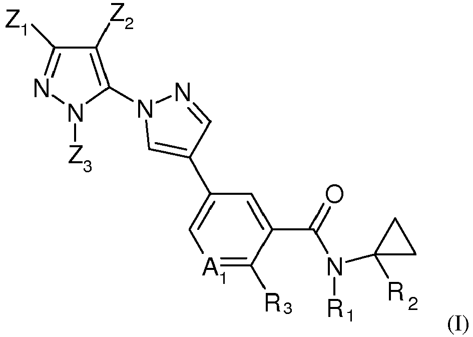 in which
- Z₁: represents 1-halogenated cyclopropyl;
- Z₂: represents -S(O)₀₋₂-(C₁-C₂)-alkyl, (C₁-C₂)-halogenalkyl, or nitro (NO₂);
- Z₃: represents (C₁-C₂)-alkyl;
- R₁: represents hydrogen (H) or (C₁-C₂)-alkyl;
- R₂: represents H or cyano (CN);
- R₃: represents CH₃ or chloro (Cl);
- A₁: represents CH or nitrogen (N).

In the following preferred embodiments any substituent R₁ to R₃, A₁, Z₁ to Z₃ of compounds of formula (I) has the meaning as defined in paragraph [0052] if not defined otherwise in the preferred embodiment. The skilled person understands that preferred embodiments can be combined as long as the combination is not against existing natural laws.

In one preferred embodiment A₁ is CH.

In another preferred embodiment A₁ is CH and R₃ is Cl.

In another preferred embodiment Z₃ is CH₃.

In another preferred embodiment Z₂ is -S-CH₃, -SO-CH₃, -SO₂-CH₃, CF₃, CF₂H, Cl, Br, I or NO₂.

In another preferred embodiment Z₂ is -S-CH₃, Cl, Br, I, CF₃, CF₂H or NO₂.

In another preferred embodiment, Z₂ is -S(O)₀₋₂- CH₃, CF₃,

In another preferred embodiment Z₂ is I, CF₂H, CF₃ or NO₂.

In another preferred embodiment Z₁ is 1-fluoro-cyclopropyl or 1-chloro-cyclopropyl.

In another preferred embodiment
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
Z₁ is 1-fluoro-cyclopropyl or 1-chloro-cyclopropyl,
Z₂ is I, CF₂H, CF₃ or NO₂, and
Z₃ is CH₃.

In another preferred embodiment
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
Z₁ is 1-fluoro-cyclopropyl,
Z₂ is -S-CH₃, Cl, Br, I, CF₂H, CF₃ or NO₂, and
Z₃ is CH₃.

In another preferred embodiment
Z₁ is 1-fluoro-cyclopropyl,
Z₂ is I, CF₂H, CF₃ or NO₂, and
Z₃ is CH₃.

In another preferred embodiment
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
Z₁ is 1-chloro-cyclopropyl,
Z₂ is -S-CH₃, Cl, Br, I, CF₂H, CF₃ or NO₂, and
Z₃ is CH₃.

In another preferred embodiment
Z₁ is or 1-chloro-cyclopropyl,
Z₂ is I, CF₂H, CF₃ or NO₂, and
Z₃ is CH₃.

In another preferred embodiment
A₁ is CH,
R₃ is Cl,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
A₁ is CH,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
A₁ is CH,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
A₁ is CH,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
A₁ is CH,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
A₁ is CH,
R₃ is Cl,
Z₁ is 1-fluoro-cyclopropyl,
Z₂ is I, CF₂H, CF₃ or NO₂, and
Z₃ is CH₃.

In another preferred embodiment
A₁ is CH,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
A₁ is CH,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
A₁ is CH,
R₃ is Cl,
Z₁ is 1-chloror-cyclopropyl,
Z₂ is I, CF₂H, CF₃ or NO₂, and
Z₃ is CH₃.

In another preferred embodiment, R₁ is H or CH₃.

In another preferred embodiment, R₁ is H.

In another preferred embodiment, R₁ is CH₃

In another preferred embodiment
R₁ is H,
A₁ is CH,
R₃ is Cl,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
A₁ is CH,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
and
Z₃ is CH₃.

In another preferred embodiment
R₁ is H,
A₁ is CH,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is H,
A₁ is CH,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
A₁ is CH,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is H,
A₁ is CH,
R₃ is Cl,
Z₁ is 1-fluoro-cyclopropyl,
Z₂ is I, CF₂H, CF₃ or NO₂, and
Z₃ is CH₃.

In another preferred embodiment
R₁ is H,
A₁ is CH,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
A₁ is CH,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is H,
A₁ is CH,
R₃ is Cl,
Z₁ is 1-chloro-cyclopropyl,
Z₂ is I, CF₂H, CF₃ or NO₂, and
Z₃ is CH₃.

In another preferred embodiment
R₁ is CH₃,
A₁ is CH,
R₃ is Cl,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
A₁ is CH,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
A₁ is CH,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is CH₃,
A₁ is CH,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
A₁ is CH,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is CH₃,
A₁ is CH,
R₃ is Cl,
Z₁ is 1-fluoro-cyclopropyl,
Z₂ is I, CF₂H, CF₃ or NO₂, and
Z₃ is CH₃.

In another preferred embodiment
R₁ is CH₃,
A₁ is CH,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
A₁ is CH,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
A₁ is CH,
R₃ is Cl,
Z₁ is 1-chloro-cyclopropyl,
Z₂ is I, CF₂H, CF₃ or NO₂, and
Z₃ is CH₃.

In another preferred embodiment, A₁ is N.

In another preferred embodiment
R₁ is H,
A₁ is N,
R₃ is Cl,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
A₁ is N,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is H,
A₁ is N,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is H,
A₁ is N,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
A₁ is N,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is H,
A₁ is N,
R₃ is Cl,
Z₁ is 1-fluoro-cyclopropyl,
Z₂ is I, CF₂H, CF₃ or NO₂, and
Z₃ is CH₃.

In another preferred embodiment
R₁ is H,
A₁ is N,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
A₁ is N,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is H,
A₁ is N,
R₃ is Cl,
Z₁ is 1-chloro-cyclopropyl,
Z₂ is I, CF₂H, CF₃ or NO₂, and
Z₃ is CH₃.

In another preferred embodiment
R₁ is CH₃,
A₁ is N,
R₃ is Cl,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
A₁ is N,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
A₁ is N,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is CH₃,
A₁ is N,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
A₁ is N,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is CH₃,
A₁ is N,
R₃ is Cl,
Z₁ is 1-fluoro-cyclopropyl,
Z₂ is I, CF₂H, CF₃ or NO₂, and
Z₃ is CH₃.

In another preferred embodiment
R₁ is CH₃,
A₁ is N,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
A₁ is N,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
A₁ is N,
R₃ is Cl,
Z₁ is 1-chloro-cyclopropyl,
Z₂ is I, CF₂H, CF₃ or NO₂, and
Z₃ is CH₃.

In another preferred embodiment R₂ is H.

In another preferred embodiment
R₁ is H,
R₂ is H,
A₁ is CH,
R₃ is Cl,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is H,
A₁ is CH,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
and
Z₃ is CH₃.

In another preferred embodiment
R₁ is H,
R₂ is H,
A₁ is CH,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is H,
R₂ is H,
A₁ is CH,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is H,
A₁ is CH,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is H,
R₂ is H,
A₁ is CH,
R₃ is Cl,
Z₁ is 1-fluoro-cyclopropyl,
Z₂ is I, CF₂H, CF₃ or NO₂, and
Z₃ is CH₃.

In another preferred embodiment
R₁ is H,
R₂ is H,
A₁ is CH,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is H,
A₁ is CH,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is H,
R₂ is H,
A₁ is CH,
R₃ is Cl,
Z₁ is 1-chloro-cyclopropyl,
Z₂ is I, CF₂H, CF₃ or NO₂, and
Z₃ is CH₃.

In another preferred embodiment
R₁ is CH₃,
R₂ is H,
A₁ is CH,
R₃ is Cl,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is H,
A₁ is CH,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is H,
A₁ is CH,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is CH₃,
R₂ is H,
A₁ is CH,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is H,
A₁ is CH,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is H,
A₁ is CH,
R₃ is Cl,
Z₁ is 1-fluoro-cyclopropyl,
Z₂ is I, CF₂H, CF₃ or NO₂, and
Z₃ is CH₃.

In another preferred embodiment
R₁ is CH₃,
R₂ is H,
A₁ is CH,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is H,
A₁ is CH,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is H,
A₁ is CH,
R₃ is Cl,
Z₁ is 1-chloro-cyclopropyl,
Z₂ is I, CF₂H, CF₃ or NO₂, and
Z₃ is CH₃.

In another preferred embodiment
R₁ is H,
R₂ is H,
A₁ is N,
R₃ is Cl,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is H,
A₁ is N,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is H,
R₂ is H,
A₁ is N,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is H,
R₂ is H,
A₁ is N,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is H,
A₁ is N,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is H,
R₂ is H,
A₁ is N,
R₃ is Cl,
Z₁ is 1-fluoro-cyclopropyl,
Z₂ is I, CF₂H, CF₃ or NO₂, and
Z₃ is CH₃.

In another preferred embodiment
R₁ is H,
R₂ is H,
A₁ is N,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is H,
A₁ is N,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is H,
R₂ is H,
A₁ is N,
R₃ is Cl,
Z₁ is 1-chloro-cyclopropyl,
Z₂ is I, CF₂H, CF₃ or NO₂, and
Z₃ is CH₃.

In another preferred embodiment
R₁ is CH₃,
R₂ is H,
A₁ is N,
R₃ is Cl,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is H,
A₁ is N,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is H,
A₁ is N,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is CH₃,
R₂ is H,
A₁ is N,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is H,
A₁ is N,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is H,
A₁ is N,
R₃ is Cl,
Z₁ is 1-fluoro-cyclopropyl,
Z₂ is I, CF₂H, CF₃ or NO₂, and
Z₃ is CH₃.

In another preferred embodiment
R₁ is CH₃,
R₂ is H,
A₁ is N,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is H,
A₁ is N,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is H,
A₁ is N,
R₃ is Cl,
Z₁ is 1-chloro-cyclopropyl,
Z₂ is I, CF₂H, CF₃ or NO₂, and
Z₃ is CH₃.

In another preferred embodiment R₂ is CN.

In another preferred embodiment
R₁ is H,
R₂ is CN,
A₁ is CH,
R₃ is Cl,
Z₁ is 1-fluoro-cyclopropyl or 1-chloro-cyclopropyl,
Z₂ is -S-CH₃, -SO-CH₃, -SO₂-CH₃, CF₃, CF₂H, Cl, Br, I or NO₂, and
Z₃ is CH₃.

In another preferred embodiment
R₁ is H,
R₂ is CN,
A₁ is CH,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
and
Z₃ is CH₃.

In another preferred embodiment
R₁ is H,
R₂ is CN,
A₁ is CH,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is H,
R₂ is CN,
A₁ is CH,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is CN,
A₁ is CH,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is H,
R₂ is CN,
A₁ is CH,
R₃ is Cl,
Z₁ is 1-fluoro-cyclopropyl,
Z₂ is I, CF₂H, CF₃ or NO₂, and
Z₃ is CH₃.

In another preferred embodiment
R₁ is H,
R₂ is CN,
A₁ is CH,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is CN,
A₁ is CH,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is H,
R₂ is CN,
A₁ is CH,
R₃ is Cl,
Z₁ is 1-chloro-cyclopropyl,
Z₂ is I, CF₂H, CF₃ or NO₂, and
Z₃ is CH₃.

In another preferred embodiment
R₁ is CH₃,
R₂ is CN,
A₁ is CH,
R₃ is Cl,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is CN,
A₁ is CH,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is CN,
A₁ is CH,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is CH₃,
R₂ is CN,
A₁ is CH,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is CN,
A₁ is CH,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is CN,
A₁ is CH,
R₃ is Cl,
Z₁ is 1-fluoro-cyclopropyl,
Z₂ is I, CF₂H, CF₃ or NO₂, and
Z₃ is CH₃.

In another preferred embodiment
R₁ is CH₃,
R₂ is CN,
A₁ is CH,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is CN,
A₁ is CH,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is CN,
A₁ is CH,
R₃ is Cl,
Z₁ is 1-chloro-cyclopropyl,
Z₂ is I, CF₂H, CF₃ or NO₂, and
Z₃ is CH₃.

In another preferred embodiment
R₁ is H,
R₂ is CN,
A₁ is N,
R₃ is Cl,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is CN,
A₁ is N,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is H,
R₂ is CN,
A₁ is N,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is H,
R₂ is CN,
A₁ is N,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is CN,
A₁ is N,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is H,
R₂ is CN,
A₁ is N,
R₃ is Cl,
Z₁ is 1-fluoro-cyclopropyl,
Z₂ is I, CF₂H, CF₃ or NO₂, and
Z₃ is CH₃.

In another preferred embodiment
R₁ is H,
R₂ is CN,
A₁ is N,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is CN,
A₁ is N,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is H,
R₂ is CN,
A₁ is N,
R₃ is Cl,
Z₁ is 1-chloro-cyclopropyl,
Z₂ is I, CF₂H, CF₃ or NO₂, and
Z₃ is CH₃.

In another preferred embodiment
R₁ is CH₃,
R₂ is CN,
A₁ is N,
R₃ is Cl,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is CN,
A₁ is N,
R₃ is Cl,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is CN,
A₁ is N,
R₃ is Cl,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is CH₃,
R₂ is CN,
A₁ is N,
R₃ is Cl,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is CN,
A₁ is N,
R₃ is Cl,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is CN,
A₁ is N,
R₃ is Cl,
Z₁ is 1-fluoro-cyclopropyl,
Z₂ is I, CF₂H, CF₃ or NO₂, and
Z₃ is CH₃.

In another preferred embodiment
R₁ is CH₃,
R₂ is CN,
A₁ is N,
R₃ is Cl,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is CN,
A₁ is N,
R₃ is Cl,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is CN,
A₁ is N,
R₃ is Cl,
Z₁ is 1-chloro-cyclopropyl,
Z₂ is I, CF₂H, CF₃ or NO₂, and
Z₃ is CH₃.

In another preferred embodiment A₁ is CH and R₃ is CH₃.

In another preferred embodiment
A₁ is CH,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
A₁ is CH,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
A₁ is CH,
R₃ is CH₃,
Z₁ is 1-fluoro-cyclopropyl or 1-chloro-cyclopropyl,
Z₂ is I, CF₃ or NO₂, and
Z₃ is CH₃.

In another preferred embodiment
A₁ is CH,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
A₁ is CH,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
A₁ is CH,
R₃ is CH₃,
Z₁ is 1-fluoro-cyclopropyl,
Z₂ is I, CF₂H, CF₃ or NO₂, and
Z₃ is CH₃.

In another preferred embodiment
A₁ is CH,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
A₁ is CH,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
A₁ is CH,
R₃ is CH₃,
Z₁ is 1-chloror-cyclopropyl,
Z₂ is I, CF₂H, CF₃ or NO₂, and
Z₃ is CH₃.

In another preferred embodiment
R₁ is H,
A₁ is CH,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
A₁ is CH,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
and
Z₃ is CH₃.

In another preferred embodiment
R₁ is H,
A₁ is CH,
R₃ is CH₃,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is H,
A₁ is CH,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
A₁ is CH,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is H,
A₁ is CH,
R₃ is CH₃,
Z₁ is 1-fluoro-cyclopropyl,
Z₂ is I, CF₂H, CF₃ or NO₂, and
Z₃ is CH₃.

In another preferred embodiment
R₁ is H,
A₁ is CH,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
A₁ is CH,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is H,
A₁ is CH,
R₃ is CH₃,
Z₁ is 1-chloro-cyclopropyl,
Z₂ is I, CF₂H, CF₃ or NO₂, and
Z₃ is CH₃.

In another preferred embodiment
R₁ is CH₃,
A₁ is CH,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
A₁ is CH,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
A₁ is CH,
R₃ is CH₃,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is CH₃,
A₁ is CH,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
A₁ is CH,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is CH₃,
A₁ is CH,
R₃ is CH₃,
Z₁ is 1-fluoro-cyclopropyl,
Z₂ is I, CF₂H, CF₃ or NO₂, and
Z₃ is CH₃.

In another preferred embodiment
R₁ is CH₃,
A₁ is CH,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
A₁ is CH,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
A₁ is CH,
R₃ is CH₃,
Z₁ is 1-chloro-cyclopropyl,
Z₂ is I, CF₂H, CF₃ or NO₂, and
Z₃ is CH₃.

In another preferred embodiment
R₁ is H,
A₁ is N,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
A₁ is N,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is H,
A₁ is N,
R₃ is CH₃,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is H,
A₁ is N,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
A₁ is N,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is H,
A₁ is N,
R₃ is CH₃,
Z₁ is 1-fluoro-cyclopropyl,
Z₂ is I, CF₂H, CF₃ or NO₂, and
Z₃ is CH₃.

In another preferred embodiment
R₁ is H,
A₁ is N,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
A₁ is N,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is H,
A₁ is N,
R₃ is CH₃,
Z₁ is 1-chloro-cyclopropyl,
Z₂ is I, CF₂H, CF₃ or NO₂, and
Z₃ is CH₃.

In another preferred embodiment
R₁ is CH₃,
A₁ is N,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
A₁ is N,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
A₁ is N,
R₃ is CH₃,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is CH₃,
A₁ is N,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
A₁ is N,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is CH₃,
A₁ is N,
R₃ is CH₃,
Z₁ is 1-fluoro-cyclopropyl,
Z₂ is I, CF₂H, CF₃ or NO₂, and
Z₃ is CH₃.

In another preferred embodiment
R₁ is CH₃,
A₁ is N,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
A₁ is N,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
A₁ is N,
R₃ is CH₃,
Z₁ is 1-chloro-cyclopropyl,
Z₂ is I, CF₂H, CF₃ or NO₂, and
Z₃ is CH₃.

In another preferred embodiment
R₁ is H,
R₂ is H,
A₁ is CH,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is H,
A₁ is CH,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
and
Z₃ is CH₃.

In another preferred embodiment
R₁ is H,
R₂ is H,
A₁ is CH,
R₃ is CH₃,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is H,
R₂ is H,
A₁ is CH,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is H,
A₁ is CH,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is H,
R₂ is H,
A₁ is CH,
R₃ is CH₃,
Z₁ is 1-fluoro-cyclopropyl,
Z₂ is I, CF₂H, CF₃ or NO₂, and
Z₃ is CH₃.

In another preferred embodiment
R₁ is H,
R₂ is H,
A₁ is CH,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is H,
A₁ is CH,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is H,
R₂ is H,
A₁ is CH,
R₃ is CH₃,
Z₁ is 1-chloro-cyclopropyl,
Z₂ is I, CF₂H, CF₃ or NO₂, and
Z₃ is CH₃.

In another preferred embodiment
R₁ is CH₃,
R₂ is H,
A₁ is CH,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is H,
A₁ is CH,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is H,
A₁ is CH,
R₃ is CH₃,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is CH₃,
R₂ is H,
A₁ is CH,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is H,
A₁ is CH,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is H,
A₁ is CH,
R₃ is CH₃,
Z₁ is 1-fluoro-cyclopropyl,
Z₂ is I, CF₂H, CF₃ or NO₂, and
Z₃ is CH₃.

In another preferred embodiment
R₁ is CH₃,
R₂ is H,
A₁ is CH,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is H,
A₁ is CH,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is H,
A₁ is CH,
R₃ is CH₃,
Z₁ is 1-chloro-cyclopropyl,
Z₂ is I, CF₂H, CF₃ or NO₂, and
Z₃ is CH₃.

In another preferred embodiment
R₁ is H,
R₂ is H,
A₁ is N,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is H,
A₁ is N,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is H,
R₂ is H,
A₁ is N,
R₃ is CH₃,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is H,
R₂ is H,
A₁ is N,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is H,
A₁ is N,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is H,
R₂ is H,
A₁ is N,
R₃ is CH₃,
Z₁ is 1-fluoro-cyclopropyl,
Z₂ is I, CF₂H, CF₃ or NO₂, and
Z₃ is CH₃.

In another preferred embodiment
R₁ is H,
R₂ is H,
A₁ is N,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is H,
A₁ is N,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is H,
R₂ is H,
A₁ is N,
R₃ is CH₃,
Z₁ is 1-chloro-cyclopropyl,
Z₂ is I, CF₂H, CF₃ or NO₂, and
Z₃ is CH₃.

In another preferred embodiment
R₁ is CH₃,
R₂ is H,
A₁ is N,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is H,
A₁ is N,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is H,
A₁ is N,
R₃ is CH₃,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is CH₃,
R₂ is H,
A₁ is N,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is H,
A₁ is N,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is H,
A₁ is N,
R₃ is CH₃,
Z₁ is 1-fluoro-cyclopropyl,
Z₂ is I, CF₂H, CF₃ or NO₂, and
Z₃ is CH₃.

In another preferred embodiment
R₁ is CH₃,
R₂ is H,
A₁ is N,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is H,
A₁ is N,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is H,
A₁ is N,
R₃ is CH₃,
Z₁ is 1-chloro-cyclopropyl,
Z₂ is I, CF₂H, CF₃ or NO₂, and
Z₃ is CH₃.

In another preferred embodiment
R₁ is H,
R₂ is CN,
A₁ is CH,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is CN,
A₁ is CH,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
and
Z₃ is CH₃.

In another preferred embodiment
R₁ is H,
R₂ is CN,
A₁ is CH,
R₃ is CH₃,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is H,
R₂ is CN,
A₁ is CH,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is CN,
A₁ is CH,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is H,
R₂ is CN,
A₁ is CH,
R₃ is CH₃,
Z₁ is 1-fluoro-cyclopropyl,
Z₂ is I, CF₂H, CF₃ or NO₂, and
Z₃ is CH₃.

In another preferred embodiment
R₁ is H,
R₂ is CN,
A₁ is CH,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is CN,
A₁ is CH,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is H,
R₂ is CN,
A₁ is CH,
R₃ is CH₃,
Z₁ is 1-chloro-cyclopropyl,
Z₂ is I, CF₂H, CF₃ or NO₂, and
Z₃ is CH₃.

In another preferred embodiment
R₁ is CH₃,
R₂ is CN,
A₁ is CH,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is CN,
A₁ is CH,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is CN,
A₁ is CH,
R₃ is CH₃,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is CH₃,
R₂ is CN,
A₁ is CH,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is CN,
A₁ is CH,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is CN,
A₁ is CH,
R₃ is CH₃,
Z₁ is 1-fluoro-cyclopropyl,
Z₂ is I, CF₂H, CF₃ or NO₂, and
Z₃ is CH₃.

In another preferred embodiment
R₁ is CH₃,
R₂ is CN,
A₁ is CH,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is CN,
A₁ is CH,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is CN,
A₁ is CH,
R₃ is CH₃,
Z₁ is 1-chloro-cyclopropyl,
Z₂ is I, CF₂H, CF₃ or NO₂, and
Z₃ is CH₃.

In another preferred embodiment
R₁ is H,
R₂ is CN,
A₁ is N,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is CN,
A₁ is N,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is H,
R₂ is CN,
A₁ is N,
R₃ is CH₃,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is H,
R₂ is CN,
A₁ is N,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is CN,
A₁ is N,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is H,
R₂ is CN,
A₁ is N,
R₃ is CH₃,
Z₁ is 1-fluoro-cyclopropyl,
Z₂ is I, CF₂H, CF₃ or NO₂, and
Z₃ is CH₃.

In another preferred embodiment
R₁ is H,
R₂ is CN,
A₁ is N,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is H,
R₂ is CN,
A₁ is N,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is H,
R₂ is CN,
A₁ is N,
R₃ is CH₃,
Z₁ is 1-chloro-cyclopropyl,
Z₂ is I, CF₂H, CF₃ or NO₂, and
Z₃ is CH₃.

In another preferred embodiment
R₁ is CH₃,
R₂ is CN,
A₁ is N,
R₃ is CH₃,
Z₁ is 1-fluoro-cyclopropyl or 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is CN,
A₁ is N,
R₃ is CH₃,
Z₁ is 1-fluoro-cyclopropyl or 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is CN,
A₁ is N,
R₃ is CH₃,
Z₁ is 1-fluoro-cyclopropyl or 1-chloro-cyclopropyl,
Z₂ is I, CF₂H, CF₃ or NO₂, and
Z₃ is CH₃.

In another preferred embodiment
R₁ is CH₃,
R₂ is CN,
A₁ is N,
R₃ is CH₃,
Z₁ is 1-fluoro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is CN,
A₁ is N,
R₃ is CH₃,
Z₁ is 1-fluoro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is CN,
A₁ is N,
R₃ is CH₃,
Z₁ is 1-fluoro-cyclopropyl,
Z₂ is I, CF₂H, CF₃ or NO₂, and
Z₃ is CH₃.

In another preferred embodiment
R₁ is CH₃,
R₂ is CN,
A₁ is N,
R₃ is CH₃,
Z₁ is 1-chloro-cyclopropyl,
Z₂ is -S-CH₃, -SO-CH₃, -SO₂-CH₃, CF₂H, CF₃, Cl, Br, I or NO₂, and
Z₃ is CH₃.

In another preferred embodiment
R₁ is CH₃,
R₂ is CN,
A₁ is N,
R₃ is CH₃,
Z₁ is 1-chloro-cyclopropyl,
Z₂ is -S-CH₃, Cl, Br, I, CF₂H, CF₃ or NO₂, and
Z₃ is CH₃.

In another preferred embodiment
R₁ is CH₃,
R₂ is CN,
A₁ is N,
R₃ is CH₃,
Z₁ is 1-chloro-cyclopropyl,
Z₂ is I, CF₂H, CF₃ or NO₂, and
Z₃ is CH₃.

Yet another preferred embodiment refers to compounds of formula (I) wherein
R₁ is H,
R₂ is CN or H,
A₁ is C-H,
R₃ is Cl,
Z₁ is 1-halo-cyclopropyl, preferably 1-fluoro-cyclopropyl, 1-chloro-cyclopropyl, 1-bromo-cyclopropyl or 1-iodo-cyclopropyl, more preferably 1-fluoro-cyclopropyl, 1-chloro-cyclopropyl or 1-bromo-cyclopropyl,
Z₂ is -S-C₁-C₃-alkyl, fluorinated C₁-C₃-alkyl or NO₂, preferably -S-CH₃, -CF₃, -CF₂H, -CFH₂ or -NO₂, and
Z₃ is CH₃.

Yet another preferred embodiment refers to compounds of formula (I) wherein
R₁ is H,
R₂ is CN or H,
A₁ is C-H,
R₃ is Cl,
Z₁ is 1-fluoro-cyclopropyl, 1-chloro-cyclopropyl, 1-bromo-cyclopropyl or 1-iodo-cyclopropyl, more preferably 1-fluoro-cyclopropyl, 1-chloro-cyclopropyl or 1-bromo-cyclopropyl, Z₂ is -S-C₁-C₃-alkyl, and
Z₃ is CH₃.

Yet another preferred embodiment refers to compounds of formula (I) wherein
R₁ is H,
R₂ is CN or H,
A₁ is C-H,
R₃ is Cl,
Z₁ is 1-fluoro-cyclopropyl, 1-chloro-cyclopropyl, 1-bromo-cyclopropyl or 1-iodo-cyclopropyl, more preferably 1-fluoro-cyclopropyl, 1-chloro-cyclopropyl or 1-bromo-cyclopropyl, Z₂ is -S-CH₃, and
Z₃ is CH₃.

Yet another preferred embodiment refers to compounds of formula (I) wherein
R₁ is H,
R₂ is CN or H,
A₁ is C-H,
R₃ is Cl,
Z₁ is 1-fluoro-cyclopropyl, 1-chloro-cyclopropyl, 1-bromo-cyclopropyl or 1-iodo-cyclopropyl, more preferably 1-fluoro-cyclopropyl, 1-chloro-cyclopropyl or 1-bromo-cyclopropyl, Z₂ is - fluorinated C₁-C₃-alkyl, preferably fluorinated methyl, and
Z₃ is CH₃.

Yet another preferred embodiment refers to compounds of formula (I) wherein
R₁ is H,
R₂ is CN or H,
A₁ is C-H,
R₃ is Cl,
Z₁ is 1-fluoro-cyclopropyl, 1-chloro-cyclopropyl, 1-bromo-cyclopropyl or 1-iodo-cyclopropyl, more preferably 1-fluoro-cyclopropyl, 1-chloro-cyclopropyl or 1-bromo-cyclopropyl, Z₂ is -CF₃, -CF₂H, -CFH₂ and
Z₃ is CH₃.

Yet another preferred embodiment refers to compounds of formula (I) wherein
R₁ is H,
R₂ is CN or H,
A₁ is C-H,
R₃ is Cl,
Z₁ is 1-fluoro-cyclopropyl, 1-chloro-cyclopropyl, 1-bromo-cyclopropyl or 1-iodo-cyclopropyl, more preferably 1-fluoro-cyclopropyl, 1-chloro-cyclopropyl or 1-bromo-cyclopropyl, Z₂ is -CF₃, and
Z₃ is CH₃.

Yet another preferred embodiment refers to compounds of formula (I) wherein
R₁ is H,
R₂ is CN or H,
A₁ is C-H,
R₃ is Cl,
Z₁ is 1-fluoro-cyclopropyl, 1-chloro-cyclopropyl, 1-bromo-cyclopropyl or 1-iodo-cyclopropyl, more preferably 1-fluoro-cyclopropyl, 1-chloro-cyclopropyl or 1-bromo-cyclopropyl, Z₂ is -CF₂H, and
Z₃ is CH₃.

Yet another preferred embodiment refers to compounds of formula (I) wherein
R₁ is H,
R₂ is CN or H,
A₁ is C-H,
R₃ is Cl,
Z₁ is 1-fluoro-cyclopropyl, 1-chloro-cyclopropyl, 1-bromo-cyclopropyl or 1-iodo-cyclopropyl, more preferably 1-fluoro-cyclopropyl, 1-chloro-cyclopropyl or 1-bromo-cyclopropyl, Z₂ is -NO₂, and
Z₃ is CH₃.

Yet another preferred embodiment refers to compounds of formula (I) wherein
R₁ is H,
R₂ is CN or H,
A₁ is N or C-H, preferably C-H,
R₃ is Cl or Me, preferably Cl,
Z₁ is 1-fluoro-cyclopropyl, 1-chloro-cyclopropyl, 1-bromo-cyclopropyl or 1-iodo-cyclopropyl, more preferably 1-fluoro-cyclopropyl, 1-chloro-cyclopropyl or 1-bromo-cyclopropyl, even more preferably 1-fluoro-cyclopropyl or 1-chloro-cyclopropyl,
Z₂ is CF₃, and
Z₃ is CH₃.

Yet another preferred embodiment refers to compounds of formula (I) wherein
R₁ is H,
R₂ is CN or H,
A₁ is C-H,
R₃ is Cl,
Z₁ is 1-fluoro-cyclopropyl or 1-chloro-cyclopropyl,
Z₂ is CF₃, and
Z₃ is CH₃.

Yet another preferred embodiment refers to compounds of formula (I) wherein
R₁ is H,
R₂ is CN or H,
A₁ is C-H or N, preferably C-H,
R₃ is Cl or Me, preferably Cl,
Z₁ is 1-fluoro-cyclopropyl, 1-chloro-cyclopropyl, 1-bromo-cyclopropyl or 1-iodo-cyclopropyl, more preferably 1-fluoro-cyclopropyl, 1-chloro-cyclopropyl or 1-bromo-cyclopropyl, even more preferably 1-fluoro-cyclopropyl or 1-chloro-cyclopropyl,
Z₂ is S(=O)ₙ-(CH₃), and
Z₃ is CH₃.

Yet another preferred embodiment refers to compounds of formula (I) wherein
R₁ is H,
R₂ is CN or H,
A₁ is C-H
R₃ is Cl or Me, preferably Cl,
Z₁ is 1-fluoro-cyclopropyl or 1-chloro-cyclopropyl,
Z₂ is S(=O)ₙ-(CH₃), and
Z₃ is CH₃.

The compounds according to the invention can be prepared by customary methods known to those skilled in the art.

### Isomers

Depending on the nature of the substituents, the compounds of the formula (I) may be in the form of geometric and/or optically active isomers or corresponding isomer mixtures in different compositions. These stereoisomers are, for example, enantiomers, diastereomers, atropisomers or geometric isomers. Accordingly, the invention encompasses both pure stereoisomers and any mixture of these isomers.

### Methods and uses

The invention also relates to methods for controlling animal pests, in which compounds of the formula (I) are allowed to act on animal pests and/or their habitat. The control of the animal pests is preferably conducted in agriculture and forestry, and in material protection. Preferably excluded herefrom are methods for the surgical or therapeutic treatment of the human or animal body and diagnostic methods carried out on the human or animal body.

The invention furthermore relates to the use of the compounds of the formula (I) as pesticides, in particular crop protection agents.

In the context of the present application, the term "pesticide" in each case also always comprises the term "crop protection agent".

The compounds of the formula (I), having good plant tolerance, favourable homeotherm toxicity and good environmental compatibility, are suitable for protecting plants and plant organs against biotic and abiotic stressors, for increasing harvest yields, for improving the quality of the harvested material and for controlling animal pests, especially insects, arachnids, helminths, nematodes and molluscs, which are encountered in agriculture, in horticulture, in animal husbandry, in aquatic cultures, in forests, in gardens and leisure facilities, in the protection of stored products and of materials, and in the hygiene sector. They can preferably be used as pesticides. They are active against normally sensitive and resistant species and against all or some stages of development. The abovementioned pests include:
pests from the phylum of the Arthropoda, in particular from the class of the Arachnida, for example Acarus spp., for example Acarus siro, Aceria kuko, Aceria sheldoni, Aculops spp., Aculus spp., for example Aculus fockeui, Aculus schlechtendali, Amblyomma spp., Amphitetranychus viennensis, Argas spp., Boophilus spp., Brevipalpus spp., for example Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., for example Eotetranychus hicoriae, Epitrimerus pyri, Eutetranychus spp., for example Eutetranychus banksi, Eriophyes spp., for example Eriophyes pyri, Glycyphagus domesticus, Halotydeus destructor, Hemitarsonemus spp., for example Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., for example Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., for example Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., for example Tarsonemus confusus, Tarsonemus pallidus, Tetranychus spp., for example Tetranychus canadensis, Tetranychus cinnabarinus, Tetranychus turkestani, Tetranychus urticae, Trombicula alfreddugesi, Vaejovis spp., Vasates lycopersici;
from the class of the Chilopoda, for example Geophilus spp., Scutigera spp.;
from the order or the class of the Collembola, for example Onychiurus armatus; Sminthurus viridis;
from the class of the Diplopoda, for example Blaniulus guttulatus;
from the class of the Insecta, for example from the order of the Blattodea, for example Blatta orientalis, Blattella asahinai, Blattella germanica, Leucophaea maderae, Panchlora spp., Parcoblatta spp., Periplaneta spp., for example Periplaneta americana, Periplaneta australasiae, Supella longipalpa;
from the order of the Coleoptera, for example Acalymma vittatum, Acanthoscelides obtectus, Adoretus spp., Agelastica alni, Agriotes spp., for example Agriotes linneatus, Agriotes mancus, Alphitobius diaperinus, Amphimallon solstitialis, Anobium punctatum, Anoplophora spp., Anthonomus spp., for example Anthonomus grandis, Anthrenus spp., Apion spp., Apogonia spp., Atomaria spp., for example Atomaria linearis, Attagenus spp., Baris caerulescens, Bruchidius obtectus, Bruchus spp., for example Bruchus pisorum, Bruchus rufimanus, Cassida spp., Cerotoma trifurcata, Ceutorrhynchus spp., for example Ceutorrhynchus assimilis, Ceutorrhynchus quadridens, Ceutorrhynchus rapae, Chaetocnema spp., for example Chaetocnema confinis, Chaetocnema denticulata, Chaetocnema ectypa, Cleonus mendicus, Conoderus spp., Cosmopolites spp., for example Cosmopolites sordidus, Costelytra zealandica, Ctenicera spp., Curculio spp., for example Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., for example Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epilachna spp., for example Epilachna borealis, Epilachna varivestis, Epitrix spp., for example Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., for example Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., for example Leucoptera coffeella, Lissorhoptrus oryzophilus, Lixus spp., Luperomorpha xanthodera, Luperodes spp., Lyctus spp., Megascelis spp., Melanotus spp., for example Melanotus longulus oregonensis, Meligethes aeneus, Melolontha spp., for example Melolontha melolontha, Migdolus spp., Monochamus spp., Naupactus xanthographus, Necrobia spp., Niptus hololeucus, Oryctes rhinoceros, Oryzaephilus surinamensis, Oryzaphagus oryzae, Otiorhynchus spp., for example Otiorhynchus cribricollis, Otiorhynchus ligustici, Otiorhynchus ovatus, Otiorhynchus rugosostriarus, Otiorhynchus sulcatus, Oxycetonia jucunda, Phaedon cochleariae, Phyllophaga spp., Phyllophaga helleri, Phyllotreta spp., for example Phyllotreta armoraciae, Phyllotreta pusilla, Phyllotreta ramosa, Phyllotreta striolata, Popillia japonica, Premnotrypes spp., Prostephanus truncatus, Psylliodes spp., for example Psylliodes affinis, Psylliodes chrysocephala, Psylliodes punctulata, Ptinus spp., Rhizobius ventralis, Rhizopertha dominica, Sitophilus spp., for example Sitophilus granarius, Sitophilus linearis, Sitophilus oryzae, Sitophilus zeamais, Sphenophorus spp., Stegobium paniceum, Sternechus spp., for example Sternechus paludatus, Symphyletes spp., Tanymecus spp., for example Tanymecus dilaticollis, Tanymecus indicus, Tanymecus palliatus, Tenebrio molitor, Tenebrioides mauretanicus, Tribolium spp., for example Tribolium audax, Tribolium castaneum, Tribolium confusum, Trogoderma spp., Tychius spp., Xylotrechus spp., Zabrus spp., for example Zabrus tenebrioides;
from the order of the Diptera, for example Aedes spp., for example Aedes aegypti, Aedes albopictus, Aedes sticticus, Aedes vexans, Agromyza spp., for example Agromyza frontella, Agromyza parvicornis, Anastrepha spp., Anopheles spp., for example Anopheles quadrimaculatus, Anopheles gambiae, Asphondylia spp., Bactrocera spp., for example Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., for example Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., for example Culex pipiens, Culex quinquefasciatus, Culicoides spp., Culiseta spp., Cuterebra spp., Dacus oleae, Dasineura spp., for example Dasineura brassicae, Delia spp., for example Delia antiqua, Delia coarctata, Delia florilega, Delia platura, Delia radicum, Dermatobia hominis, Drosophila spp., for example Drosphila melanogaster, Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., for example Liriomyza brassicae, Liriomyza huidobrensis, Liriomyza sativae, Lucilia spp., for example Lucilia cuprina, Lutzomyia spp., Mansonia spp., Musca spp., for example Musca domestica, Musca domestica vicina, Oestrus spp., Oscinella frit, Paratanytarsus spp., Paralauterborniella subcincta, Pegomya spp., for example Pegomya betae, Pegomya hyoscyami, Pegomya rubivora, Phlebotomus spp., Phorbia spp., Phormia spp., Piophila casei, Prodiplosis spp., Psila rosae, Rhagoletis spp., for example Rhagoletis cingulata, Rhagoletis completa, Rhagoletis fausta, Rhagoletis indifferens, Rhagoletis mendax, Rhagoletis pomonella, Sarcophaga spp., Simulium spp., for example Simulium meridionale, Stomoxys spp., Tabanus spp., Tetanops spp., Tipula spp., for example Tipula paludosa, Tipula simplex;
from the order of the Hemiptera, for example Acizzia acaciaebaileyanae, Acizzia dodonaeae, Acizzia uncatoides, Acrida turrita, Acyrthosipon spp., for example Acyrthosiphon pisum, Acrogonia spp., Aeneolamia spp., Agonoscena spp., Aleyrodes proletella, Aleurolobus barodensis, Aleurothrixus floccosus, Allocaridara malayensis, Amrasca spp., for example Amrasca bigutulla, Amrasca devastans, Anuraphis cardui, Aonidiella spp., for example Aonidiella aurantii, Aonidiella citrina, Aonidiella inornata, Aphanostigma piri, Aphis spp., for example Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., for example Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., for example Cacopsylla pyricola, Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., for example Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes citri, Diaphorina citri, Diaspis spp., Drosicha spp., Dysaphis spp., for example Dysaphis apiifolia, Dysaphis plantaginea, Dysaphis tulipae, Dysmicoccus spp., Empoasca spp., for example Empoasca abrupta, Empoasca fabae, Empoasca maligna, Empoasca solana, Empoasca stevensi, Eriosoma spp., for example Eriosoma americanum, Eriosoma lanigerum, Eriosoma pyricola, Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Hyalopterus pruni, Icerya spp., for example Icerya purchasi, Idiocerus spp., Idioscopus spp., Laodelphax striatellus, Lecanium spp., for example Lecanium corni (=Parthenolecanium corni), Lepidosaphes spp., for example Lepidosaphes ulmi, Lipaphis erysimi, Lycorma delicatula, Macrosiphum spp., for example Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., for example Myzus ascalonicus, Myzus cerasi, Myzus ligustri, Myzus ornatus, Myzus persicae,. Myzus nicotianae, Nasonovia ribisnigri, Nephotettix spp., for example Nephotettix cincticeps,, Nephotettix nigropictus, Nilaparvata lugens, Oncometopia spp., Orthezia praelonga, Oxya chinensis, Pachypsylla spp., Parabemisia myricae, Paratrioza spp., for example Paratrioza cockerelli, Parlatoria spp., Pemphigus spp., for example Pemphigus bursarius, Pemphigus populivenae, Peregrinus maidis, Phenacoccus spp., for example Phenacoccus madeirensis, Phloeomyzus passerinii, Phorodon humuli, Phylloxera spp., for example Phylloxera devastatrix, Phylloxera notabilis, Pinnaspis aspidistrae, Planococcus spp., for example Planococcus citri, Prosopidopsylla flava, Protopulvinaria pyriformis, Pseudaulacaspis pentagona, Pseudococcus spp., for example Pseudococcus calceolariae, Pseudococcus comstocki, Pseudococcus longispinus, Pseudococcus maritimus, Pseudococcus viburni, Psyllopsis spp., Psylla spp., for example Psylla buxi, Psylla mali, Psylla pyri, Pteromalus spp., Pyrilla spp., Quadraspidiotus spp., for example Quadraspidiotus juglansregiae, Quadraspidiotus ostreaeformis, Quadraspidiotus perniciosus, Quesada gigas, Rastrococcus spp., Rhopalosiphum spp., for example Rhopalosiphum maidis, Rhopalosiphum oxyacanthae, Rhopalosiphum padi, Rhopalosiphum rufiabdominale, Saissetia spp., for example Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., for example Toxoptera aurantii, Toxoptera citricidus, Trialeurodes vaporariorum, Trioza spp., for example Trioza diospyri, Typhlocyba spp., Unaspis spp., Viteus vitifolii, Zygina spp.;
from the suborder of the Heteroptera, for example Anasa tristis, Antestiopsis spp., Boisea spp., Blissus spp., Calocoris spp., Campylomma livida, Cavelerius spp., Cimex spp., for example Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., for example Euschistus heros, Euschistus servus, Euschistus tristigmus, Euschistus variolarius, Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., for example Lygocoris pabulinus, Lygus spp., for example Lygus elisus, Lygus hesperus, Lygus lineolaris, Macropes excavatus, Monalonion atratum, Nezara spp., for example Nezara viridula, Oebalus spp., Piesma quadrata, Piezodorus spp., for example Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
from the order of the Hymenoptera, for example Acromyrmex spp., Athalia spp., for example Athalia rosae, Atta spp., Diprion spp., for example Diprion similis, Hoplocampa spp., for example Hoplocampa cookei, Hoplocampa testudinea, Lasius spp., Linepithema humile, Monomorium pharaonis, Sirex spp., Solenopsis invicta, Tapinoma spp., Urocerus spp., Vespa spp., for example Vespa crabro, Xeris spp.;
from the order of the Isopoda, for example Armadillidium vulgare, Oniscus asellus, Porcellio scaber;
from the order of the Isoptera, for example Coptotermes spp., for example Coptotermes formosanus, Cornitermes cumulans, Cryptotermes spp., Incisitermes spp., Microtermes obesi, Odontotermes spp., Reticulitermes spp., for example Reticulitermes flavipes, Reticulitermes hesperus;
from the order of the Lepidoptera, for example Achroia grisella, Acronicta major, Adoxophyes spp., for example Adoxophyes orana, Aedia leucomelas, Agrotis spp., for example Agrotis segetum, Agrotis ipsilon, Alabama spp., for example Alabama argillacea, Amyelois transitella, Anarsia spp., Anticarsia spp., for example Anticarsia gemmatalis, Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., for example Chilo plejadellus, Chilo suppressalis, Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., for example Cydia nigricana, Cydia pomonella, Dalaca noctuides, Diaphania spp., Diatraea saccharalis, Earias spp., Ecdytolopha aurantium, Elasmopalpus lignosellus, Eldana saccharina, Ephestia spp., for example Ephestia elutella, Ephestia kuehniella, Epinotia spp., Epiphyas postvittana, Etiella spp., Eulia spp., Eupoecilia ambiguella, Euproctis spp., for example Euproctis chrysorrhoea, Euxoa spp., Feltia spp., Galleria mellonella, Gracillaria spp., Grapholitha spp., for example Grapholita molesta, Grapholita prunivora, Hedylepta spp., Helicoverpa spp., for example Helicoverpa armigera, Helicoverpa zea, Heliothis spp., for example Heliothis virescens Hofmannophila pseudospretella, Homoeosoma spp., Homona spp., Hyponomeuta padella, Kakivoria flavofasciata, Laphygma spp., Leucinodes orbonalis, Leucoptera spp., for example Leucoptera coffeella, Lithocolletis spp., for example Lithocolletis blancardella, Lithophane antennata, Lobesia spp., for example Lobesia botrana, Loxagrotis albicosta, Lymantria spp., for example Lymantria dispar, Lyonetia spp., for example Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Oria spp., Orthaga spp., Ostrinia spp., for example Ostrinia nubilalis, Oulema melanopus, Oulema oryzae, Panolis flammea, Parnara spp., Pectinophora spp., for example Pectinophora gossypiella, Perileucoptera spp., Phthorimaea spp., for example Phthorimaea operculella, Phyllocnistis citrella, Phyllonorycter spp., for example Phyllonorycter blancardella, Phyllonorycter crataegella, Pieris spp., for example Pieris rapae, Platynota stultana, Plodia interpunctella, Plusia spp., Plutella xylostella (=Plutella maculipennis), Prays spp., Prodenia spp., Protoparce spp., Pseudaletia spp., for example Pseudaletia unipuncta, Pseudoplusia includens, Pyrausta nubilalis, Rachiplusia nu, Schoenobius spp., for example Schoenobius bipunctifer, Scirpophaga spp., for example Scirpophaga innotata, Scotia segetum, Sesamia spp., for example Sesamia inferens, Sparganothis spp., Spodoptera spp., for example Spodoptera eradiana, Spodoptera exigua, Spodoptera frugiperda, Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., for example Trichoplusia ni, Tryporyza incertulas, Tuta absoluta, Virachola spp.;
from the order of the Orthoptera or Saltatoria, for example Acheta domesticus, Dichroplus spp., Gryllotalpa spp., for example Gryllotalpa gryllotalpa, Hieroglyphus spp., Locusta spp., for example Locusta migratoria, Melanoplus spp., for example Melanoplus devastator, Paratlanticus ussuriensis, Schistocerca gregaria;
from the order of the Phthiraptera, for example Damalinia spp., Haematopinus spp., Linognathus spp., Pediculus spp., Phylloxera vastatrix, Phthirus pubis, Trichodectes spp.;
from the order of the Psocoptera, for example Lepinotus spp., Liposcelis spp.;
from the order of the Siphonaptera, for example, Ceratophyllus spp., Ctenocephalides spp., for example Ctenocephalides canis, Ctenocephalides felis, Pulex irritans, Tunga penetrans, Xenopsylla cheopis;
from the order of the Thysanoptera, for example Anaphothrips obscurus, Baliothrips biformis, Drepanothrips reuteri, Enneothrips Havens, Frankliniella spp., for example Frankliniella fusca, Frankliniella occidentalis, Frankliniella schultzei, Frankliniella tritici, Frankliniella vaccinii, Frankliniella williamsi, Heliothrips spp., Hercinothrips femoralis, Rhipiphorothrips cruentatus, Scirtothrips spp., Taeniothrips cardamomi, Thrips spp., for example Thrips palmi, Thrips tabaci;
from the order of the Zygentoma (= Thysanura), for example Ctenolepisma spp., Lepisma saccharina, Lepismodes inquilinus, Thermobia domestica;
from the class of the Symphyla, for example Scutigerella spp., for example Scutigerella immaculata;
pests from the phylum of the Mollusca, for example from the class of the Bivalvia, for example Dreissena spp.,
and also from the class of the Gastropoda, for example Arion spp., for example Arion ater rufus, Biomphalaria spp., Bulinus spp., Deroceras spp., for example Deroceras laeve, Galba spp., Lymnaea spp., Oncomelania spp., Pomacea spp., Succinea spp.;
animal and human parasites from the phyla of the Platylminthes and Nematoda, for example Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;
plant pests from the phylum of the Nematoda, i.e. phytoparasitic nematodes, in particular Aglenchus spp., for example Aglenchus agricola, Anguina spp., for example Anguina tritici, Aphelenchoides spp., for example Aphelenchoides arachidis, Aphelenchoides fragariae, Belonolaimus spp., for example Belonolaimus gracilis, Belonolaimus longicaudatus, Belonolaimus nortoni, Bursaphelenchus spp., for example Bursaphelenchus cocophilus, Bursaphelenchus eremus, Bursaphelenchus xylophilus, Cacopaurus spp., for example Cacopaurus pestis, Criconemella spp., for example Criconemella curvata, Criconemella onoensis, Criconemella ornata, Criconemella rusium, Criconemella xenoplax (= Mesocriconema xenoplax), Criconemoides spp., for example Criconemoides ferniae, Criconemoides onoense, Criconemoides ornatum, Ditylenchus spp., for example Ditylenchus dipsaci, Dolichodorus spp., Globodera spp., for example Globodera pallida, Globodera rostochiensis, Helicotylenchus spp., for example Helicotylenchus dihystera, Hemicriconemoides spp., Hemicycliophora spp., Heterodera spp., for example Heterodera avenae, Heterodera glycines, Heterodera schachtii, Hoplolaimus spp., Longidorus spp., for example Longidorus africanus, Meloidogyne spp., for example Meloidogyne chitwoodi, Meloidogyne fallax, Meloidogyne hapla, Meloidogyne incognita, Meloinema spp., Nacobbus spp., Neotylenchus spp., Paraphelenchus spp., Paratrichodorus spp., for example Paratrichodorus minor, Pratylenchus spp., for example Pratylenchus penetrans, Pseudohalenchus spp., Psilenchus spp., Punctodera spp., Quinisulcius spp., Radopholus spp., for example Radopholus citrophilus, Radopholus similis, Rotylenchulus spp., Rotylenchus spp., Scutellonema spp., Subanguina spp., Trichodorus spp., for example Trichodorus obtusus, Trichodorus primitivus, Tylenchorhynchus spp., for example Tylenchorhynchus annulatus, Tylenchulus spp., for example Tylenchulus semipenetrans, Xiphinema spp., for example Xiphinema index.

Furthermore, it is possible to control, from the subkingdom of the Protozoa, the order of the Coccidia, for example Eimeria spp.

The compounds of the formula (I) can optionally, at certain concentrations or application rates, also be used as herbicides, safeners, growth regulators or agents to improve plant properties, as microbicides or gametocides, for example as fungicides, antimycotics, bactericides, viricides (including agents against viroids) or as agents against MLO (mycoplasma-like organisms) and RLO (rickettsia-like organisms). If appropriate, they can also be used as intermediates or precursors for the synthesis of other active compounds.

### Formulations

The present invention further relates to formulations and use forms prepared therefrom as pesticides, for example drench, drip and spray liquors, comprising at least one compound of the formula (I). In some cases, the use forms comprise further pesticides and/or adjuvants which improve action, such as penetrants, e.g. vegetable oils, for example rapeseed oil, sunflower oil, mineral oils, for example paraffin oils, alkyl esters of vegetable fatty acids, for example rapeseed oil methyl ester or soya oil methyl ester, or alkanol alkoxylates and/or spreaders, for example alkylsiloxanes and/or salts, for example organic or inorganic ammonium or phosphonium salts, for example ammonium sulphate or diammonium hydrogenphosphate and/or retention promoters, for example dioctyl sulphosuccinate or hydroxypropyl guar polymers and/or humectants, for example glycerol and/or fertilizers, for example ammonium-, potassium- or phosphorus-containing fertilizers.

Customary formulations are, for example, water-soluble liquids (SL), emulsion concentrates (EC), emulsions in water (EW), suspension concentrates (SC, SE, FS, OD), water-dispersible granules (WG), granules (GR) and capsule concentrates (CS); these and further possible formulation types are described, for example, by Crop Life International and in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576. The formulations, in addition to one or more compounds of the formula (I), optionally comprise further agrochemically active compounds.

These are preferably formulations or use forms which comprise auxiliaries, for example extenders, solvents, spontaneity promoters, carriers, emulsifiers, dispersants, frost protectants, biocides, thickeners and/or further auxiliaries, for example adjuvants. An adjuvant in this context is a component which enhances the biological effect of the formulation, without the component itself having any biological effect. Examples of adjuvants are agents which promote retention, spreading, attachment to the leaf surface or penetration.

These formulations are prepared in a known way, for example by mixing the compounds of the formula (I) with auxiliaries such as, for example, extenders, solvents and/or solid carriers and/or other auxiliaries such as, for example, surfactants. The formulations are prepared either in suitable facilities or else before or during application.

The auxiliaries used may be substances suitable for imparting special properties, such as certain physical, technical and/or biological properties, to the formulation of the compounds of the formula (I), or to the use forms prepared from these formulations (for example ready-to-use pesticides such as spray liquors or seed dressing products).

Suitable extenders are, for example, water, polar and nonpolar organic chemical liquids, for example from the classes of the aromatic and non-aromatic hydrocarbons (such as paraffins, alkylbenzenes, alkylnaphthalenes, chlorobenzenes), the alcohols and polyols (which, if appropriate, may also be substituted, etherified and/or esterified), the ketones (such as acetone, cyclohexanone), esters (including fats and oils) and (poly)ethers, the unsubstituted and substituted amines, amides, lactams (such as N-alkylpyrrolidones) and lactones, the sulphones and sulphoxides (such as dimethyl sulphoxide).

If the extender used is water, it is also possible to employ, for example, organic solvents as auxiliary solvents. Essentially, suitable liquid solvents are: aromatics such as xylene, toluene or alkylnaphthalenes, chlorinated aromatics or chlorinated aliphatic hydrocarbons such as chlorobenzenes, chloroethylenes or methylene chloride, aliphatic hydrocarbons such as cyclohexane or paraffins, for example mineral oil fractions, mineral and vegetable oils, alcohols such as butanol or glycol and their ethers and esters, ketones such as acetone, methyl ethyl ketone, methyl isobutyl ketone or cyclohexanone, strongly polar solvents such as dimethylformamide and dimethyl sulphoxide, and also water.

In principle, it is possible to use all suitable solvents. Examples of suitable solvents are aromatic hydrocarbons, such as xylene, toluene or alkylnaphthalenes, chlorinated aromatic or chlorinated aliphatic hydrocarbons, such as chlorobenzene, chloroethylene or methylene chloride, aliphatic hydrocarbons, such as cyclohexane, paraffins, petroleum fractions, mineral and vegetable oils, alcohols, such as methanol, ethanol, isopropanol, butanol or glycol and their ethers and esters, ketones such as acetone, methyl ethyl ketone, methyl isobutyl ketone or cyclohexanone, strongly polar solvents, such as dimethyl sulphoxide, and also water.

In principle, it is possible to use all suitable carriers. Useful carriers include especially: for example ammonium salts and ground natural minerals such as kaolins, clays, talc, chalk, quartz, attapulgite, montmorillonite or diatomaceous earth, and ground synthetic materials such as finely divided silica, alumina and natural or synthetic silicates, resins, waxes and/or solid fertilizers. Mixtures of such carriers can likewise be used. Useful carriers for granules include: for example crushed and fractionated natural rocks such as calcite, marble, pumice, sepiolite, dolomite, and synthetic granules of inorganic and organic meals, and also granules of organic material such as sawdust, paper, coconut shells, corn cobs and tobacco stalks.

Liquefied gaseous extenders or solvents can also be used. Particularly suitable extenders or carriers are those which are gaseous at ambient temperature and under atmospheric pressure, for example aerosol propellant gases, such as halohydrocarbons, and also butane, propane, nitrogen and carbon dioxide.

Examples of emulsifiers and/or foam-formers, dispersants or wetting agents with ionic or nonionic properties, or mixtures of these surfactants, are salts of polyacrylic acid, salts of lignosulphonic acid, salts of phenolsulphonic acid or naphthalenesulphonic acid, polycondensates of ethylene oxide with fatty alcohols or with fatty acids or with fatty amines, with substituted phenols (preferably alkylphenols or arylphenols), salts of sulphosuccinic esters, taurine derivatives (preferably alkyl taurates), phosphoric esters of polyethoxylated alcohols or phenols, fatty esters of polyols, and derivatives of the compounds containing sulphates, sulphonates and phosphates, for example alkylaryl polyglycol ethers, alkylsulphonates, alkyl sulphates, arylsulphonates, protein hydrolysates, lignosulphite waste liquors and methylcellulose. The presence of a surfactant is advantageous if one of the compounds of the formula (I) and/or one of the inert carriers is insoluble in water and when the application takes place in water.

It is possible to use colorants such as inorganic pigments, for example iron oxide, titanium oxide and Prussian Blue, and organic dyes such as alizarin dyes, azo dyes and metal phthalocyanine dyes, and nutrients and trace nutrients such as salts of iron, manganese, boron, copper, cobalt, molybdenum and zinc as further auxiliaries in the formulations and the use forms derived therefrom.

Additional components may be stabilizers, such as low-temperature stabilizers, preservatives, antioxidants, light stabilizers or other agents which improve chemical and/or physical stability. Foam formers or antifoams may also be present.

Tackifiers such as carboxymethylcellulose and natural and synthetic polymers in the form of powders, granules or latices, such as gum arabic, polyvinyl alcohol and polyvinyl acetate, or else natural phospholipids such as cephalins and lecithins and synthetic phospholipids may also be present as additional auxiliaries in the formulations and the use forms derived therefrom. Further possible auxiliaries are mineral and vegetable oils.

Optionally, further auxiliaries may be present in the formulations and the use forms derived therefrom. Examples of such additives include fragrances, protective colloids, binders, adhesives, thickeners, thixotropic agents, penetrants, retention promoters, stabilizers, sequestrants, complexing agents, humectants, spreaders. In general, the compounds of the formula (I) can be combined with any solid or liquid additive commonly used for formulation purposes.

Useful retention promoters include all those substances which reduce the dynamic surface tension, for example dioctyl sulphosuccinate, or increase the viscoelasticity, for example hydroxypropylguar polymers.

Suitable penetrants in the present context are all those substances which are usually used for improving the penetration of agrochemical active compounds into plants. Penetrants are defined in this context by their ability to penetrate from the (generally aqueous) application liquor and/or from the spray coating into the cuticle of the plant and thereby increase the mobility of active compounds in the cuticle. The method described in the literature (Baur et al., 1997, Pesticide Science 51, 131-152) can be used to determine this property. Examples include alcohol alkoxylates such as coconut fatty ethoxylate (10) or isotridecyl ethoxylate (12), fatty acid esters, for example rapeseed oil methyl ester or soya oil methyl ester, fatty amine alkoxylates, for example tallowamine ethoxylate (15), or ammonium and/or phosphonium salts, for example ammonium sulphate or diammonium hydrogenphosphate.

The formulations preferably comprise between 0.00000001 and 98% by weight of the compound of the formula (I) or, with particular preference, between 0.01% and 95% by weight of the compound of the formula (I), more preferably between 0.5% and 90% by weight of the compound of the formula (I), based on the weight of the formulation.

The content of the compound of the formula (I) in the use forms prepared from the formulations (in particular pesticides) may vary within wide ranges. The concentration of the compound of the formula (I) in the use forms is usually between 0.00000001 and 95% by weight of the compound of the formula (I), preferably between 0.00001 and 1% by weight, based on the weight of the use form. The compounds are employed in a customary manner appropriate for the use forms.

### Mixtures

The compounds of the formula (I) may also be employed as a mixture with one or more suitable fungicides, bactericides, acaricides, molluscicides, nematicides, insecticides, microbiologicals, beneficial species, herbicides, fertilizers, bird repellents, phytotonics, sterilants, safeners, semiochemicals and/or plant growth regulators, in order thus, for example, to broaden the spectrum of action, to prolong the duration of action, to increase the rate of action, to prevent repulsion or prevent evolution of resistance. In addition, such active compound combinations may improve plant growth and/or tolerance to abiotic factors, for example high or low temperatures, to drought or to elevated water content or soil salinity. It is also possible to improve flowering and fruiting performance, optimize germination capacity and root development, facilitate harvesting and improve yields, influence maturation, improve the quality and/or the nutritional value of the harvested products, prolong storage life and/or improve the processability of the harvested products.

Furthermore, the compounds of the formula (I) can be present in a mixture with other active compounds or semiochemicals such as attractants and/or bird repellants and/or plant activators and/or growth regulators and/or fertilizers. Likewise, the compounds of the formula (I) can be used to improve plant properties such as, for example, growth, yield and quality of the harvested material.

In a particular embodiment according to the invention, the compounds of the formula (I) are present in formulations or the use forms prepared from these formulations in a mixture with further compounds, preferably those as described below.

If one of the compounds mentioned below can occur in different tautomeric forms, these forms are also included even if not explicitly mentioned in each case.

### Insecticides/acaricides/nematicides

The active compounds identified here by their common names are known and are described, for example, in the pesticide handbook ("The Pesticide Manual" 16th Ed., British Crop Protection Council 2012) or can be found on the Internet (e.g. http://www.alanwood.net/pesticides).
(1) Acetylcholinesterase (AChE) inhibitors, such as, for example, carbamates, for example alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC and xylylcarb; or organophosphates, for example acephate, azamethiphos, azinphos-ethyl, azinphos-methyl, cadusafos, chlorethoxyfos, chlorfenvinphos, chlormephos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothiophosphoryl) salicylate, isoxathion, malathion, mecarbam, methamidophos, methidathion, mevinphos, monocrotophos, naled, omethoate, oxydemeton-methyl, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, triclorfon and vamidothion.
(2) GABA-gated chloride channel antagonists, such as, for example, cyclodiene-organochlorines, for example chlordane and endosulfan or phenylpyrazoles (fiproles), for example ethiprole and fipronil.
(3) Sodium channel modulators / voltage-gated sodium channel blockers such as, for example, pyrethroids, e.g. acrinathrin, allethrin, d-cis-trans allethrin, d-trans allethrin, bifenthrin, bioallethrin, bioallethrin s-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans-isomer], deltamethrin, empenthrin [(EZ)-(1R)-isomer], esfenvalerate, etofenprox, fenpropathrin, fenvalerate, flucythrinate, flumethrin, tau-fluvalinate, halfenprox, imiprothrin, kadethrin, permethrin, phenothrin [(1R)-transisomer], prallethrin, pyrethrins (pyrethrum), resmethrin, silafluofen, tefluthrin, tetramethrin, tetramethrin [(1R)- isomer)], tralomethrin and transfluthrin or DDT or methoxychlor.
(4) Nicotinergic acetylcholine receptor (nAChR) agonists, such as, for example, neonicotinoids, e.g. acetamiprid, clothianidin, dinotefuran, imidacloprid, nitenpyram, thiacloprid and thiamethoxam or nicotine or sulfoxaflor.
(5) Allosteric activators of the nicotinergic acetylcholine receptor (nAChR) such as, for example, spinosyns, e.g. spinetoram and spinosad.
(6) Chloride channel activators, such as, for example, avermectins/milbemycins, for example abamectin, emamectin benzoate, lepimectin and milbemectin.
(7) Juvenile hormone imitators such as, for example, juvenile hormone analogues, e.g. hydroprene, kinoprene and methoprene or fenoxycarb or pyriproxyfen.
(8) Active compounds with unknown or nonspecific mechanisms of action such as, for example, alkyl halides, e.g. methyl bromide and other alkyl halides; or chloropicrine or sulphuryl fluoride or borax or tartar emetic.
(9) Selective antifeedants, for example pymetrozine or flonicamid.
(10) Mite growth inhibitors, for example clofentezine, hexythiazox and diflovidazin or etoxazole.
(11) Microbial disruptors of the insect gut membrane, for example Bacillus thuringiensis subspecies israelensis, Bacillus sphaericus, Bacillus thuringiensis subspecies aizawai, Bacillus thuringiensis subspecies kurstaki, Bacillus thuringiensis subspecies tenebrionis, and BT plant proteins: Cry1Ab, Cry1Ac, Cry1Fa, Cry2Ab, mCry3A, Cry3Ab, Cry3Bb, Cry34/35Ab1.
(12) Oxidative phosphorylation inhibitors, ATP disruptors such as, for example, diafenthiuron or organotin compounds, for example azocyclotin, cyhexatin and fenbutatin oxide or propargite or tetradifon;
(13) Oxidative phosphorylation decouplers acting by interrupting the H proton gradient such as, for example, chlorfenapyr, DNOC and sulfluramid.
(14) Nicotinergic acetylcholine receptor antagonists such as, for example, bensultap, cartap hydrochloride, thiocylam, and thiosultap-sodium.
(15) Chitin biosynthesis inhibitors, type 0, such as, for example, bistrifluron, chlorfluazuron, diflubenzuron, flucycloxuron, flufenoxuron, hexaflumuron, lufenuron, novaluron, noviflumuron, teflubenzuron and triflumuron.
(16) Chitin biosynthesis inhibitors, type 1, for example buprofezin.
(17) Moulting inhibitors (in particular for Diptera, i.e. dipterans) such as, for example, cyromazine.
(18) Ecdysone receptor agonists such as, for example, chromafenozide, halofenozide, methoxyfenozide and tebufenozide.
(19) Octopaminergic agonists such as, for example, amitraz.
(20) Complex-III electron transport inhibitors such as, for example, hydramethylnone or acequinocyl or fluacrypyrim.
(21) Complex-I electron transport inhibitors, for example from the group of the METI acaricides, e.g. fenazaquin, fenpyroximate, pyrimidifen, pyridaben, tebufenpyrad and tolfenpyrad or rotenone (Derris).
(22) Voltage-gated sodium channel blockers, for example indoxacarb or metaflumizone.
(23) Inhibitors of acetyl-CoA carboxylase such as, for example, tetronic and tetramic acid derivatives, e.g. spirodiclofen, spiromesifen and spirotetramat.
(24) Complex-IV electron transport inhibitors such as, for example, phosphines, e.g. aluminium phosphide, calcium phosphide, phosphine and zinc phosphide or cyanide.
(25) Complex II electron transport inhibitors, such as, for example, cyenopyrafen and cyflumetofen.
(28) Ryanodine receptor effectors, such as, for example, diamides, e.g. chlorantraniliprole, cyantraniliprole and flubendiamide,
further active compounds such as, for example, afidopyropen, azadirachtin, benclothiaz, benzoximate, bifenazate, bromopropylate, chinomethionat, cryolite,
dicofol, diflovidazin, fluensulfone, flometoquin, flufenerim, flufenoxystrobin, flufiprole, fluopyram, flupyradifurone, fufenozide, heptafluthrin, imidaclothiz, iprodione, meperfluthrin, paichongding, pyflubumide, pyrifluquinazon, pyriminostrobin, tetramethylfluthrin and iodomethane; furthermore preparations based on Bacillus firmus (1-1582, BioNeem, Votivo), and also the following compounds: 3-bromo-N-{2-bromo-4-chloro-6-[(1-cyclopropylethyl)carbamoyl]phenyl}-1-(3-chloropyridin-2-yl)-1H-pyrazole-5-carboxamide (known from WO2005/077934) and 1-{2-fluoro-4-methyl-5-[(2,2,2-trifluoroethyl)sulphinyl]phenyl}-3-(trifluoromethyl)-1H-1,2,4-triazole-5-amine (known from WO2006/043635), {1'-[(2E)-3-(4-chlorophenyl)prop-2-en-1-yl]-5-fluorospiro[indol-3,4'-piperidin]-1(2H)-yl}(2-chloropyridin-4-yl)methanone (known from WO2003/106457), 2-chloro-N-[2-11-[(2E)-3-(4-chlorophenyl)prop-2-en-1-yl]piperidin-4-yl}-4-(trifluoromethyl)phenyl]isonicotinamide (known from WO2006/003494), 3-(2,5-dimethylphenyl)-4-hydroxy-8-methoxy-1,8-diazaspiro[4.5]dec-3-en-2-one (known from WO2009/049851), 3-(2,5-dimethylphenyl)-8-methoxy-2-oxo-1,8-diazaspiro[4.5]dec-3-en-4-yl-ethylcarbonate (known from WO2009/049851), 4-(but-2-yn-1-yloxy)-6-(3,5-dimethylpiperidin-1-yl)-5-fluoropyrimidine (known from WO2004/099160), 4-(but-2-yn-1-yloxy)-6-(3-chlorophenyl)pyrimidine (known from WO2003/076415), PF1364 (CAS Reg. No. 1204776-60-2), 4-[5-(3,5-dichlorophenyl)-5-(trifluoromethyl)-4,5-dihydro-1,2-oxazol-3-yl]-2-methyl-N-{2-oxo-2-[(2,2,2-trifluoroethyl)amino]ethyl}benzamide (known from WO2005/085216), 4-{5-[3-chloro-5-(trifluoromethyl)phenyl]-5-(trifluoromethyl)-4,5-dihydro-1,2-oxazol-3-yl}-N-{2-oxo-2-[(2,2,2-trifluoroethyl)amino]ethyl}-1-naphthamide (known from WO2009/002809), methyl 2-[2-({ [3-bromo-1-(3-chloropyridin-2-yl)-1H-pyrazol-5-yl]carbonyl}amino)-5-chloro-3-methylbenzoyl]-2-methylhydrazinecarboxylate (known from WO2005/085216), methyl 2-[2-({[3-bromo-1-(3-chloropyridin-2-yl)-1H-pyrazol-5-yl]carbonyl}amino)-5-cyano-3-methylbenzoyl]-2-ethylhydrazinecarboxylate (known from WO2005/085216), methyl 2-[2-({[3-bromo-1-(3-chloropyridin-2-yl)-1H-pyrazol-5-yl]carbonyl}amino)-5-cyano-3-methylbenzoyl]-2-methylhydrazinecarboxylate (known from WO2005/085216), methyl 2-[3,5-dibromo-2-({[3-bromo-1-(3-chloropyridin-2-yl)-1H-pyrazol-5-yl]carbonyl}amino)benzoyl]-2-ethylhydrazinecarboxylate (known from WO2005/085216), 1-(3-chloropyridin-2-yl)-N-[4-cyano-2-methyl-6-(methylcarbamoyl)phenyl]-3-1[5-(trifluoromethyl)-2H-tetrazol-2-yl]methyl}-1H-pyrazole-5-carboxamide (known from WO2010/069502), N-[2-(5-amino-1,3,4-thiadiazol-2-yl)-4-chloro-6-methylphenyl]-3-bromo-1-(3-chloropyridin-2-yl)-1H-pyrazole-5-carboxamide (known from CN102057925), 3-chloro-N-(2-cyanopropan-2-yl)-N-[4-(1,1,1,2,3,3,3-heptafluoropropan-2-yl)-2-methylphenyl]phthalamide (known from WO2012/034472), 8-chloro-N-[(2-chloro-5-methoxyphenyl)sulphonyl]-6-(trifluoromethyl)imidazo[1,2-a]pyridine-2-carboxamide (known from WO2010/129500), 4-[5-(3,5-dichlorophenyl)-5-(trifluoromethyl)-4,5-dihydro-1,2-oxazol-3-yl]-2-methyl-N-(1-oxidothietan-3-yl)benzamide (known from WO2009/080250), N-[(2E)-1-[(6-chloropyridin-3-yl)methyl]pyridin-2(1H)-ylidene]-2,2,2-trifluoroacetamide (known from WO2012/029672), 1-[(2-chloro-1,3-thiazol-5-yl)methyl]-4-oxo-3-phenyl-4H-pyrido[1,2-a]pyrimidin-1-ium-2-olate (known from WO2009/099929), 1-[(6-chloropyridin-3-yl)methyl]-4-oxo-3-phenyl-4H-pyrido[1,2-a]pyrimidin-1-ium-2-olate (known from WO2009/099929), (5S,8R)-1-[(6-chloropyridin-3-yl)methyl]-9-nitro-2,3,5,6,7,8-hexahydro-1H-5,8-epoxyimidazo[1,2-a]azepine (known from WO2010/069266), (2E)-1-[(6-chloropyridin-3-yl)methyl]-N'-nitro-2-pentylidenehydrazinecarboximidamide (known from WO2010/060231), 4-(3-{2,6-dichloro-4-[(3,3-dichloroprop-2-en-1-yl)oxy]phenoxy}propoxy)-2-methoxy-6-(trifluoromethyl)pyrimidine (known from CN101337940), N-[2-(tert-butylcarbamoyl)-4-chloro-6-methylphenyl]-1-(3-chloropyridin-2-yl)-3-(fluoromethoxy)-1H-pyrazole-5-carboxamide (known from WO2008/134969).

### Fungicides

The active compounds specified herein by their common name are known and described, for example, in "Pesticide Manual" or on the Internet (for example: http://www.alanwood.net/pesticides).
(1) Inhibitors of ergosterol biosynthesis such as, for example, (1.1) aldimorph, (1.2) azaconazole, (1.3) bitertanol, (1.4) bromuconazole, (1.5) cyproconazole, (1.6) diclobutrazole, (1.7) difenoconazole, (1.8) diniconazole, (1.9) diniconazole-M, (1.10) dodemorph, (1.11) dodemorph acetate, (1.12) epoxiconazole, (1.13) etaconazole, (1.14) fenarimol, (1.15) fenbuconazole, (1.16) fenhexamid, (1.17) fenpropidin, (1.18) fenpropimorph, (1.19) fluquinconazole, (1.20) flurprimidol, (1.21) flusilazole, (1.22) flutriafole, (1.23) furconazole, (1.24) furconazole-cis, (1.25) hexaconazole, (1.26) imazalil, (1.27) imazalil sulphate, (1.28) imibenconazole, (1.29) ipconazole, (1.30) metconazole, (1.31) myclobutanil, (1.32) naftifin, (1.33) nuarimol, (1.34) oxpoconazole, (1.35) paclobutrazole, (1.36) pefurazoate, (1.37) penconazole, (1.38) piperalin, (1.39) prochloraz, (1.40) propiconazole, (1.41) prothioconazole, (1.42) pyributicarb, (1.43) pyrifenox, (1.44) quinconazole, (1.45) simeconazole, (1.46) spiroxamine, (1.47) tebuconazole, (1.48) terbinafin, (1.49) tetraconazole, (1.50) triadimefon, (1.51) triadimenol, (1.52) tridemorph, (1.53) triflumizole, (1.54) triforine, (1.55) triticonazole, (1.56) uniconazole, (1.57) uniconazole-P, (1.58) viniconazole, (1.59) voriconazole, (1.60) 1-(4-chlorophenyl)-2-(1H-1,2,4-triazol-1-yl)cycloheptanol, (1.61) methyl 1-(2,2-dimethyl-2,3-dihydro-1H-inden-1-yl)-1H-imidazole-5-carboxylate, (1.62) N'-{5-(difluoromethyl)-2-methyl-4-[3-(trimethylsilyl)propoxy]phenyl}-N-ethyl-N-methylimidoformamide, (1.63) N-ethyl-N-methyl-N'- {2-methyl-5-(trifluoromethyl)-4-[3-(trimethylsilyl)propoxy]phenyl}imidoformamide and (1.64) O-[1-(4-methoxyphenoxy)-3,3-dimethylbutan-2-yl]-1H-imidazole-1-carbothioate, (1.65) pyrisoxazole.
(2) Respiration inhibitors (respiratory chain inhibitors) such as, for example, (2.1) bixafen, (2.2) boscalid, (2.3) carboxin, (2.4) diflumetorim, (2.5) fenfuram, (2.6) fluopyram, (2.7) flutolanil, (2.8) fluxapyroxad, (2.9) furametpyr, (2.10) furmecyclox, (2.11) isopyrazam mixture of the syn-epimeric racemate 1RS,4SR,9RS and the anti-empimeric racemate 1RS,4SR,9SR, (2.12) isopyrazam (anti-epimeric racemate), (2.13) isopyrazam (anti-epimeric enantiomer 1R,4S,9S), (2.14) isopyrazam (anti-epimeric enantiomer 1S,4R,9R), (2.15) isopyrazam (syn-epimeric racemate 1RS,4SR,9RS), (2.16) isopyrazam (syn-epimeric enantiomer 1R,4S,9R), (2.17) isopyrazam (syn-epimeric enantiomer 1S,4R,9S), (2.18) mepronil, (2.19) oxycarboxin, (2.20) penflufen, (2.21) penthiopyrad, (2.22) sedaxane, (2.23) thifluzamide, (2.24) 1-methyl-N-[2-(1,1,2,2-tetrafluoroethoxy)phenyl]-3-(trifluoromethyl)-1H-pyrazole-4-carboxamide, (2.25) 3-(difluoromethyl)-1-methyl-N-[2-(1,1,2,2-tetrafluoroethoxy)phenyl]-1H-pyrazole-4-carboxamide, (2.26) 3-(difluoromethyl)-N-[4-fluoro-2-(1,1,2,3,3,3-hexafluoropropoxy)phenyl]-1-methyl-1H-pyrazole-4-carboxamide, (2.27) N-[1-(2,4-dichlorophenyl)-1-methoxypropan-2-yl]-3-(difluoromethyl)-1-methyl-1H-pyrazole-4-carboxamide, (2.28) 5,8-difluoro-N-[2-(2-fluoro-4-{[4-(trifluoromethyl)pyridin-2-yl]oxy}phenyl)ethyl]quinazoline-4-amine, (2.29) benzovindiflupyr, (2.30) N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide and (2.31) N-[(1R,4S)-9-(dichloromethylene)-1,2,3,4-tetrahydro-1,4-methanonaphthalen-5-yl]-3-(difluoromethyl)-1-methyl-1H-pyrazole-4-carboxamide, (2.32) 3-(difluoromethyl)-1-methyl-N-(1,1,3-trimethyl-2,3-dihydro-1H-inden-4-yl)-1H-pyrazole-4-carboxamide, (2.33) 1,3,5-trimethyl-N-(1,1,3-trimethyl-2,3-dihydro-1H-inden-4-yl)-1H-pyrazole-4-carboxamide, (2.34) 1-methyl-3-(trifluoromethyl)-N-(1,1,3-trimethyl-2,3-dihydro-1H-inden-4-yl)-1H-pyrazole-4-carboxamide, (2.35) 1-methyl-3-(trifluoromethyl)-N-[(3R)-1,1,3-trimethyl-2,3-dihydro-1H-inden-4-yl]-1H-pyrazole-4-carboxamide, (2.36) 1-methyl-3-(trifluoromethyl)-N-[(3S)-1,1,3-trimethyl-2,3-dihydro-1H-inden-4-yl]-1H-pyrazole-4-carboxamide, (2.37) 3-(difluoromethyl)-1-methyl-N-[(3S)-1,1,3-trimethyl-2,3-dihydro-1H-inden-4-yl]-1H-pyrazole-4-carboxamide, (2.38) 3-(difluoromethyl)-1-methyl-N-[(3R)-1,1,3-trimethyl-2,3-dihydro-1H-inden-4-yl]-1H-pyrazole-4-carboxamide, (2.39) 1,3,5-trimethyl-N-[(3R)-1,1,3-trimethyl-2,3-dihydro-1H-inden-4-yl]-1H-pyrazole-4-carboxamide, (2.40) 1,3,5-trimethyl-N-[(3S)-1,1,3-trimethyl-2,3-dihydro-1H-inden-4-yl]-1H-pyrazole-4-carboxamide, (2.41) benodanil, (2.42) 2-chloro-N-(1,1,3-trimethyl-2,3-dihydro-1H-inden-4-yl)pyridine-3-carboxamide, (2.43) isofetamid
(3) Respiration inhibitors (respiratory chain inhibitors) acting on complex III of the respiratory chain such as, for example, (3.1) ametoctradin, (3.2) amisulbrom, (3.3) azoxystrobin, (3.4) cyazofamid, (3.5) coumethoxystrobin, (3.6) coumoxystrobin, (3.5) dimoxystrobin, (3.8) enestroburin, (3.9) famoxadone, (3.10) fenamidone, (3.11) flufenoxystrobin, (3.12) fluoxastrobin, (3.13) kresoxim-methyl, (3.14) metominostrobin, (3.15) orysastrobin, (3.16) picoxystrobin, (3.17) pyraclostrobin, (3.18) pyrametostrobin, (3.19) pyraoxystrobin, (3.20) pyribencarb, (3.21) triclopyricarb, (3.22) trifloxystrobin, (3.23) (2E)-2-(2-{[6-(3-chloro-2-methylphenoxy)-5-fluoropyrimidin-4-yl]oxy}phenyl)-2-(methoxyimino)-N-methylethanamide, (3.24) (2E)-2-(methoxyimino)-N-methyl-2-(2-{[({(1E)-1-[3-(trifluoromethyl)phenyl]ethylidene}amino)oxy]methyl}phenyl)ethanamide, (3.25) (2E)-2-(methoxyimino)-N-methyl-2-{2-[(E)-({1-[3-(trifluoromethyl)phenyl]ethoxy}imino)methyl]phenyl}ethanamide, (3.26) (2E)-2-{2-[({[(1E)-1-(3-{[(E)-1-fluoro-2-phenylethenyl]oxy}phenyl)ethylidene]amino}oxy)methyl]phenyl}-2-(methoxyimino)-N-methylethanamide, (3.27) (2E)-2-{2-[({[(2E,3E)-4-(2,6-dichlorophenyl)but-3-en-2-ylidene]amino}oxy)methyl]phenyl} -2-(methoxyimino)-N-methylethanamide, (3.28) 2-chloro-N-(1,1,3-trimethyl-2,3-dihydro-1H-inden-4-yl)pyridine-3-carboxamide, (3.29) 5-methoxy-2-methyl-4-(2-{[({(1E)-1-[3-(trifluoromethyl)phenyl]ethylidene}amino)oxy]methyl}phenyl)-2,4-dihydro-3H-1,2,4-triazol-3-one, (3.30) methyl (2E)-2-{2-[({cyclopropyl[(4-methoxyphenyl)imino]methyl}sulphanyl)methyl]phenyl}-3-methoxyprop-2-enoate, (3.31)N-(3-ethyl-3,5,5-trimethylcyclohexyl)-3-(formylamino)-2-hydroxybenzamide, (3.32) 2-{2-[(2,5-dimethylphenoxy)methyl]phenyl}-2-methoxy-N-methylacetamide,
(4) inhibitors of mitosis and cell division such as, for example, (4.1) benomyl, (4.2) carbendazim, (4.3) chlorfenazole, (4.4) diethofencarb, (4.5) ethaboxam, (4.6) fluopicolid, (4.7) fuberidazole, (4.8) pencycuron, (4.9) thiabendazole, (4.10) thiophanate-methyl, (4.11) thiophanate, (4.12) zoxamide, (4.13) 5-chloro-7-(4-methylpiperidin-1-yl)-6-(2,4,6-trifluorophenyl)[1,2,4]triazolo[1,5-a]pyrimidine and (4.14) 3-chloro-5-(6-chloropyridin-3-yl)-6-methyl-4-(2,4,6-trifluorophenyl)pyridazine.
(5) Compounds having multisite activity such as, for example, (5.1) Bordeaux mixture, (5.2) captafol, (5.3) captan, (5.4) chlorothalonil, (5.5) copper preparations such as copper hydroxide, (5.6) copper naphthenate, (5.7) copper oxide, (5.8) copper oxychloride, (5.9) copper sulphate, (5.10) dichlofluanid, (5.11) dithianon, (5.12) dodine, (5.13) dodine free base, (5.14) ferbam, (5.15) fluorfolpet, (5.16) folpet, (5.17) guazatine, (5.18) guazatine acetate, (5.19) iminoctadine, (5.20) iminoctadine albesilate, (5.21) iminoctadine triacetate, (5.22) mancopper, (5.23) mancozeb, (5.24) maneb, (5.25) metiram, (5.26) zinc metiram, (5.27) copper-oxine, (5.28) propamidine, (5.29) propineb, (5.30) sulphur and sulphur preparations such as, for example calcium polysulphide, (5.31) thiram, (5.32) tolylfluanid, (5.33) zineb, (5.34) ziram and (5.35) anilazine.
(6) Resistance inducers such as, for example, (6.1) acibenzolar-S-methyl, (6.2) isotianil, (6.3) probenazole, (6.4) tiadinil and (6.5) laminarin.
(7) Inhibitors of amino acid and protein biosynthesis such as, for example, (7.1), (7.2) blasticidin-S, (7.3) cyprodinil, (7.4) kasugamycin, (7.5) kasugamycin hydrochloride hydrate, (7.6) mepanipyrim, (7.7) pyrimethanil, (7.8) 3-(5-fluoro-3,3,4,4-tetramethyl-3,4-dihydroisoquinolin-1-yl)quinoline and (7.9) oxytetracycline and (7.10) streptomycin.
(8) ATP production inhibitors such as, for example, (8.1) fentin acetate, (8.2) fentin chloride, (8.3) fentin hydroxide and (8.4) silthiofam.
(9) Inhibitors of cell wall synthesis such as, for example, (9.1) benthiavalicarb, (9.2) dimethomorph, (9.3) flumorph, (9.4) iprovalicarb, (9.5) mandipropamid, (9.6) polyoxins, (9.7) polyoxorim, (9.8) validamycin A, (9.9) valifenalate and (9.10) polyoxin B.
(10) Inhibitors of lipid and membrane synthesis such as, for example, (10.1) biphenyl, (10.2) chlorneb, (10.3) dicloran, (10.4) edifenphos, (10.5) etridiazole, (10.6) iodocarb, (10.7) iprobenfos, (10.8) isoprothiolane, (10.9) propamocarb, (10.10) propamocarb hydrochloride, (10.11) prothiocarb,, (10.12) pyrazophos, (10.13) quintozene, (10.14) tecnazene and (10.15) tolclofos-methyl.
(11) Melanin biosynthesis inhibitors, for example (11.1) carpropamid, (11.2) diclocymet, (11.3) fenoxanil, (11.4) fthalide, (11.5) pyroquilon, (11.6) tricyclazole and (11.7) 2,2,2-trifluoroethyl {3-methyl-1-[(4-methylbenzoyl)amino]butan-2-yl}carbamate.
(12) Inhibitors of nucleic acid synthesis such as, for example, (12.1) benalaxyl, (12.2) benalaxyl-M (kiralaxyl), (12.3) bupirimate, (12.4) clozylacon, (12.5) dimethirimol, (12.6) ethirimol, (12.7) furalaxyl, (12.8) hymexazole, (12.9) metalaxyl, (12.10) metalaxyl-M (mefenoxam), (12.11) ofurace, (12.12) oxadixyl, (12.13) oxolinic acid and (12.14) octhilinone.
(13) Signal transduction inhibitors such as, for example, (13.1) chlozolinate, (13.2) fenpiclonil, (13.3) fludioxonil, (13.4) iprodione, (13.5) procymidone, (13.6) quinoxyfen, (13.7) vinclozolin and (13.8) proquinazid.
(14) Decouplers such as, for example, (14.1) binapacryl, (14.2) dinocap, (14.3) ferimzone, (14.4) fluazinam and (14.5) meptyldinocap.
(15) Further compounds such as, for example, (15.1) benthiazole, (15.2) bethoxazine, (15.3) capsimycin, (15.4) carvone, (15.5) chinomethionat, (15.6) pyriofenone (chlazafenone), (15.7) cufraneb, (15.8) cyflufenamid, (15.9) cymoxanil, (15.10) cyprosulfamide, (15.11) dazomet, (15.12) debacarb, (15.13) dichlorophen, (15.14) diclomezine, (15.15) difenzoquat, (15.16) difenzoquat methylsulphate, (15.17) diphenylamine, (15.18) EcoMate, (15.19) fenpyrazamine, (15.20) flumetover, (15.21) fluorimid, (15.22) flusulfamide, (15.23) flutianil, (15.24) fosetyl-aluminium, (15.25) fosetyl-calcium, (15.26) fosetyl-sodium, (15.27) hexachlorobenzene, (15.28) irumamycin, (15.29) methasulfocarb, (15.30) methyl isothiocyanate, (15.31) metrafenone, (15.32) mildiomycin, (15.33) natamycin, (15.34) nickel dimethyldithiocarbamate, (15.35) nitrothal-isopropyl, (15.36) octhilinone, (15.37) oxamocarb, (15.38) oxyfenthiin, (15.39) pentachlorophenol and its salts, (15.40) phenothrin, (15.41) phosphoric acid and its salts, (15.42) propamocarb-fosetylate, (15.43) propanosine-sodium, (15.44) pyrimorph, (15.45) (2E)-3-(4-tert-butylphenyl)-3-(2-chloropyridin-4-yl)-1-(morpholin-4-yl)prop-2-en-1-one, (15.46) (2Z)-3-(4-tert-butylphenyl)-3-(2-chloropyridin-4-yl)-1-(morpholin-4-yl)prop-2-en-1-one, (15.47) pyrrolnitrin, (15.48) tebufloquin, (15.49) tecloftalam, (15.50) tolnifanide, (15.51) triazoxide, (15.52) trichlamide, (15.53) zarilamid, (15.54) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (15.55) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.56) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.57) 1-(4-14-[5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.58) 1-(4-methoxyphenoxy)-3,3-dimethylbutan-2-yl 1H-imidazole-1-carboxylate, (15.59) 2,3,5,6-tetrachloro-4-(methylsulphonyl)pyridine, (15.60) 2,3-dibutyl-6-chlorothieno[2,3-d]pyrimidin-4(3H)-one, (15.61) 2,6-dimethyl-1H,5H-[1,4]dithiino[2,3-c:5,6-c']dipyrrole-1,3,5,7(2H,6H)-tetrone, (15.62) 2-[5-methyl-3-(trifluoromethyl)-1H-pyrazol-1-yl]-1-(4-{4-[(SR)-5-phenyl-4,5-dihydro-1,2-oxazol-3-yl]-1,3-thiazol-2-yl}piperidin-1-yl)ethanone, (15.63) 2-[5-methyl-3-(trifluoromethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanone, (15.64) 2-[5-methyl-3-(trifluoromethyl)-1H-pyrazol-1-yl]-1-{4-[4-(5-phenyl-4,5-dihydro-1,2-oxazol-3-yl)-1,3-thiazol-2-yl]piperidin-1-yl}ethanone, (15.65) 2-butoxy-6-iodo-3-propyl-4H-chromen-4-one, (15.66) 2-chloro-5-[2-chloro-1-(2,6-difluoro-4-methoxyphenyl)-4-methyl-1H-imidazol-5-yl]pyridine, (15.67) 2-phenylphenol and salts, (15.68) 3-(4,4,5-trifluoro-3,3-dimethyl-3,4-dihydroisoquinolin-1-yl)quinoline, (15.69) 3,4,5-trichloropyridine-2,6-dicarbonitrile, (15.70) 3-chloro-5-(4-chlorophenyl)-4-(2,6-difluorophenyl)-6-methylpyridazine, (15.71) 4-(4-chlorophenyl)-5-(2,6-difluorophenyl)-3,6-dimethylpyridazine, (15.72) 5-amino-1,3,4-thiadiazole-2-thiol, (15.73) 5-chloro-N'-phenyl-N'-(prop-2-yn-1-yl)thiophene-2-sulphonohydrazide, (15.74) 5-fluoro-2-[(4-fluorobenzyl)oxy]pyrimidine-4-amine, (15.75) 5-fluoro-2-[(4-methylbenzyl)oxy]pyrimidine-4-amine, (15.76) 5-methyl-6-octyl[1,2,4]triazolo[1,5-a]pyrimidine-7-amine, (15.77) ethyl (2Z)-3-amino-2-cyano-3-phenylacrylate, (15.78) N'-(4-{[3-(4-chlorobenzyl)-1,2,4-thiadiazol-5-yl]oxy}-2,5-dimethylphenyl)-N-ethyl-N-methylimidoformamide, (15.79) N-(4-chlorobenzyl)-3-[3-methoxy-4-(prop-2-yn-1-yloxy)phenyl]propanamide, (15.80) N- [(4-chlorophenyl)(cyano)methyl] -3- [3 -methoxy-4-(prop-2-yn-1-yloxy)phenyl]propanamide, (15.81) N-[(5-bromo-3-chloropyridin-2-yl)methyl]-2,4-dichloronicotinamide, (15.82) N-[1-(5-bromo-3-chloropyridin-2-yl)ethyl]-2,4-dichloronicotinamide, (15.83) N-[1-(5-bromo-3-chloropyridin-2-yl)ethyl]-2-fluoro-4-iodonicotinamide, (15.84) N-{(E)-[(cyclopropylmethoxy)imino][6-(difluoromethoxy)-2,3-difluorophenyl]methyl}-2-phenylacetamide, (15.85) N-{(Z)-[(cyclopropylmethoxy)imino][6-(difluoromethoxy)-2,3-difluorophenyl]methyl}-2-phenylacetamide, (15.86) N'-{4-[(3-tert-butyl-4-cyano-1,2-thiazol-5-yl)oxy]-2-chloro-5-methylphenyl}-N-ethyl-N-methylimidoformamide, (15.87) N-methyl-2-(1-{[5-methyl-3-(trifluoromethyl)-1H-pyrazol-1-yl]acetyl}piperidin-4-yl)-N-(1,2,3,4-tetrahydronaphthalen-1-yl)-1,3-thiazole-4-carboxamide, (15.88) N-methyl-2-(1-{[5-methyl-3-(trifluoromethyl)-1H-pyrazol-1-yl]acetyl}piperidin-4-yl)-N-[(1R)-1,2,3,4-tetrahydronaphthalen-1-yl]-1,3-thiazole-4-carboxamide, (15.89) N-methyl-2-(1-{[5-methyl-3-(trifluoromethyl)-1H-pyrazol-1-yl]acetyl}piperidin-4-yl)-N-[(1S)-1,2,3,4-tetrahydronaphthalen-1-yl]-1,3-thiazole-4-carboxamide, (15.90) pentyl {6-[({[(1-methyl-1H-tetrazol-5-yl)(phenyl)methylene]amino}oxy)methyl]pyridin-2-yl}carbamate, (15.91) phenazine-1-carboxylic acid, (15.92) quinolin-8-ol, (15.93) quinolin-8-ol sulphate (2:1), (15.94) tert-butyl {6-[({[(1-methyl-1H-tetrazol-5-yl)(phenyl)methylene]amino}oxy)methyl]pyridin-2-yl}carbamate, (15.95) 1-methyl-3-(trifluoromethyl)-N-[2'-(trifluoromethyl)biphenyl-2-yl]-1H-pyrazole-4-carboxamide, (15.96) N-(4'-chlorobiphenyl-2-yl)-3-(difluoromethyl)-1-methyl-1H-pyrazole-4-carboxamide, (15.97) N-(2',4'-dichlorobiphenyl-2-yl)-3-(difluoromethyl)-1-methyl-1H-pyrazole-4-carboxamide, (15.98) 3-(difluoromethyl)-1-methyl-N-[4'-(trifluoromethyl)biphenyl-2-yl]-1H-pyrazole-4-carboxamide, (15.99) N-(2',5'-difluorobiphenyl-2-yl)-1-methyl-3-(trifluoromethyl)-1H-pyrazole-4-carboxamide, (15.100) 3-(difluoromethyl)-1-methyl-N-[4'-(prop-1-yn-1-yl)biphenyl-2-yl]-1H-pyrazole-4-carboxamide, (15.101) 5-fluoro-1 ,3 -dimethyl-N- [4'-(prop-1 -yn-1 -yl)biphenyl-2-yl] -1H-pyrazole-4-carboxamide, (15.102) 2-chloro-N-[4'-(prop-1-yn-1-yl)biphenyl-2-yl]nicotinamide, (15.103) 3-(difluoromethyl)-N-[4'-(3,3-dimethylbut-1-yn-1-yl)biphenyl-2-yl]-1-methyl-1H-pyrazole-4-carboxamide, (15.104) N-[4'-(3,3-dimethylbut-1-yn-1-yl)biphenyl-2-yl]-5-fluoro-1,3-dimethyl-1H-pyrazole-4-carboxamide, (15.105) 3-(difluoromethyl)-N-(4'-ethynylbiphenyl-2-yl)-1-methyl-1H-pyrazole-4-carboxamide, (15.106) N-(4'-ethynylbiphenyl-2-yl)-5-fluoro-1,3-dimethyl-1H-pyrazole-4-carboxamide, (15.107) 2-chloro-N-(4'-ethynylbiphenyl-2-yl)nicotinamide, (15.108) 2-chloro-N- [4'-(3,3 -dimethylbut-1-yn-1-yl)biphenyl-2-yl]nicotinamide, (15.109) 4-(difluoromethyl)-2-methyl-N-[4'-(trifluoromethyl)biphenyl-2-yl]-1,3-thiazole-5-carboxamide, (15.110) 5-fluoro-N-[4'-(3-hydroxy-3-methylbut-1-yn-1-yl)biphenyl-2-yl]-1,3-dimethyl-1H-pyrazole-4-carboxamide, (15.111) 2-chloro-N-[4'-(3-hydroxy-3-methylbut-1-yn-1-yl)biphenyl-2-yl]nicotinamide, (15.112) 3-(difluoromethyl)-N-[4'-(3-methoxy-3-methylbut-1-yn-1-yl)biphenyl-2-yl]-1-methyl-1H-pyrazole-4-carboxamide, (15.113) 5-fluoro-N-[4'-(3-methoxy-3-methylbut-1-yn-1-yl)biphenyl-2-yl]-1,3-dimethyl-1H-pyrazole-4-carboxamide, (15.114) 2-chloro-N- [4'-(3-methoxy-3-methylbut-1-yn-1-yl)biphenyl-2-yl]nicotinamide, (15.115) (5-bromo-2-methoxy-4-methylpyridin-3-yl)(2,3,4-trimethoxy-6-methylphenyl)methanone, (15.116) N-[2-(4-{ [3-(4-chlorophenyl)prop-2-yn-1-yl]oxy}-3-methoxyphenyl)ethyl]-N2-(methylsulphonyl)valinamide, (15.117) 4-oxo-4-[(2-phenylethyl)amino]butanoic acid, (15.118) but-3-yn-1-yl{6-[({[(Z)-(1-methyl-1H-tetrazol-5-yl)(phenyl)methylene]amino}oxy)methyl]pyridin-2-yl}carbamate, (15.119) 4-amino-5-fluoropyrimidin-2-ol (tautomeric form: 4-amino-5-fluoropyrimidin-2(1H)-one), (15.120) propyl 3,4,5-trihydroxybenzoate, (15.121) 1,3-dimethyl-N-(1,1,3-trimethyl-2,3-dihydro-1H-inden-4-yl)-1H-pyrazole-4-carboxamide, (15.122) 1,3-dimethyl-N-[(3R)-1,1,3-trimethyl-2,3-dihydro-1H-inden-4-yl]-1H-pyrazole-4-carboxamide, (15.123) 1,3-dimethyl-N-[(3S)-1,1,3-trimethyl-2,3-dihydro-1H-inden-4-yl]-1H-pyrazole-4-carboxamide, (15.124) [3-(4-chloro-2-fluorophenyl)-5-(2,4-difluorophenyl)-1,2-oxazol-4-yl](pyridin-3-yl)methanol, (15.125) (S)-[3-(4-chloro-2-fluorophenyl)-5-(2,4-difluorophenyl)-1,2-oxazol-4-yl](pyridin-3-yl)methanol, (15.126) (R)-[3-(4-chloro-2-fluorophenyl)-5-(2,4-difluorophenyl)-1,2-oxazol-4-yl](pyridin-3-yl)methanol, (15.127) 2-{[3-(2-chlorophenyl)-2-(2,4-difluorophenyl)oxiran-2-yl]methyl}-2,4-dihydro-3H-1,2,4-triazole-3-thione, (15.128) 1-{[3-(2-chlorophenyl)-2-(2,4-difluorophenyl)oxiran-2-yl]methyl}-1H-1,2,4-triazol-5-yl thiocyanate, (15.129) 5-(allylsulfanyl)-1-{[3-(2-chlorophenyl)-2-(2,4-difluorophenyl)oxiran-2-yl]methyl}-1H-1,2,4-triazole, (15.130) 2-[1-(2,4-dichlorophenyl)-5-hydroxy-2,6,6-trimethylheptan-4-yl]-2,4-dihydro-3H-1,2,4-triazole-3-thione, (15.131) 2-{[rel(2R,3S)-3-(2-chlorophenyl)-2-(2,4-difluorophenyl)oxiran-2-yl]methyl}-2,4-dihydro-3H-1,2,4-triazole-3-thione, (15.132) 2-{ [rel(2R,3R)-3-(2-chlorophenyl)-2-(2,4-difluorophenyl)oxiran-2-yl]methyl}-2,4-dihydro-3H-1,2,4-triazole-3-thione, (15.133) 1-{[rel(2R,3S)-3-(2-chlorophenyl)-2-(2,4-difluorophenyl)oxiran-2-yl]methyl}-1H-1,2,4-triazol-5-yl thiocyanate, (15.134) 1-{ [rel(2R,3R)-3-(2-chlorophenyl)-2-(2,4-difluorophenyl)oxiran-2-yl] methyl} -1H-1,2,4-triazol-5 -yl thiocyanate, (15.135) 5-(allylsulphanyl)-1-{[rel(2R,3S)-3-(2-chlorophenyl)-2-(2,4-difluorophenyl)oxiran-2-yl]methyl}-1H-1,2,4-triazole, (15.136) 5-(allylsulphanyl)-1-{[rel(2R,3R)-3-(2-chlorophenyl)-2-(2,4-difluorophenyl)oxiran-2-yl]methyl}-1H-1,2,4-triazole, (15.137) 2-[(2S,4S,5S)-1-(2,4-dichlorophenyl)-5-hydroxy-2,6,6-trimethylheptan-4-yl]-2,4-dihydro-3H-1,2,4-triazole-3-thione, (15.138) 2-[(2R,4S,5S)-1-(2,4-dichlorophenyl)-5-hydroxy-2,6,6-trimethylheptan-4-yl]-2,4-dihydro-3H-1,2,4-triazole-3-thione, (15.139) 2-[(2R,4R,5R)-1-(2,4-dichlorophenyl)-5-hydroxy-2,6,6-trimethylheptan-4-yl]-2,4-dihydro-3H-1,2,4-triazole-3-thione, (15.140) 2-[(2S,4R,5R)-1-(2,4-dichlorophenyl)-5-hydroxy-2,6,6-trimethylheptan-4-yl]-2,4-dihydro-3H-1,2,4-triazole-3-thione, (15.141) 2-[(2S,4S,5R)-1-(2,4-dichlorophenyl)-5-hydroxy-2,6,6-trimethylheptan-4-yl]-2,4-dihydro-3H-1,2,4-triazole-3-thione, (15.142) 2-[(2R,4S,5R)-1-(2,4-dichlorophenyl)-5-hydroxy-2,6,6-trimethylheptan-4-yl]-2,4-dihydro-3H-1,2,4-triazole-3-thione, (15.143) 2-[(2R,4R,5S)-1-(2,4-dichlorophenyl)-5-hydroxy-2,6,6-trimethylheptan-4-yl]-2,4-dihydro-3H-1,2,4-triazole-3-thione, (15.144) 2-[(2S,4R,5S)-1-(2,4-dichlorophenyl)-5-hydroxy-2,6,6-trimethylheptan-4-yl]-2,4-dihydro-3H-1,2,4-triazole-3-thione, (15.145) 2-fluoro-6-(trifluoromethyl)-N-(1,1,3-trimethyl-2,3-dihydro-1H-inden-4-yl)benzamide, (15.146) 2-(6-benzylpyridin-2-yl)quinazoline, (15.147) 2-[6-(3-fluoro-4-methoxyphenyl)-5-methylpyridin-2-yl]quinazoline, (15.148) 3-(4,4-difluoro-3,3-dimethyl-3,4-dihydroisoquinolin-1 -yl)quinoline, (15.149) abscisic acid, (15.150) 3-(difluoromethyl)-N-methoxy-1-methyl-N-[1-(2,4,6-trichlorophenyl)propan-2-yl]-1H-pyrazole-4-carboxamide, (15.151) N'-[5-bromo-6-(2,3-dihydro-1H-inden-2-yloxy)-2-methylpyridin-3-yl]-N-ethyl-N-methylimidoformamide, (15.152) N'-{5-bromo-6-[1-(3,5-difluorophenyl)ethoxy]-2-methylpyridin-3 -yl} -N-ethyl-N-methylimidoformamide, (15.153) N'-{5-bromo-6-[(1R)-1-(3,5-difluorophenyl)ethoxy]-2-methylpyridin-3-yl}-N-ethyl-N-methylimidoformamide, (15.154) N'-{5-bromo-6-[(1S)-1-(3,5-difluorophenyl)ethoxy]-2-methylpyridin-3-yl}-N-ethyl-N-methylimidoformamide, (15.155) N'-{5-bromo-6-[(cis-4-isopropylcyclohexyl)oxy]-2-methylpyridin-3-yl}-N-ethyl-N-methylimidoformamide, (15.156) N'-{5-bromo-6-[(trans-4-isopropylcyclohexyl)oxy] -2-methylpyridin-3 -yl} -N-ethyl-N-methylimidoformamide, (15.157) N-cyclopropyl-3-(difluoromethyl)-5-fluoro-N-(2-isopropylbenzyl)-1-methyl-1H-pyrazole-4-carboxamide, (15.158) N-cyclopropyl-N-(2-cyclopropylbenzyl)-3 -(difluoromethyl)-5-fluoro-1-methyl-1H-pyrazole-4-carboxamide, (15.159) N-(2-tert-butylbenzyl)-N-cyclopropyl-3-(difluoromethyl)-5-fluoro-1-methyl-1H-pyrazole-4-carboxamide, (15.160) N-(5-chloro-2-ethylbenzyl)-N-cyclopropyl-3-(difluoromethyl)-5-fluoro-1-methyl-1H-pyrazole-4-carboxamide, (15.161) N-(5-chloro-2-isopropylbenzyl)-N-cyclopropyl-3-(difluoromethyl)-5-fluoro-1-methyl-1H-pyrazole-4-carboxamide, (15.162) N-cyclopropyl-3-(difluoromethyl)-N-(2-ethyl-5-fluorobenzyl)-5-fluoro-1-methyl-1H-pyrazole-4-carboxamide, (15.163) N-cyclopropyl-3-(difluoromethyl)-5-fluoro-N-(5-fluoro-2-isopropylbenzyl)-1-methyl-1H-pyrazole-4-carboxamide, (15.164) N-cyclopropyl-N-(2-cyclopropyl-5-fluorobenzyl)-3-(difluoromethyl)-5-fluoro-1-methyl-1H-pyrazole-4-carboxamide, (15.165) N-(2-cyclopentyl-5-fluorobenzyl)-N-cyclopropyl-3-(difluoromethyl)-5-fluoro-1-methyl-1H-pyrazole-4-carboxamide, (15.166) N-cyclopropyl-3-(difluoromethyl)-5-fluoro-N-(2-fluoro-6-isopropylbenzyl)-1-methyl-1H-pyrazole-4-carboxamide, (15.167) N-cyclopropyl-3-(difluoromethyl)-N-(2-ethyl-5-methylbenzyl)-5-fluoro-1-methyl-1H-pyrazole-4-carboxamide, (15.168) N-cyclopropyl-3-(difluoromethyl)-5-fluoro-N-(2-isopropyl-5-methylbenzyl)-1-methyl-1H-pyrazole-4-carboxamide, (15.169) N-cyclopropyl-N-(2-cyclopropyl-5-methylbenzyl)-3-(difluoromethyl)-5-fluoro-1-methyl-1H-pyrazole-4-carboxamide, (15.170) N-(2-tert-butyl-5-methylbenzyl)-N-cyclopropyl-3-(difluoromethyl)-5-fluoro-1-methyl-1H-pyrazole-4-carboxamide, (15.171) N-[5-chloro-2-(trifluoromethyl)benzyl]-N-cyclopropyl-3-(difluoromethyl)-5-fluoro-1-methyl-1H-pyrazole-4-carboxamide, (15.172) N-cyclopropyl-3-(difluoromethyl)-5-fluoro-1-methyl-N-[5-methyl-2-(trifluoromethyl)benzyl]-1H-pyrazole-4-carboxamide, (15.173) N- [2-chloro-6-(trifluoromethyl)benzyl]-N-cyclopropyl-3-(difluoromethyl)-5-fluoro-1-methyl-1H-pyrazole-4-carboxamide, (15.174) N-[3-chloro-2-fluoro-6-(trifluoromethyl)benzyl]-N-cyclopropyl-3-(difluoromethyl)-5-fluoro-1-methyl-1H-pyrazole-4-carboxamide, (15.175) N-cyclopropyl-3-(difluoromethyl)-N-(2-ethyl-4,5-dimethylbenzyl)-5-fluoro-1-methyl-1H-pyrazole-4-carboxamide, (15.176) N-cyclopropyl-3-(difluoromethyl)-5-fluoro-N-(2-isopropylbenzyl)-1-methyl-1H-pyrazol-4-carbothioamide, (15.177) 3-(difluoromethyl)-N-(7-fluoro-1,1,3-trimethyl-2,3-dihydro-1H-inden-4-yl)-1-methyl-1H-pyrazole-4-carboxamide, (15.178) 3-(difluoromethyl)-N-[(3R)-7-fluoro-1,1,3-trimethyl-2,3-dihydro-1H-inden-4-yl]-1-methyl-1H-pyrazole-4-carboxamide, (15.179) 3-(difluoromethyl)-N-[(3S)-7-fluoro-1,1,3-trimethyl-2,3-dihydro-1H-inden-4-yl]-1-methyl-1H-pyrazole-4-carboxamide, (15.180) N'-(2,5-dimethyl-4-phenoxyphenyl)-N-ethyl-N-methylimidoformamide, (15.181) N'-{4-[(4,5-dichloro-1,3-thiazol-2-yl)oxy]-2,5-dimethylphenyl}-N-ethyl-N-methylimidoformamide, (15.182) N-(4-chloro-2,6-difluorophenyl)-4-(2-chloro-4-fluorophenyl)-1,3-dimethyl-1H-pyrazole-5-amine. All mixing components mentioned in classes (1) to (15) can, if they are capable on the basis of their functional groups, optionally form salts with suitable bases or acids.

### Biological pesticides as mixing components

The compounds of the formula (I) can be combined with biological pesticides.

Biological pesticides comprise in particular bacteria, fungi, yeasts, plant extracts and products formed by microorganisms, including proteins and secondary metabolites.

Biological pesticides comprise bacteria such as spore-forming bacteria, root-colonising bacteria and bacteria which act as biological insecticides, fungicides or nematicides.

Examples of such bacteria which are employed or can be used as biological pesticides are:
*Bacillus amyloliquefaciens,* strain FZB42 (DSM 231179), or *Bacillus cereus,* in particular *B. cereus* strain CNCM 1-1562 or *Bacillus firmus,* strain 1-1582 (Accession number CNCM 1-1582) or *Bacillus pumilus,* in particular strain GB34 (Accession No. ATCC 700814) and strain QST2808 (Accession No. NRRL B-30087), or *Bacillus subtilis,* in particular strain GB03 (Accession No. ATCC SD-1397), or *Bacillus subtilis* strain QST713 (Accession No. NRRL B-21661) or *Bacillus subtilis strain* OST 30002 (Accession No. NRRL B-50421) *Bacillus thuringiensis,* in particular *B. thuringiensis* subspecies *israelensis* (serotype H-14), strain AM65-52 (Accession No. ATCC 1276), or *B. thuringiensis subsp. aizawai,* in particular strain ABTS-1857 (SD-1372), or *B. thuringiensis subsp. kurstaki* strain HD-1, or *B. thuringiensis subsp. tenebrionis* strain NB 176 (SD-5428), *Pasteuria penetrans, Pasteuria spp.* (Rotylenchulus reniformis nematode)-PR3 (Accession Number ATCC SD-5834), *Streptomyces microflavus* strain AQ6121 (= QRD 31.013, NRRL B-50550), *Streptomyces galbus* strain AQ 6047 (Acession Number NRRL 30232).

Examples of fungi and yeasts which are employed or can be used as biological pesticides are:
*Beauveria bassiana,* in particular strain ATCC 74040, *Coniothyrium minitans,* in particular strain CON/M/91-8 (Accession No. DSM-9660), *Lecanicillium spp.,* in particular strain HRO LEC 12, *Lecanicillium lecanii,* (formerly known as *Verticillium lecanii*), in particular strain KV01, *Metarhizium anisopliae,* in particular strain F52 (DSM3884/ ATCC 90448), *Metschnikowia fructicola,* in particular strain NRRL Y-30752, *Paecilomyces fumosoroseus* (now: *Isaria fumosorosea*), in particular strain IFPC 200613, or strain Apopka 97 (Accesion No. ATCC 20874), *Paecilomyces lilacinus,* in particular P. *lilacinus* strain 251 (AGAL 89/030550), *Talaromyces flavus,* in particular strain V117b, *Trichoderma atroviride,* in particular strain SC1 (Accession Number CBS 122089), *Trichoderma harzianum,* in particular *T. harzianum rifai T39.* (Accession Number CNCM 1-952).

Examples of viruses which are employed or can be used as biological pesticides are:
*Adoxophyes orana* (summer fruit tortrix) granulosis virus (GV), *Cydia pomonella* (codling moth) granulosis virus (GV), *Helicoverpa armigera* (cotton bollworm) nuclear polyhedrosis virus (NPV), *Spodoptera exigua* (beet armyworm) mNPV, *Spodoptera frugiperda* (fall armyworm) mNPV, *Spodoptera littoralis* (African cotton leafworm) NPV.

Also included are bacteria and fungi which are added as 'inoculant' to plants or plant parts or plant organs and which, by virtue of their particular properties, promote plant growth and plant health. Examples which may be mentioned are:
*Agrobacterium spp., Azorhizobium caulinodans, Azospirillum spp., Azotobacter spp., Bradyrhizobium spp., Burkholderia spp.,* in particular *Burkholderia cepacia* (formerly known as *Pseudomonas cepacia*), *Gigaspora spp.,* or *Gigaspora monosporum, Glomus spp., Laccaria spp., Lactobacillus buchneri, Paraglomus spp., Pisolithus tinctorus, Pseudomonas spp., Rhizobium spp.,* in particular *Rhizobium trifolii, Rhizopogon spp., Scleroderma spp., Suillus spp., Streptomyces spp.*

Examples of plant extracts and products formed by microorganisms including proteins and secondary metabolites which are employed or can be used as biological pesticides are:
Allium sativum, Artemisia absinthium, azadirachtin, Biokeeper WP, Cassia nigricans, Celastrus angulatus, Chenopodium anthelminticum, chitin, Armour-Zen, Dryopteris filix-mas, Equisetum arvense, Fortune Aza, Fungastop, Heads Up (Chenopodium quinoa saponin extract), Pyrethrum/Pyrethrins, Quassia amara, Quercus, Quillaja, Regalia, "Requiem ™ Insecticide", rotenone, ryania/ryanodine, Symphytum officinale, Tanacetum vulgare, thymol, Triact 70, TriCon, Tropaeulum majus, Urtica dioica, Veratrin, Viscum album, Brassicaceae extract, in particular oilseed rape powder or mustard powder.

### Safener as mixing components

The compounds of the formula (I) can be combined with safeners such as, for example, benoxacor, cloquintocet (-mexyl), cyometrinil, cyprosulfamide, dichlormid, fenchlorazole (-ethyl), fenclorim, flurazole, fluxofenim, furilazole, isoxadifen (-ethyl), mefenpyr (-diethyl), naphthalic anhydride, oxabetrinil, 2-methoxy-N-({4-[(methylcarbamoyl)amino]phenyl}sulphonyl)benzamide (CAS 129531-12-0), 4-(dichloroacetyl)-1-oxa-4-azaspiro[4.5]decane (CAS 71526-07-3), 2,2,5-trimethyl-3-(dichloroacetyl)-1,3-oxazolidine (CAS 52836-31-4).

### Plants and plant parts

All plants and plant parts can be treated in accordance with the invention. Here, plants are to be understood to mean all plants and plant parts such as wanted and unwanted wild plants or crop plants (including naturally occurring crop plants), for example cereals (wheat, rice, triticale, barley, rye, oats), maize, soya bean, potato, sugar beet, sugar cane, tomatoes, peas and other vegetable species, cotton, tobacco, oilseed rape, and also fruit plants (with the fruits apples, pears, citrus fruits and grapevines). Crop plants can be plants which can be obtained by conventional breeding and optimization methods or by biotechnological and genetic engineering methods or combinations of these methods, including the transgenic plants and including the plant varieties which can or cannot be protected by varietal property rights. Plant parts should be understood to mean all parts and organs of the plants above and below ground, such as shoot, leaf, flower and root, examples given being leaves, needles, stalks, stems, flowers, fruit bodies, fruits and seeds, and also tubers, roots and rhizomes. Parts of plants also include harvested plants and vegetative and generative propagation material, for example seedlings, tubers, rhizomes, cuttings and seeds.

Treatment according to the invention of the plants and plant parts with the compounds of the formula (I) is carried out directly or by allowing the compounds to act on the surroundings, environment or storage space by the customary treatment methods, for example by immersion, spraying, evaporation, fogging, scattering, painting on, injection and, in the case of propagation material, in particular in the case of seeds, also by applying one or more coats.

As already mentioned above, it is possible to treat all plants and their parts according to the invention. In a preferred embodiment, wild plant species and plant cultivars, or those obtained by conventional biological breeding methods, such as crossing or protoplast fusion, and also parts thereof, are treated. In a further preferred embodiment, transgenic plants and plant cultivars obtained by genetic engineering methods, if appropriate in combination with conventional methods (genetically modified organisms), and parts thereof are treated. The term "parts" or "parts of plants" or "plant parts" has been explained above. The invention is used with particular preference to treat plants of the respective commercially customary cultivars or those that are in use. Plant cultivars are to be understood as meaning plants having new properties ("traits") and which have been obtained by conventional breeding, by mutagenesis or by recombinant DNA techniques. They can be cultivars, varieties, bio- or genotypes.

### Transgenic plant, seed treatment and integration events

The transgenic plants or plant cultivars (those obtained by genetic engineering) which are to be treated with preference in accordance with the invention include all plants which, through the genetic modification, received genetic material which imparts particular advantageous useful properties ("traits") to these plants. Examples of such properties are better plant growth, increased tolerance to high or low temperatures, increased tolerance to drought or to levels of water or soil salinity, enhanced flowering performance, easier harvesting, accelerated ripening, higher yields, higher quality and/or a higher nutritional value of the harvested products, better storage life and/or processability of the harvested products. Further and particularly emphasized examples of such properties are increased resistance of the plants against animal and microbial pests, such as against insects, arachnids, nematodes, mites, slugs and snails owing, for example, to toxins formed in the plants, in particular those formed in the plants by the genetic material from Bacillus thuringiensis (for example by the genes CryIA(a), CryIA(b), CryIA(c), CryIIA, CryIIIA, CryIIIB2, Cry9c Cry2Ab, Cry3Bb and CrylF and also combinations thereof), furthermore increased resistance of the plants against phytopathogenic fungi, bacteria and/or viruses owing, for example, to systemic acquired resistance (SAR), systemin, phytoalexins, elicitors and also resistance genes and correspondingly expressed proteins and toxins, and also increased tolerance of the plants to certain herbicidally active compounds, for example imidazolinones, sulphonylureas, glyphosate or phosphinothricin (for example the "PAT" gene). The genes which impart the desired traits in question may also be present in combinations with one another in the transgenic plants. Examples of transgenic plants which may be mentioned are the important crop plants, such as cereals (wheat, rice, triticale, barley, rye, oats), maize, soya beans, potatoes, sugar beet, sugar cane, tomatoes, peas and other types of vegetable, cotton, tobacco, oilseed rape and also fruit plants (with the fruits apples, pears, citrus fruits and grapes), with particular emphasis being given to maize, soya beans, wheat, rice, potatoes, cotton, sugar cane, tobacco and oilseed rape. Traits which are particularly emphasized are the increased resistance of the plants to insects, arachnids, nematodes and slugs and snails.

### Crop protection - types of treatment

The treatment of the plants and plant parts with the compounds of the formula (I) is carried out directly or by action on their surroundings, habitat or storage space using customary treatment methods, for example by dipping, spraying, atomizing, irrigating, evaporating, dusting, fogging, broadcasting, foaming, painting, spreading-on, injecting, watering (drenching), drip irrigating and, in the case of propagation material, in particular in the case of seed, furthermore as a powder for dry seed treatment, a solution for liquid seed treatment, a water-soluble powder for slurry treatment, by incrusting, by coating with one or more coats, etc. It is furthermore possible to apply the compounds of the formula (I) by the ultra-low volume method or to inject the application form or the compound of the formula (I) itself into the soil.

A preferred direct treatment of the plants is foliar application, i.e. the compounds of the formula (I) are applied to the foliage, where treatment frequency and the application rate should be adjusted according to the level of infestation with the pest in question.

In the case of systemically active compounds, the compounds of the formula (I) also access the plants via the root system. The plants are then treated by the action of the compounds of the formula (I) on the habitat of the plant. This may be done, for example, by drenching, or by mixing into the soil or the nutrient solution, i.e. the locus of the plant (e.g. soil or hydroponic systems) is impregnated with a liquid form of the compounds of the formula (I), or by soil application, i.e. the compounds of the formula (I) according to the invention are introduced in solid form (e.g. in the form of granules) into the locus of the plants. In the case of paddy rice crops, this can also be done by metering the compound of the formula (I) in a solid application form (for example as granules) into a flooded paddy field.

### Treatment of seed

The control of animal pests by treating the seed of plants has been known for a long time and is the subject of continuous improvements. However, the treatment of seed entails a series of problems which cannot always be solved in a satisfactory manner. Thus, it is desirable to develop methods for protecting the seed and the germinating plant which dispense with, or at least reduce considerably, the additional application of pesticides during storage, after sowing or after emergence of the plants. It is furthermore desirable to optimize the amount of active compound employed in such a way as to provide optimum protection for the seed and the germinating plant from attack by animal pests, but without damaging the plant itself by the active compound employed. In particular, methods for the treatment of seed should also take into consideration the intrinsic insecticidal or nematicidal properties of pest-resistant or -tolerant transgenic plants in order to achieve optimum protection of the seed and also the germinating plant with a minimum of pesticides being employed.

The present invention therefore in particular also relates to a method for the protection of seed and germinating plants, from attack by pests, by treating the seed with one of the compounds of the formula (I). The method according to the invention for protecting seed and germinating plants against attack by pests furthermore comprises a method where the seed is treated simultaneously in one operation or sequentially with a compound of the formula (I) and a mixing component. It also comprises a method where the seed is treated at different times with a compound of the formula (I) and a mixing component.

The invention likewise relates to the use of the compounds of the formula (I) for the treatment of seed for protecting the seed and the resulting plant from animal pests.

Furthermore, the invention relates to seed which has been treated with a compound of the formula (I) according to the invention so as to afford protection from animal pests. The invention also relates to seed which has been treated simultaneously with a compound of the formula (I) and a mixing component. The invention furthermore relates to seed which has been treated at different times with a compound of the formula (I) and a mixing component. In the case of seed which has been treated at different points in time with a compound of the formula (I) and a mixing component, the individual substances may be present on the seed in different layers. Here, the layers comprising a compound of the formula (I) and mixing components may optionally be separated by an intermediate layer. The invention also relates to seed where a compound of the formula (I) and a mixing component have been applied as component of a coating or as a further layer or further layers in addition to a coating.

Furthermore, the invention relates to seed which, after the treatment with a compound of the formula (I), is subjected to a film-coating process to prevent dust abrasion on the seed.

One of the advantages encountered with a systemically acting compound of the formula (I) is the fact that, by treating the seed, not only the seed itself but also the plants resulting therefrom are, after emergence, protected against animal pests. In this manner, the immediate treatment of the crop at the time of sowing or shortly thereafter can be dispensed with.

It has to be considered a further advantage that by treatment of the seed with a compound of the formula (I), germination and emergence of the treated seed may be enhanced.

It is likewise to be considered advantageous that compounds of the formula (I) can be used in particular also for transgenic seed.

Furthermore, compounds of the formula (I) can be employed in combination with compositions or compounds of signalling technology, leading to better colonization by symbionts such as, for example, rhizobia, mycorrhizae and/or endophytic bacteria or fungi, and/or to optimized nitrogen fixation.

The compounds of the formula (I) are suitable for protection of seed of any plant variety which is used in agriculture, in the greenhouse, in forests or in horticulture. In particular, this takes the form of seed of cereals (for example wheat, barley, rye, millet and oats), corn, cotton, soya beans, rice, potatoes, sunflowers, coffee, tobacco, canola, oilseed rape, beets (for example sugarbeets and fodder beets), peanuts, vegetables (for example tomatoes, cucumbers, bean, cruciferous vegetables, onions and lettuce), fruit plants, lawns and ornamental plants. The treatment of the seed of cereals (such as wheat, barley, rye and oats), maize, soya beans, cotton, canola, oilseed rape and rice is of particular importance.

As already mentioned above, the treatment of transgenic seed with a compound of the formula (I) is also of particular importance. This takes the form of seed of plants which, as a rule, comprise at least one heterologous gene which governs the expression of a polypeptide with in particular insecticidal and/or nematicidal properties. The heterologous genes in transgenic seed can originate from microorganisms such as Bacillus, Rhizobium, Pseudomonas, Serratia, Trichoderma, Clavibacter, Glomus or Gliocladium. The present invention is particularly suitable for the treatment of transgenic seed which comprises at least one heterologous gene originating from Bacillus sp. It is particularly preferably a heterologous gene derived from Bacillus thuringiensis.

In the context of the present invention, the compound of the formula (I) is applied to the seed. Preferably, the seed is treated in a state in which it is stable enough to avoid damage during treatment. In general, the seed may be treated at any point in time between harvest and sowing. The seed usually used has been separated from the plant and freed from cobs, shells, stalks, coats, hairs or the flesh of the fruits. For example, it is possible to use seed which has been harvested, cleaned and dried down to a moisture content which allows storage. Alternatively, it is also possible to use seed which, after drying, has been treated with, for example, water and then dried again, for example priming. In the case of rice seed, it is also possible to use seed which has been soaked, for example in water to a certain stage of the rice embryo ('pigeon breast stage'), stimulating the germination and a more uniform emergence.

When treating the seed, care must generally be taken that the amount of the compound of the formula (I) applied to the seed and/or the amount of further additives is chosen in such a way that the germination of the seed is not adversely affected, or that the resulting plant is not damaged. This must be ensured particularly in the case of active compounds which can exhibit phytotoxic effects at certain application rates.

In general, the compounds of the formula (I) are applied to the seed in a suitable formulation. Suitable formulations and processes for seed treatment are known to the person skilled in the art.

The compounds of the formula (I) can be converted to the customary seed dressing formulations, such as solutions, emulsions, suspensions, powders, foams, slurries or other coating compositions for seed, and also ULV formulations.

These formulations are prepared in a known manner, by mixing the compounds of the formula (I) with customary additives such as, for example, customary extenders and also solvents or diluents, colorants, wetting agents, dispersants, emulsifiers, antifoams, preservatives, secondary thickeners, adhesives, gibberellins and also water.

Colorants which may be present in the seed-dressing formulations which can be used in accordance with the invention are all colorants which are customary for such purposes. It is possible to use either pigments, which are sparingly soluble in water, or dyes, which are soluble in water. Examples include the dyes known by the names Rhodamine B, C.I. Pigment Red 112 and C.I. Solvent Red 1.

Useful wetting agents which may be present in the seed dressing formulations usable in accordance with the invention are all substances which promote wetting and which are conventionally used for the formulation of agrochemically active compounds. Preference is given to using alkylnaphthalenesulphonates, such as diisopropyl- or diisobutylnaphthalenesulphonates.

Useful dispersants and/or emulsifiers which may be present in the seed dressing formulations usable in accordance with the invention are all nonionic, anionic and cationic dispersants conventionally used for the formulation of active agrochemical ingredients. Preference is given to using nonionic or anionic dispersants or mixtures of nonionic or anionic dispersants. Suitable nonionic dispersants include in particular ethylene oxide/propylene oxide block polymers, alkylphenol polyglycol ethers and tristryrylphenol polyglycol ethers, and the phosphated or sulphated derivatives thereof. Suitable anionic dispersants are in particular lignosulphonates, polyacrylic acid salts and arylsulphonate/formaldehyde condensates.

Antifoams which may be present in the seed dressing formulations usable in accordance with the invention are all foam-inhibiting substances conventionally used for the formulation of active agrochemical ingredients. Preference is given to using silicone antifoams and magnesium stearate.

Preservatives which may be present in the seed dressing formulations usable in accordance with the invention are all substances usable for such purposes in agrochemical compositions. Examples include dichlorophene and benzyl alcohol hemiformal.

Secondary thickeners which may be present in the seed dressing formulations usable in accordance with the invention are all substances which can be used for such purposes in agrochemical compositions. Cellulose derivatives, acrylic acid derivatives, xanthan, modified clays and finely divided silica are preferred.

Adhesives which may be present in the seed dressing formulations usable in accordance with the invention are all customary binders usable in seed dressing products. Polyvinylpyrrolidone, polyvinyl acetate, polyvinyl alcohol and tylose may be mentioned as being preferred.

Gibberellins which can be present in the seed-dressing formulations which can be used in accordance with the invention are preferably the gibberellins A1, A3 (= gibberellic acid), A4 and A7; gibberellic acid is especially preferably used. The gibberellins are known (cf. R. Wegler "Chemie der Pflanzenschutz- and Schädlingsbekämpfungsmittel", vol. 2, Springer Verlag, 1970, pp. 401-412).

The seed dressing formulations usable in accordance with the invention can be used to treat a wide variety of different kinds of seed either directly or after prior dilution with water. For instance, the concentrates or the preparations obtainable therefrom by dilution with water can be used to dress the seed of cereals, such as wheat, barley, rye, oats, and triticale, and also the seed of maize, rice, oilseed rape, peas, beans, cotton, sunflowers, soya beans and beets, or else a wide variety of different vegetable seed. The seed dressing formulations usable in accordance with the invention, or the dilute use forms thereof, can also be used to dress seed of transgenic plants.

For treatment of seed with the seed dressing formulations usable in accordance with the invention, or the use forms prepared therefrom by adding water, all mixing units usable customarily for the seed dressing are useful. Specifically, the procedure in the seed dressing is to place the seed into a mixer, operated batch-wise or continously, to add the particular desired amount of seed dressing formulations, either as such or after prior dilution with water, and to mix everything until the formulation is distributed homogeneously on the seed. If appropriate, this is followed by a drying operation.

The application rate of the seed dressing formulations usable in accordance with the invention can be varied within a relatively wide range. It is guided by the particular content of the compounds of the formula (I) in the formulations and by the seed. The application rates of the compound of the formula (I) are generally between 0.001 and 50 g per kilogram of seed, preferably between 0.01 and 15 g per kilogram of seed.

### Animal health

In the animal health field, i.e. in the field of veterinary medicine, the compounds of the formula (I) are active against animal parasites, in particular ectoparasites or endoparasites. The term endoparasites includes in particular helminths and protozoans, such as coccidia. Ectoparasites are typically and preferably arthropods, in particular insects and acarids.

In the field of veterinary medicine the compounds of the formula (I) are suitable, with favourable homeotherm toxicity, for controlling parasites which occur in animal breeding and animal husbandry in livestock, breeding, zoo, laboratory, experimental and domestic animals. They are active against all or specific stages of development of the parasites.

Agricultural livestock include, for example, mammals, such as sheep, goats, horses, donkeys, camels, buffaloes, rabbits, reindeers, fallow deers, and in particular cattle and pigs; or poultry such as turkeys, ducks, geese, and in particular chickens; fish and crustaceans, for example in aquaculture; and also insects such as bees.

Domestic animals include, for example, mammals, such as hamsters, guinea pigs, rats, mice, chinchillas, ferrets and in particular dogs, cats, cage birds, reptiles, amphibians and aquarium fish.

According to a preferred embodiment, the compounds of the formula (I) are administered to mammals.

According to another preferred embodiment, the compounds of the formula (I) are administered to birds, namely cage birds and in particular poultry.

By using the compounds of the formula (I) to control animal parasites, it is intended to reduce or prevent illness, cases of deaths and performance reductions (in the case of meat, milk, wool, hides, eggs, honey and the like), so that more economical and simpler animal keeping is made possible and better animal well-being is achievable.

The term "control" or "controlling" as used herein with regard to the animal health field, means that the compounds of the formula (I) are effective in reducing the incidence of the respective parasite in an animal infected with such parasites to innocuous levels. More specifically, "controlling", as used herein, means that the compound of the formula (I) is effective in killing the respective parasite, inhibiting its growth, or inhibiting its proliferation.

Arthropods include:
from the order of the Anoplurida, for example Haematopinus spp., Linognathus spp., Pediculus spp., Phtirus spp., Solenopotes spp.; from the order of the Mallophagida and the suborders Amblycerina and Ischnocerina, for example Trimenopon spp., Menopon spp., Trinoton spp., Bovicola spp., Werneckiella spp., Lepikentron spp., Damalina spp., Trichodectes spp., Felicola spp.; from the order of the Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; from the order of the Siphonapterida, for example Pulex spp., Ctenocephalides spp., Tunga spp., Xenopsylla spp., Ceratophyllus spp.;
from the order of the Heteropterida, for example Cimex spp., Triatoma spp., Rhodnius spp., Panstrongylus spp.; as well as nuisance and hygiene pests from the order of the Blattarida.

Arthropods furthermore include:
from the subclass of the Acari (Acarina) and the order of the Metastigmata, for example from the family of argasidae like Argas spp., Ornithodorus spp., Otobius spp., from the family of Ixodidae like Ixodes spp., Amblyomma spp., Rhipicephalus (Boophilus) spp Dermacentor spp., Haemophysalis spp., Hyalomma spp., Rhipicephalus spp. (the original genus of multi-host ticks); from the order of mesostigmata like Dermanyssus spp., Ornithonyssus spp., Pneumonyssus spp., Raillietia spp., Pneumonyssus spp., Sternostoma spp., Varroa spp., Acarapis spp.; from the order of the Actinedida (Prostigmata), for example Acarapis spp., Cheyletiella spp., Ornithocheyletia spp., Myobia spp., Psorergates spp., Demodex spp., Trombicula spp., Neotrombiculla spp., Listrophorus spp.; and from the order of the Acaridida (Astigmata), for example Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Parasitic Protozoa include:
Mastigophora (Flagellata) such as, for example, Trypanosomatidae, for example, Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, such as, for example, Trichomonadidae, for example, Giardia lamblia, G. canis.;
Sarcomastigophora (Rhizopoda) such as Entamoebidae, for example, Entamoeba histolytica, Hartmanellidae, for example, Acanthamoeba sp., Harmanella sp.;
Apicomplexa (Sporozoa) such as Eimeridae, for example, Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., in particular C. parvum; such as Toxoplasmadidae, for example, Toxoplasma gondii, Hammondia heydornii, Neospora caninum, Besnoitia besnoitii; such as Sarcocystidae, for example, Sarcocystis bovicanis, S. bovihominis, S. ovicanis, S. ovifelis, S. neurona, S. spec., S. suihominis, such as Leucozoidae, for example, Leucozytozoon simondi, such as Plasmodiidae, for example, Plasmodium berghei, P. falciparum, P. malariae, P. ovale, P. vivax, P. spec., such as Piroplasmea, for example, Babesia argentina, B. bovis, B. canis, B. spec., Theileria parva, Theileria spec., such as Adeleina, for example, Hepatozoon canis, H. spec..

Pathogenic endoparasites, which are helminths, include Platyhelmintha (e.g. Monogenea, cestodes and trematodes), nematodes, Acanthocephala, and Pentastoma, including:
Monogenea: e.g.: Gyrodactylus spp., Dactylogyrus spp., Polystoma spp.;
Cestodes: from the order of the Pseudophyllidea for example: Diphyllobothrium spp., Spirometra spp., Schistocephalus spp., Ligula spp., Bothridium spp., Diplogonoporus spp.;
from the order of the Cyclophyllida for example: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: from the class of the Digenea for example: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Nematodes: Trichinellida zum Beispiel: Trichuris spp., Capillaria spp., Paracapillaria spp., Eucoleus spp., Trichomosoides spp., Trichinella spp.;
from the order of the Tylenchida for example: Micronema spp., Strongyloides spp.;
from the order of the Rhabditida for example: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
from the order of the Spirurida, for example: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: from the order of the Oligacanthorhynchida, for example: Macracanthorhynchus spp., Prosthenorchis spp.; from the order of the Polymorphida, for example: Filicollis spp.; from the order of the Moniliformida, for example: Moniliformis spp.;
from the order of the Echinorhynchida, for example, Acanthocephalus spp., Echinorhynchus spp., Leptorhynchoides spp.;
Pentastoma: from the order of the Porocephalida, for example, Linguatula spp..

In the veterinary field and in animal keeping, administration of the compounds of the formula (I) is carried out by methods generally known in the art, such as enterally, parenterally, dermally or nasally in the form of suitable preparations. Administration can be carried out prophylactically or therapeutically.

Thus, one embodiment of the present invention refers to the use of a compound of the formula (I) as medicament.

A further aspect refers to the use of a compound of the formula (I) as an antiendoparasitic agent, in particular a helminthicidal agent or antiprotozoic agent. Compounds of the formula (I) are suitable for use as an antiendoparasitic agent, in particular a helminthicidal agent or antiprotozoic agent, for example in animal husbandry, in animal breeding, in animal housing and in the hygiene sector.

A further aspect in turn relates to the use of a compound of the formula (I) as an antiectoparasitic, in particular an arthropodicide such as an insecticide or an acaricide. A further aspect relates to the use of a compound of the formula (I) as an antiectoparasitic, in particular an arthropodicide such as an insecticide or an acaricide, for example in animal husbandry, in animal breeding, in stables or in the hygiene sector.

### Vector control

The compounds of the formula (I) can also be used in vector control. For the purpose of the present invention, a vector is an arthropod, in particular an insect or arachnid, capable of transmitting pathogens such as, for example, viruses, worms, single-cell organisms and bacteria from a reservoir (plant, animal, human, etc.) to a host. The pathogens can be transmitted either mechanically (for example trachoma by non-stinging flies) to a host, or by injection (for example malaria parasites by mosquitoes) into a host.

Examples of vectors and the diseases or pathogens they transmit are:
1) Mosquitoes
   - Anopheles: malaria, filariasis;
   - Culex: Japanese encephalitis, filariasis, other viral diseases, transmission of worms;
   - Aedes: yellow fever, dengue fever, filariasis, other viral diseases;
   - Simuliidae: transmission of worms, in particular Onchocerca volvulus;
2) Lice: skin infections, epidemic typhus;
3) Fleas: plague, endemic typhus;
4) Flies: sleeping sickness (trypanosomiasis); cholera, other bacterial diseases;
5) Mites: acariosis, epidemic typhus, rickettsialpox, tularaemia, Saint Louis encephalitis, tick-borne encephalitis (TBE), Crimean-Congo haemorrhagic fever, borreliosis;
6) Ticks: borellioses such as Borrelia duttoni, tick-borne encephalitis, Q fever (Coxiella burnetii), babesioses (Babesia canis canis).

Examples of vectors in the sense of the present invention are insects, for example aphids, flies, leafhoppers or thrips, which are capable of transmitting plant viruses to plants. Other vectors capable of transmitting plant viruses are spider mites, lice, beetles and nematodes.

Further examples of vectors in the sence of the present invention are insects and arachnids such as mosquitoes, in particular of the genera Aedes, Anopheles, for example A. gambiae, A. arabiensis, A. funestus, A. dirus (malaria) and Culex, lice, fleas, flies, mites and ticks capable of transmitting pathogens to animals and/or humans.

Vector control is also possible if the compounds of the formula (I) are resistance-breaking.

Compounds of the formula (I) are suitable for use in the prevention of diseases and/or pathogens transmitted by vectors. Thus, a further aspect of the present invention is the use of compounds of the formula (I) for vector control, for example in agriculture, in horticulture, in gardens and in leisure facilities, and also in the protection of materials and stored products.

### Protection of industrial materials

The compounds of the formula (I) are suitable for protecting industrial materials against attack or destruction by insects, for example from the orders Coleoptera, Hymenoptera, Isoptera, Lepidoptera, Psocoptera and Zygentoma.

Industrial materials in the present context are understood to mean inanimate materials, such as preferably plastics, adhesives, sizes, papers and cards, leather, wood, processed wood products and coating compositions. The use of the invention for protecting wood is particularly preferred.

In a further embodiment, the compounds of the formula (I) are used together with at least one further insecticide and/or at least one fungicide.

In a further embodiment, the compounds of the formula (I) are present as a ready-to-use pesticide, i.e. they can be applied to the material in question without further modifications. Suitable further insecticides or fungicides are in particular those mentioned above.

Surprisingly, it has also been found that the compounds of the formula (I) can be employed for protecting objects which come into contact with saltwater or brackish water, in particular hulls, screens, nets, buildings, moorings and signalling systems, against fouling. Likewise, the compounds of the formula (I), alone or in combinations with other active compounds, can be used as antifouling agents.

### Control of animal pests in the hygiene sector

The compounds of the formula (I) are suitable for controlling animal pests in the hygiene sector. In particular, the invention can be applied in the domestic sector, in the hygiene sector and in the protection of stored products, especially for controlling insects, arachnids and mites encountered in enclosed spaces such as dwellings, factory halls, offices, vehicle cabins. For controlling animal pests, the compounds of the formula (I) are used alone or in combination with other active compounds and/or auxiliaries. They are preferably used in domestic insecticide products. The compounds of the formula (I) are effective against sensitive and resistant species, and against all developmental stages.

These pests include, for example, pests from the class Arachnida, from the orders Scorpiones, Araneae and Opiliones, from the classes Chilopoda and Diplopoda, from the class Insecta the order Blattodea, from the orders Coleoptera, Dermaptera, Diptera, Heteroptera, Hymenoptera, Isoptera, Lepidoptera, Phthiraptera, Psocoptera, Saltatoria or Orthoptera, Siphonaptera and Zygentoma and from the class Malacostraca the order Isopoda.

They are used, for example, in aerosols, pressure-free spray products, for example pump and atomizer sprays, automatic fogging systems, foggers, foams, gels, evaporator products with evaporator tablets made of cellulose or plastic, liquid evaporators, gel and membrane evaporators, propeller-driven evaporators, energy-free, or passive, evaporation systems, moth papers, moth bags and moth gels, as granules or dusts, in baits for spreading or in bait stations.

### Reaction Schemes

The compounds of the invention may be made by the following methods.

The synthesis route for compounds of the invention (**I**) where Z₂ represents -S(O)₁₋₂-(C₁-C₂)-alkyl, (C₁-C₂)-halogenalkyl, or nitro (NO₂), is shown below. 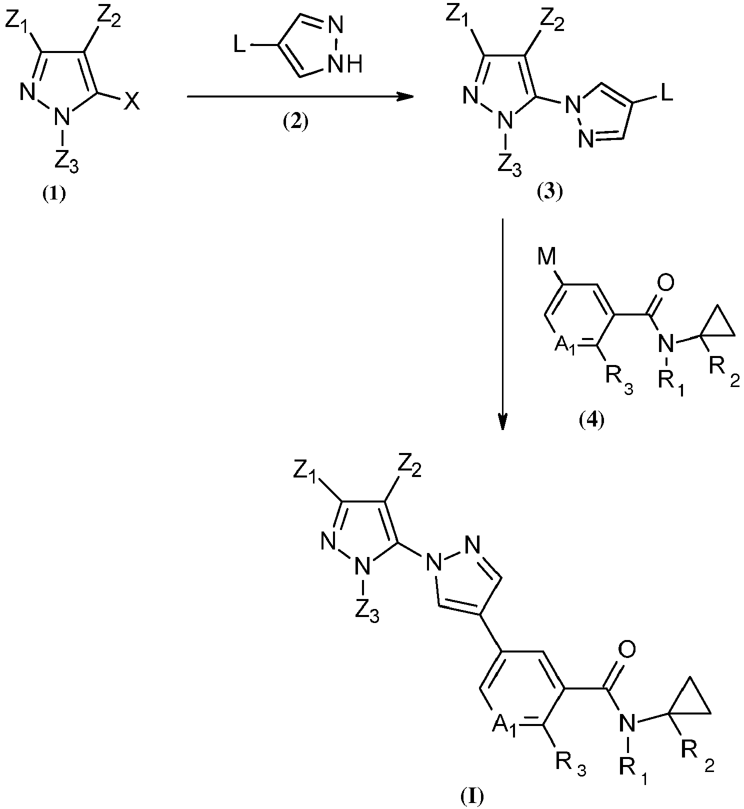 Z₁, Z₃, R₁, R₂, R₃ and A₁ represent the above described residues. L represents bromine or iodine, whereas M represents boronic acid, boronic acid esters or trifluoroboronate. Z₂ represents -S(O)₁₋₂-(C₁-C₂)-alkyl, (C₁-C₂)-halogenalkyl, or nitro (NO₂). X represents a suitable leaving group like chloride or fluoride.

Compounds of formula (**I**) where Z2 represents -S(O)₁₋₂-(C₁-C₂)-alkyl, (C₁-C₂)-halogenalkyl, or nitro (NO₂) can be synthesized by a transition metal (e.g. Palladium) catalysed reactions of a compound formula (**3**) with a compound of the formula (**4**). Those reactions may be performed in analogy to procedures described in literature [see, for example: WO2005-040110; WO2009-089508]. Compounds of the formula (**4**) are either commercially available or can be synthesized in analogy to procedures known from literature. Compounds of formula (**3**) may be prepared in a nucleophilic substitution from compounds of formula (**1**) and a corresponding pyrazole derivative (**2**). Examples for similar reactions are described in literature [see, for example: European Chemical Bulletin 2013, 2 (12), 981-984; Monatshefte fuer Chemie 1981, 112 (5), 675-678]. The general synthesis routes for compounds of formula (**1**) is described later in this chapter.

An example of a synthesis route for compounds of the invention (I) where Z₂ represents -S-(C₁-C₂)-alkyl is shown below. 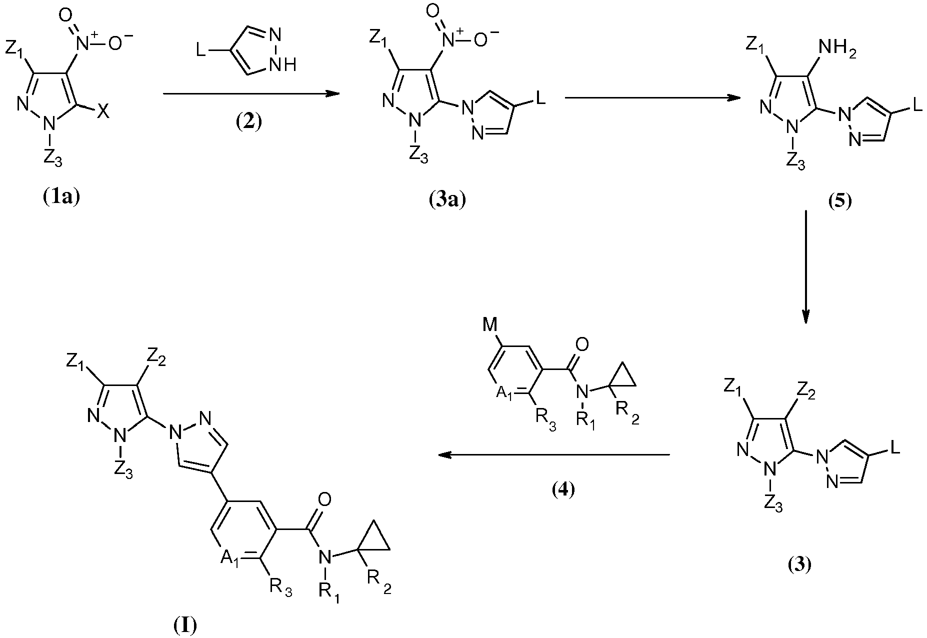 Z₁, Z₃, R₁, R₂, R₃ and A₁ represent the above described residues. Z₂ represents -S-(C₁-C₂)-alkyl, L represents bromine or iodine, whereas M represents boronic acid, boronic acid esters or trifluoroboronate. X represents a suitable leaving group like chloride or fluoride.

Compounds of formula (**I**) where Z₂ represents -S-(C₁-C₂)-alkyl can be synthesized by a transition metal (e.g. Palladium) catalysed reactions of a compound formula (**3**) with a compound of the formula (**4**). Those reactions may be performed in analogy to procedures described in literature [see, for example: WO2005-040110; WO2009-089508]. Compounds of the formula (**4**) are either commercially available or can be synthesized in analogy to procedures known from literature. Compounds of formula (**3**) may be synthesized in analogy to literature-known procedures from compounds of formula (**5**) [see, for example: WO2013-092522]. Compounds of formula (**5**) may be prepared by the reduction of compounds of formula (**3a**). Suitable reducing agents may be Tin(II)chloride or Iron(0). Examples are widely described in literature [see, for example: Fe(0) WO2012-062783 or WO2014-023258; see, for example: Sn(II)Cl₂ WO2009-056556 or WO2002-016364]. Compounds of formula (**3a**) may be prepared in a nucleophilic substitution from compounds of formula (**1a**) and a corresponding pyrazole derivative (**2**) as described above in the synthesis of (**3**). The general synthesis route for compounds of formula (**1a**) is described below.

General syntheses of the central intermediates (**1a**) - (**1e**) is shown below.

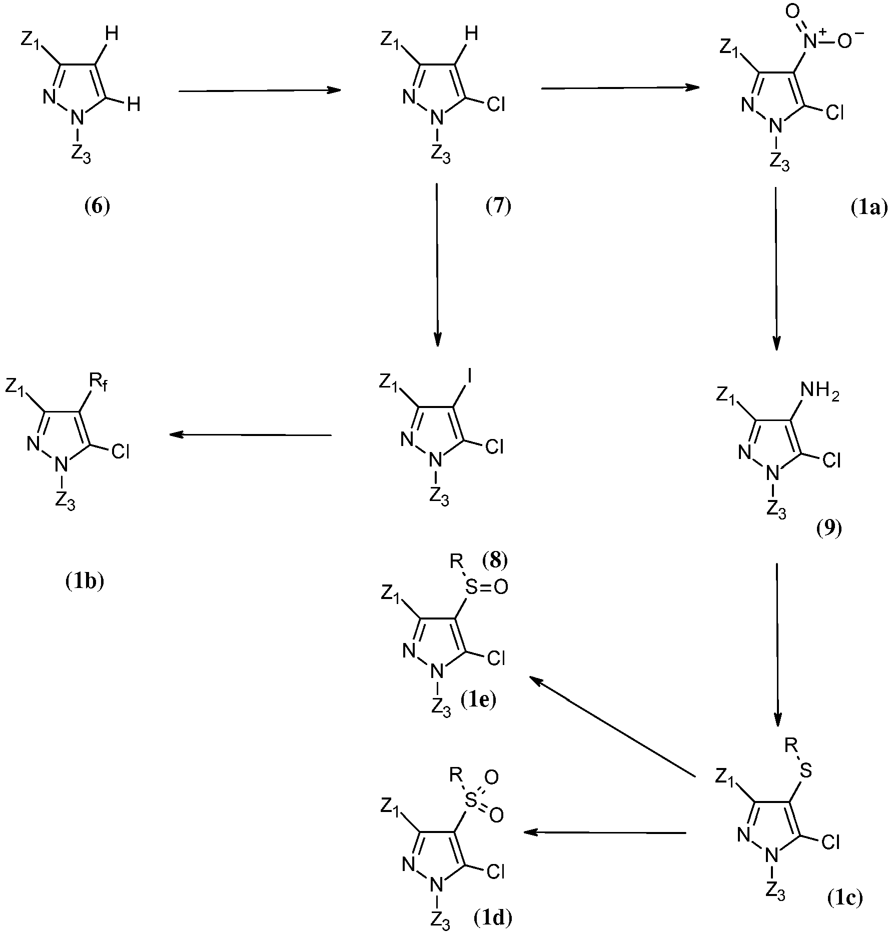

Compounds of formula (**1d**) and (**1e**) may be synthesized by oxidation from compounds of formula (**1c**)**.** Suitable oxidants are e.g. m-chlorperbenzoic acid and hydrogen peroxide. Suitable procedures are already described in literature [see, for example: oxidation to sulfone: WO2013-018804; WO2007-138050; oxidation to sulfoxide: WO2010-035915; WO2007-138050].

Compounds of formula (**1c**) may be synthesized in analogy to literature-known procedures from compounds of formula (**9**) [see, for example: WO2013-092522]. Compounds of formula (**9**) may be synthesized in analogy to literature-known procedures from compounds of formula (**1a**) as described above in the synthesis of (**5**). Compounds of formula (**1a**) may be prepared starting from compounds of formula (**7**) by a nitration reaction. Similar reactions are described in literature [see, for example: EP1987-110490; DE1985-3528478]. Compounds of formula (**7**) may be prepared starting from compounds of formula (**6**) by a chlorination reaction. Similar reactions are described in literature [see, for example: WO2012-062783; WO2011-131615].

Compounds of formula (**1b**) may be synthesized from compounds of formula (**8**). Similar reactions are described in literature [see, for example: WO2010-051926, WO2005-095351]. Compounds of formula (**8**) can be synthesized by iodination from compounds of formula (**7**). Similar reactions are described in literature [see, for example: WO2010-051926, WO2008-098104].

One preferred embodiment of the present invention refers to any of the intermediates

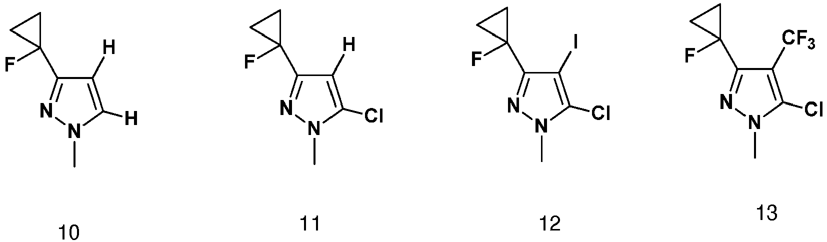

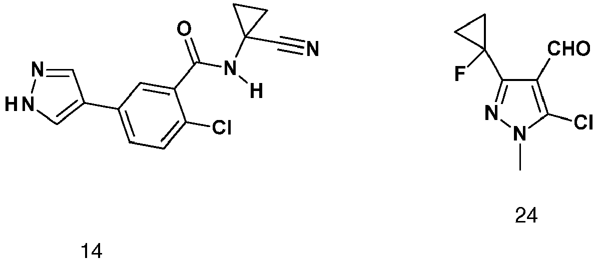

### Experimental part

### Synthesis of 2-chloro-N-(1-cyanocyclopropyl)-5-[5'-(1-fluorocyclopropyl)-2'-methyl-4'-(trifluoromethyl)-2'H-1,3'-bipyrazol-4-yl]benzamide (I-1)

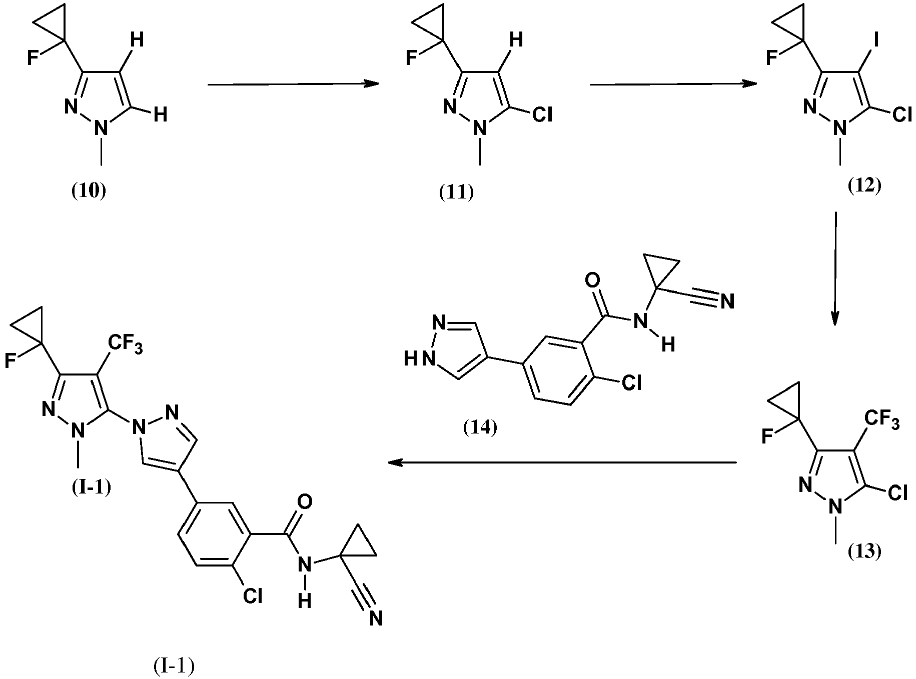

### 5-chloro-3-(1-fluorocyclopropyl)-1-methyl-1H-pyrazole (11)

840 mg (5,99 mmol) 3-(1-fluorocyclopropyl)-1-methyl-1*H*-pyrazole were dissolved in 10 mL THF abs. under argon atmosphere and cooled to -75°C. The solution was treated dropwise with 5,62 mL (8,99 mmol) n-butyllithium as 1.6 M solution in hexanes. The mixture was stirred for 30 minutes at - 75°C. 1,70 g (7.19 mmol) hexchloroethane were dissolved in 10 mL THF abs. under argon atmosphere and cooled to -75°C. The pyrazole reaction mixture was transferred slowly via Teflon canula to the cooled hexachloroethane solution. The combined reaction mixture was stirred 1h at -75°C. The reaction was quenched by the addition of saturated sodium carbonate solution. The reaction mixture was warmed to room temperature. The crude product was extracted several times with ethyl acetate. The combined organic phases were dried over MgSO₄, filtered and concentrated under vacuum (max. 35°C; > 80 mbar). The residue was purified by flash chromatography on SiO₂.

535 mg 5-chloro-3-(1-fluorocyclopropyl)-1-methyl-1*H*-pyrazole (**11**) as a 1:1 mixture with 3-(1-fluorocyclopropyl)-1-methyl-1*H*-pyrazole were isolated as yellow liquid.
GC-MS**⁾: Index = 1128, Masse (m/z) = 174 [M]⁺.
¹H-NMR (400 MHz, DMSO-d6): 6,51 (s,1H), 3,76 (s, 3H), 1,45 - 1,30 (m, 2H), 1,10 - 1,00 (m, 2H).

### 5-chloro-3-(1-fluorocyclopropyl)-4-iodo-1-methyl-1H-pyrazole (12)

The above mixture (535 mg) was dissolved in 5 mL MeCN. 266 mg I₂ (1,05 mmol) and 479 mg (NH₄)Ce(NO₂)₆ (0,87 mmol) were added and the mixture was stirred at reflux for 3 h and stirring was continued at room temperature overnight. The mixture was partitioned between H₂O and EtOAc. The organic layer was washed with Na₂S₂O₃ aq. and dried over MgSO₄. The solvent was evaporated.

863 mg 5-chloro-3-(1-fluorocyclopropyl)-4-iodo-1-methyl-1*H*-pyrazole (**12**) were isolated as a mixture with 3-(1-fluorocyclopropyl)-4-iodo-1-methyl-1*H*-pyrazole and used in the next step without purification.
GC-MS**⁾: Index = 1490, Masse (m/z) = 300 [M]⁺.
¹H-NMR (400 MHz, DMSO-d6): 3,86 (s, 3H), 1,41 - 1,31 (m, 2H), 1,06 - 0,99.

### 5-chloro-3-(1-fluorocyclopropyl)-1-methyl-4-(trifluoromethyl)-1H-pyrazole (13)

665 mg CuI (3,49 mmol) and 162 mg KF (2,80 mmol) were dissolved in 6 mL DMF abs. and the solution was saturated with argon. 700 mg of the above isolated mixture of (**12**) and 994 mg Trimethyl(trifluoromethyl)silan were added and the mixture was stirred at 80 °C for 6 h. The mixture was diluted with EtOAc and filtered over a plug of SiO₂. The filtrate was washed with H₂O and the organic layer was dried over MgSO₄. The solvent was evaporated.
776 mg crude 5-chloro-3-(1-fluorocyclopropyl)-1-methyl-4-(trifluoromethyl)-1*H*-pyrazole (**13**) were isolated as brown liquid.
GC-MS**⁾: Index = 1195, Masse (m/z) = 242 [M]⁺.

### 2-chloro-N-(1-cyanocyclopropyl)-5-[5'-(1-fluorocyclopropyl)-2'-methyl-4'-(trifluoromethyl)-2'H-1,3'-bipyrazol-4-yl]benzamide (I-1)

300 mg 5-chloro-3-(1-fluorocyclopropyl)-1-methyl-4-(trifluoromethyl)-1*H*-pyrazole (**13**), 121 mg 2-chloro-*N*-(1-cyanocyclopropyl)-5-(1*H*-pyrazol-4-yl)benzamide (**14**) (0,42 mmol) and 342 mg Cs₂CO₃ (1,05 mmol) were dissolved in 10 mL DMF abs. under argon atmosphere. The mixture was stirred at 80 °C overnight. The mixture was diluted with EtOAc and NH₄Cl aq. was added. The mixture was extracted with EtOAc. The combined organic layer was washed with H₂O and brine and dried over MgSO₄. The solvent was evaporated and the residue was purified by flash chromatography and HPLC.

3 mg 2-chloro-*N*-(1-cyanocyclopropyl)-5-[5'-(1-fluorocyclopropyl)-2'-methyl-4'-(trifluoromethyl)-2'*H*-1,3'-bipyrazol-4-yl]benzamide (**I-1**) were isolated as colorless resin.
HPLC-MS*⁾: please refer to Table 1
¹H-NMR data: please refer to peak list.

**Table 1**

| | | | | | | | | | |
|---|---|---|---|---|---|---|---|---|---|
| | | 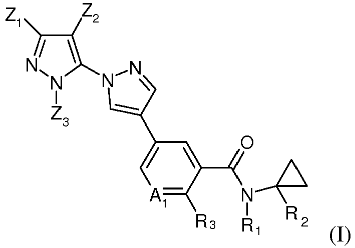 | | | | | | | |

| **Ex. No** | **Z¹** | **Z**² | **Z³** | **A₁** | **R¹** | **R₂** | **R₃** | **logP*⁾** | **Mass [m/z] *⁾** |
|---|---|---|---|---|---|---|---|---|---|
| I-1 | 1-fluorocyclopropyl | CF₃ | Me | CH | H | CN | Cl | 3,09 | 493 |
| I-2 | 1-fluorocyclopropyl | CF₂H | Me | CH | H | CN | Cl | 2,78 | 475 |
| I-3 | 1-fluorocyclopropyl | CF₂H | Me | CH | H | H | Cl | 2,81 | 450 |
| I-4 | 1-fluorocyclopropyl | NO₂ | Me | CH | H | CN | Cl | 2,69 | 470 |
| I-5 | 1-fluorocyclopropyl | NO₂ | Me | CH | H | H | Cl | 2,70 | 445 |
| I-6 | 1-bromocyclopropyl | NO₂ | Me | CH | H | H | Cl | 2,98 | 505 |
| I-7 | 1-chlorocyclopropyl | CF₂H | Me | CH | H | CN | Cl | 2,96 | 491 |
| I-8 | 1-chlorocyclopropyl | CF₂H | Me | CH | H | H | Cl | 3,05 | 466 |
| I-9 | 1-chlorocyclopropyl | CF₂H | Me | N | H | CN | Cl | 2,63 | 492 |
| I-10 | 1-chlorocyclopropyl | CF₂H | Me | N | H | H | Cl | 2,60 | 467 |
| I-11 | 1-fluorocyclopropyl | CF₂H | Me | N | H | CN | Cl | 2,44 | 476 |
| I-12 | 1-fluorocyclopropyl | CF₂H | Me | N | H | H | Cl | 2,42 | 451 |

The stated mass is the peak of the isotope pattern of the [M+H]⁺ ion of the highest intensity; if the [M-H]⁻ ion was detected, the stated mass is marked with ².
² The stated mass is the peak of the isotope pattern of the [M-H]⁻ ion of the highest intensity. If the mass was determined by a GCMS (see below for methods) measurement, the stated mass is marked with ³.
*⁾ Note regarding the determination of the logP values and mass detection: The determination of the given logP values was carried out in accordance with EEC Directive 79/831 Annex V.A8 by HPLC (High Performance Liquid Chromatography) on a reversed-phase column (C18). Agilent 1100 LC system; 50*4.6 Zorbax Eclipse Plus C18 1.8 micron; mobile phase A: acetonitrile (0.1% formic acid); mobile phase B: water (0.09% formic acid); linear gradient from 10% acetonitrile to 95% acetonitrile in 4.25 min, then 95% acetonitrile for a further 1.25 min; oven temperature 55°C; flow rate: 2.0 ml/min. Mass detection is carried out via an Agilend MSD system.
**⁾ Agilent 6890 GC, HP5979 MSD, 10m DB-1, iD=0.18mm, FILM=0.4µm, Inj.:250°C, const. flow: 1.6mm/min He, Det.:MSD:280°C, FID: 320°C, Oven:50°C(1 min) - 40°C/min - 320°C (3.25 min).

### NMR data of selected examples

### NMR peak list method

1H-NMR data of selected examples are written in form of 1H-NMR-peak lists. To each signal peak are listed the δ-value in ppm and the signal intensity in round brackets. Between the δ-value - signal intensity pairs are semicolons as delimiters.

The peak list of an example has therefore the form:
δ₁ (intensity₁); δ₂ (intensity₂);........; δᵢ (intensityᵢ); ...... δₙ (intensityₙ)

Intensity of sharp signals correlates with the height of the signals in a printed example of a NMR spectrum in cm and shows the real relations of signal intensities. From broad signals several peaks or the middle of the signal and their relative intensity in comparison to the most intensive signal in the spectrum can be shown.

For calibrating chemical shift for 1H spectra, we use tetramethylsilane and/or the chemical shift of the solvent used, especially in the case of spectra measured in DMSO. Therefore in NMR peak lists, tetramethylsilane peak can occur but not necessarily.

The 1H-NMR peak lists are similar to classical 1H-NMR prints and contains therefore usually all peaks, which are listed at classical NMR-interpretation.

Additionally they can show like classical 1H-NMR prints signals of solvents, stereoisomers of the target compounds, which are also object of the invention, and/or peaks of impurities.

To show compound signals in the delta-range of solvents and/or water the usual peaks of solvents, for example peaks of DMSO in DMSO-D₆ and the peak of water are shown in our 1H-NMR peak lists and have usually on average a high intensity .

The peaks of stereoisomers of the target compounds and/or peaks of impurities have usually on average a lower intensity than the peaks of target compounds (for example with a purity >90%).

Such stereoisomers and/or impurities can be typical for the specific preparation process. Therefore their peaks can help to recognize the reproduction of our preparation process via "side-products-fingerprints".

An expert, who calculates the peaks of the target compounds with known methods (MestreC, ACD-simulation, but also with empirically evaluated expectation values) can isolate the peaks of the target compounds as needed optionally using additional intensity filters. This isolation would be similar to relevant peak picking at classical 1H-NMR interpretation.

Further details of NMR-data description with peak lists you find in the publication "Citation of NMR Peaklist Data within Patent Applications" of the Research Disclosure Database Number 564025.

| |
|---|
| Example I-1: ¹H-NMR(400.0 MHz, d₆-DMSO): δ= 9.447(2.3);8.806(3.1);8.535(3.4);8.337(0.4);8.317(1.1);7.832(0.6);7.827(1.7);7.823(2.4);7.817(2.2);7.810(1.6);7.804(0.8);7.589(2.0);7.579(0.4);7.567(1.7);6.578(2.2);5.396(1.0);3.687(7.7);3.328(330.5);2.718(0.8);2.675(2.3);2.671(3.2);2.666(2.4);2.541(2.0);2.524(8.0);2.511(180.1);2.506(368.9);2.502(488.4);2.497(360.3);2.493(179.7);2.333(2.3);2.329(3.2);2.324(2.4);2.075(2.0);1.621(0.9);1.607(2.0);1.599(2.2);1.586(1.0);1.514(0.4);1.497(1.1);1.493(1.1);1.478(0.5);1.468(0.5);1.452(1.1);1.448(1.2);1.432(0.6);1.411(1.0);1.285(1.1);1.270(4.2);1.251(1.2);1.233(1.8);1.217(1.6);1.196(0.4);1.169(16.0);0.008(1.4);0.000(47.9);-0.009(1.9) |
| Example I-2: ¹H-NMR(400.0 MHz, d₆-DMSO): δ= 9.437(4.5);8.756(6.5);8.503(7.0);8.313(0.4);7.831(3.8);7.827(5.0);7.822(4.2);7.814(3.2);7.809(1.5);7.585(3.9);7.575(0.8);7.562(3.3);7.128(1.4);6.994(3.2);6.859(1.6);5.754(3.3);3.702(16.0);3.315(140.8);2.675(1.2);2.670(1.7);2.666(1.2);2.505(204.4);2.501(266.9);2.497(198.3);2.332(1.2);2.328(1.6);2.323(1.2);1.620(1.6);1.605(4.2);1.599(4.4);1.586(1.8);1.494(0.7);1.478(2.3);1.474(2.2);1.459(1.0);1.448(0.8);1.432(2.2);1.427(2.3);1.413(1.0);1.288(2.0);1.274(4.2);1.267(4.6);1.253(1.7);1.236(0.4);1.221(1.0);1.205(2.6);1.200(3.3);1.184(3.0);1.163(0.7);0.008(0.3);0.000(8.5) |
| Example I-3: ¹H-NMR(400.0 MHz, d₆-DMSO): δ= 8.733(6.8);8.528(2.2);8.517(2.4);8.490(7.3);8.312(0.6);7.778(1.3);7.772(3.8);7.768(5.1);7.763(4.7);7.756(3.6);7.750(1.6);7.535(4.3);7.525(0.8);7.512(3.6);7.126(1.5);6.991(3.4);6.856(1.6);5.753(0.8);3.722(0.7);3.706(16.0);3.317(224.2);2.862(0.7);2.853(0.9);2.844(1.5);2.834(1.4);2.825(0.9);2.816(0.7);2.675(1.0);2.670(1.4);2.666(1.0);2.523(4.3);2.510(86.6);2.506(174.2);2.501(231.1);2.497(170.0);2.492(85.2);2.337(0.5);2.332(1.0);2.328(1.4);2.323(1.0);1.492(0.7);1.477(2.3);1.472(2.3);1.457(1.0);1.447(0.8);1.431(2.2);1.426(2.3);1.411(1.0);1.296(0.4);1.221(0.9);1.205(2.5);1.200(3.2);1.184(3.0);1.180(2.5);1.163(0.7);0.732(0.9);0.719(2.6);0.714(3.7);0.702(3.4);0.696(2.9);0.685(1.2);0.564(1.2);0.553(3.6);0.547(3.3);0.543(3.1);0.537(3.0);0.525(1.0);0.000(5.3) |
| Example I-4: ¹H-NMR(400.0 MHz, d₆-DMSO): δ= 9.470(4.3);8.903(6.3);8.591(6.3);8.317(0.6);7.832(1.3);7.826(2.4);7.815(3.3);7.809(7.7);7.607(3.4);7.600(0.9);7.590(0.7);7.584(2.8);3.798(16.0);3.329(231.5);2.675(1.2);2.671(1.6);2.666(1.3);2.524(4.0);2.510(90.3);2.506(185.0);2.502(246.5);2.497(184.6);2.493(93.9);2.333(1.1);2.329(1.6);2.324(1.2);2.075(3.1);1.622(1.4);1.608(3.6);1.601(3.9);1.588(1.7);1.529(0.6);1.508(1.8);1.492(0.8);1.484(0.7);1.463(1.9);1.447(0.8);1.288(2.3);1.273(5.3);1.268(6.3);1.252(3.6);1.230(0.6);0.008(0.5);0.000(15.5);-0.008(0.7) |
| Example I-5: ¹H-NMR(400.0 MHz, d₆-DMSO): δ=8.885(6.1);8.579(6.2);8.563(2.1);8.551(2.1);7.773(1.4);7.767(2.2);7.749(8.7);7.557(2.7);7.535(2.2);3.799(16.0);3.332(77.1);2.862(0.6);2.853(0.9);2.844(1.3);2.834(1.3);2.826(0.9);2.816(0.6);2.675(0.4);2.671(0.6);2.667(0.4);2.506(64.1);2.502(84.3);2.498(65.6);2.333(0.4);2.329(0.5);2.325(0.4);2.075(0.5);1.529(0.6);1.508(2.0);1.492(0.9);1.484(0.8);1.462(2.1);1.447(0.9);1.290(0.8);1.269(2.8);1.252(2.6);1.231(0.6);0.734(0.8);0.721(2.4);0.716(3.3);0.704(3.1);0.698(2.7);0.687(1.1);0.562(1.0);0.551(3.2);0.545(3.2);0.536(2.9);0.524(0.9);0.000(28.3) |
| Example I-6: ¹H-NMR(600.1 MHz, d₆-DMSO): δ= 8.883(4.7);8.565(4.8);8.547(1.6);8.540(1.7);7.766(1.4);7.762(1.8);7.752(0.9);7.748(4.8);7.744(1.8);7.548(2.4);7.5344(2.1);7.5337(2.0);5.755(6.9);4.015(0.7);3.850(1.0);3.806(0.8);3.780(0.6);3.768(16.0);3.744(0.4);3.335(43.7);3.186(1.1);2.853(0.5);2.847(0.7);2.841(1.0);2.834(1.1);2.828(0.7);2.822(0.6);2.523(0.4);2.520(0.5);2.517(0.5);2.508(12.8);2.505(27.0);2.502(37.0);2.499(26.8);2.496(12.5);1.552(14.7);1.512(0.4);0.725(0.7);0.716(1.9);0.713(2.6);0.705(2.6);0.701(2.3);0.693(1.0);0.688(0.4);0.557(0.8);0.550(2.3);0.546(2.3);0.543(2.2);0.539(2.3);0.531(1.0);0.527(0.4);0.000(1.1) |
| Example I-7: ¹H-NMR(400.0 MHz, d₆-DMSO): δ= 9.433(4.4);8.747(5.8);8.493(6.1);8.482(0.6);8.313(0.9);7.820(4.9);7.814(3.9);7.806(3.0);7.801(1.8);7.581(3.2);7.572(0.8);7.559(2.8);7.181(1.3);7.047(3.0);6.912(1.5);4.056(0.9);4.038(2.5);4.020(2.5);4.002(0.8);3.810(0.4);3.750(0.7);3.692(16.0);3.313(209.1);2.670(2.9);2.505(315.8);2.501(413.5);2.497(328.8);2.328(2.7);1.988(10.3);1.618(1.5);1.604(4.0);1.597(4.3);1.584(1.8);1.454(15.5);1.426(0.9);1.398(0.4);1.286(1.8);1.272(4.2);1.266(4.3);1.251(1.6);1.235(0.5);1.193(2.8);1.175(5.5);1.157(2.7);1.069(0.4);0.146(0.5);0.000(106.1);-0.150(0.5) |
| Example I-8: ¹H-NMR(601.6 MHz, d₆-DMSO): δ= 8.724(5.6);8.518(2.0);8.511(2.1);8.481(5.8);7.761(3.5);7.758(4.6);7.755(3.7);7.750(2.8);7.746(1.3);7.529(3.0);7.523(0.7);7.520(0.6);7.514(2.8);7.134(1.2);7.044(2.8);6.955(1.3);5.754(3.4);3.711(0.6);3.694(16.0);3.335(0.4);3.311(142.8);2.853(0.5);2.846(0.8);2.841(1.2);2.834(1.2);2.828(0.8);2.822(0.6);2.615(0.8);2.612(1.1);2.609(0.8);2.521(2.1);2.518(2.6);2.515(2.9);2.503(122.5);2.500(162.2);2.497(124.2);2.384(1.1);2.382(0.8);1.453(11.7);0.722(0.8);0.714(2.4);0.711(3.1);0.702(3.0);0.699(2.5);0.691(0.9);0.556(0.9);0.549(3.0);0.545(3.0);0.542(2.8);0.539(2.8);0.531(0.8);0.000(6.2) |
| Example I-9: ¹H-NMR(400.0 MHz, d₆-DMSO): δ= 9.604(4.1);8.911(3.3);8.905(3.3);8.855(5.5);8.589(5.8);8.327(3.4);8.322(3.3);8.313(0.6);7.195(1.2);7.060(2.7);6.926(1.3);4.038(0.5);4.020(0.5);3.757(0.4);3.700(14.7);3.314(93.0);2.670(1.1);2.504(121.4);2.501(154.2);2.328(1.0);1.988(2.0);1.648(1.4);1.634(3.6);1.627(3.8);1.614(1.5);1.458(16.0);1.436(0.7);1.296(1.7);1.282(3.6);1.276(3.8);1.261(1.4);1.193(0.5);1.175(1.0);1.157(0.5);0.000(36.8) |
| Example I-10: ¹H-NMR(400.0 MHz, d₆-DMSO): δ= 8.855(3.3);8.849(3.4);8.839(5.2);8.690(1.9);8.680(1.9);8.582(5.3);8.252(3.2);8.246(3.1);7.191(1.2);7.056(2.6);6.922(1.3);5.753(2.2);3.717(0.7);3.702(13.6);3.685(0.4);3.314(38.5);2.873(0.5);2.864(0.7);2.855(1.1);2.845(1.1);2.837(0.8);2.827(0.5);2.671(0.5);2.505(54.4);2.501(70.5);2.497(55.5);2.328(0.4);1.988(1.3);1.457(16.0);1.413(0.4);1.356(0.6);1.193(0.4);1.175(0.7);1.157(0.4);0.759(0.7);0.746(2.2);0.741(2.8);0.729(2.7);0.724(2.3);0.712(0.9);0.568(0.9);0.558(2.8);0.551(2.8);0.542(2.4);0.530(0.7);0.000(16.9) |
| Example I-11: ¹H-NMR(400.0 MHz, d₆-DMSO): δ= 9.607(4.8);8.919(4.7);8.913(4.7);8.863(7.0);8.598(7.5);8.336(4.8);8.330(4.7);8.313(0.4);7.141(1.4);7.007(3.3);6.873(1.7);3.710(16.0);3.317(82.7);2.675(0.6);2.670(0.8);2.666(0.6);2.510(53.4);2.506(105.7);2.501(139.2);2.497(102.4);2.492(51.2);2.332(0.6);2.328(0.9);2.323(0.6);1.649(1.7);1.635(4.2);1.628(4.4);1.615(1.9);1.498(0.7);1.482(2.2);1.478(2.2);1.463(0.9);1.452(0.8);1.436(2.2);1.431(2.3);1.416(1.0);1.298(1.9);1.284(4.1);1.277(4.4);1.263(1.6);1.225(0.9);1.210(2.4);1.205(3.1);1.189(2.9);1.184(2.4);1.167(0.7);0.000(4.3) |
| Example I-12: ¹H-NMR(400.0 MHz, d₆-DMSO): δ= 8.863(4.6);8.857(4.8);8.847(6.9);8.695(2.1);8.684(2.1);8.606(0.3);8.591(7.3);8.313(0.5);8.260(4.5);8.254(4.4);7.138(1.5);7.004(3.3);6.869(1.6);5.753(5.1);3.727(0.7);3.712(16.0);3.325(127.6);3.320(106.7);2.874(0.7);2.865(0.9);2.856(1.4);2.846(1.4);2.837(0.9);2.828(0.7);2.675(0.7);2.670(1.0);2.666(0.7);2.506(122.3);2.501(160.1);2.497(117.6);2.332(0.7);2.328(1.0);2.324(0.7);1.497(0.7);1.481(2.3);1.476(2.2);1.461(0.9);1.451(0.8);1.434(2.2);1.430(2.3);1.415(1.0);1.225(1.0);1.209(2.5);1.205(3.2);1.189(3.0);1.167(0.7);0.760(0.9);0.747(2.7);0.742(3.6);0.730(3.5);0.724(2.8);0.713(1.1);0.569(1.2);0.558(3.5);0.552(3.3);0.543(3.0);0.531(0.9);0.000(4.4) |

### Preparation of the starting materials

### Synthesis of 3-(1-fluorocyclopropyl)-1-methyl-1H-pyrazole (10)

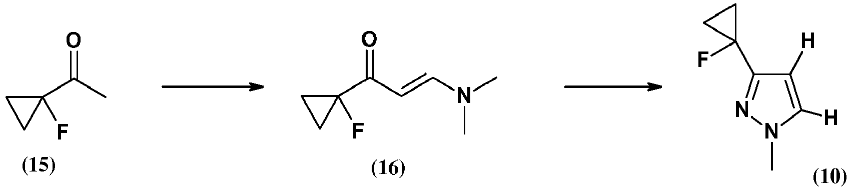

### (2E)-3-(dimethylamino)-1-(1-fluorocyclopropyl)prop-2-en-1-one (16)

(2*E*)-3-(dimethylamino)-1-(1-fluorocyclopropyl)prop-2-en-1-one (**16**) was synthesized as described for (2*E*)-3-(dimethylamino)-1-(cyclopropyl)prop-2-en-1-one in J. Med. Chem. 2011, 54, 7974 starting from (**15**).
HPLC-MS*⁾: logP = 1,16, Masse (m/z) = 158 [M+H]⁺.
¹H-NMR (400 MHz, DMSO-d6): 7,63 (d,1H), 5,47-5,43 (m,1H), 3,12 (s,3H), 2,84 (s,3H), 1,28-1,10 (m,4H)

### 3-(1-fluorocyclopropyl)-1-methyl-1H-pyrazole (10)

685 mg (4,35 mmol) (2*E*)-3-(dimethylamino)-1-(1-fluorocyclopropyl)prop-2-en-1-one (**16**) was dissolved in 10 mL EtOH and 0,35 mL methylhydrazin (6.53 mmol) were added. The mixture was stirred at 60 °C overnight. 1,16 mL methylhydrazin (2,18 mmol) were added and stirring at 60 °C was continued for another 5 h. The mixture was cooled to ambient temperature and the solvent was evaporated. The residue was dissolved in EtOAc and the solution was washed with saturated aqueous Na₂CO₃ solution and brine. The organic layer was dried over MgSO4 and the solvent was evaporated. The residue was purified by flash chromatography on SiO2.

156 mg 3-(1-fluorocyclopropyl)-1-methyl-1*H*-pyrazole (**10**) as a 6:4 mixture with 5-(1-fluorocyclopropyl)-1-methyl-1H-pyrazole were isolated as yellow liquid.
GC-MS**⁾: Index = 1086, Masse (m/z) = 140 [M]⁺.
¹H-NMR (400 MHz, DMSO-d6): 7,67 (d,1H), 6,29 (d,1H), 3,79 (s,3H), 1,38 - 1,30 (m,2H), 1,07-1,00 (m,2H).

### Synthesis of 2-chloro-N-(1-cyanocyclopropyl)-5-(1H-pyrazol-4-yl)benzamide (14)

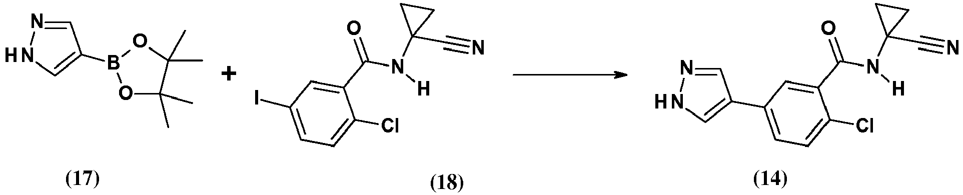

2,25 g 4-(4,4,5,5-tetramethyl-1,3,2-dioxaborolan-2-yl)-1*H*-pyrazole (**17**) (11,5 mmol), 2,00 g 2-chloro-*N*-(1-cyanocyclopropyl)-5-iodobenzamide (**18**) (5,77 mmol) and 667 mg Pd(PPh₃)₄ (1,16 mmol) were dissolved in 186 mL 2-propanol and 18 mL NaHCO₃ aq. 1M were added. The mixture was degassed and stirred under reflux overnight. The solvent was evaporated and the residue was dissolved in EtOAc. The organic layer was washed with H₂O and brine, dried over MgSO₄ and the solvent was evaporated. The residue was purified by flash chromatography on SiO₂.

995 mg 2-chloro-*N*-(1-cyanocyclopropyl)-5-(1*H*-pyrazol-4-yl)benzamide (**14**) were isolated as colorless solid.
HPLC-MS*⁾: logP = 1,31, Masse (m/z) = 287 [M+H]⁺.
¹H-NMR (400 MHz, acetonitrile-d3): 7,93 (s,2H), 7,66 - 7,59 (m,3H), 7,43 (d,1H), 1,59 - 1,56 (m,2H), 1,36 - 1,32 (m,2H).

An example for the synthesis for compounds of the invention (I) where Z₂ represents -CHF₂ is shown below. 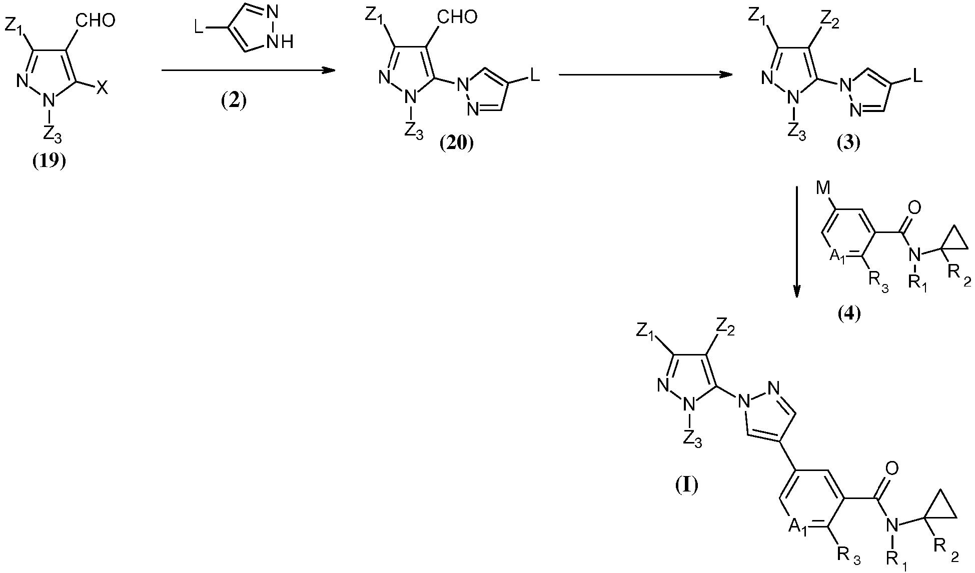 Z₁, Z₃, R₁, R₂, R₃ and A₁ represent the above described residues. Z₂ represents -CHF₂, L represents bromine or iodine, whereas M represents boronic acid, boronic acid esters or trifluoroboronate. X represents a suitable leaving group like chloride or fluoride

Compounds of formula (**I**) where Z₂ represents -CHF₂ can be synthesized by a transition metal (e.g. Palladium) catalysed reactions of a compound formula (**3**) with a compound of the formula (**4**). Those reactions may be performed in analogy to procedures described in literature [see, for example: WO2005-040110; WO2009-089508]. Compounds of the formula (**4**) are either commercially available or can be synthesized in analogy to procedures known from literature. Compounds of formula (**3**) may be synthesized in analogy to literature-known procedures from compounds of formula (**20**) [see, for example: US2008-0033013]. Compounds of formula (**20**) may be prepared in a nucleophilic substitution from compounds of formula (**19**) and a corresponding pyrazole derivative (**2**) as described above in the synthesis of (**3**). The general synthesis route for compounds of formula (**19**) is described below.

The general synthesis of intermediats (**19**) is shown below.

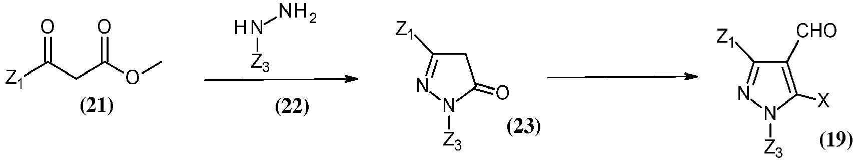

Compounds of formula (**19**) may be synthesized from compounds of formula (**23**). Similar reactions are described in literature [see, for example: Org. Lett. 2015, 17, 932-935.].Compounds of formula (**23**) can be synthesized from compounds of formula (**21**) by condensation with hydrazine derivatives (**22**). Similar reactions are described in literature [see, for example: US2008-0194617]

### Synthesis of 2-chloro-N-(1-cyanocyclopropyl)-5-[4'-(difluoromethyl)-5'-(1-fluorocyclopropyl)-2'-methyl-2'H-1,3'-bipyrazol-4-yl]benzamide (I-2)

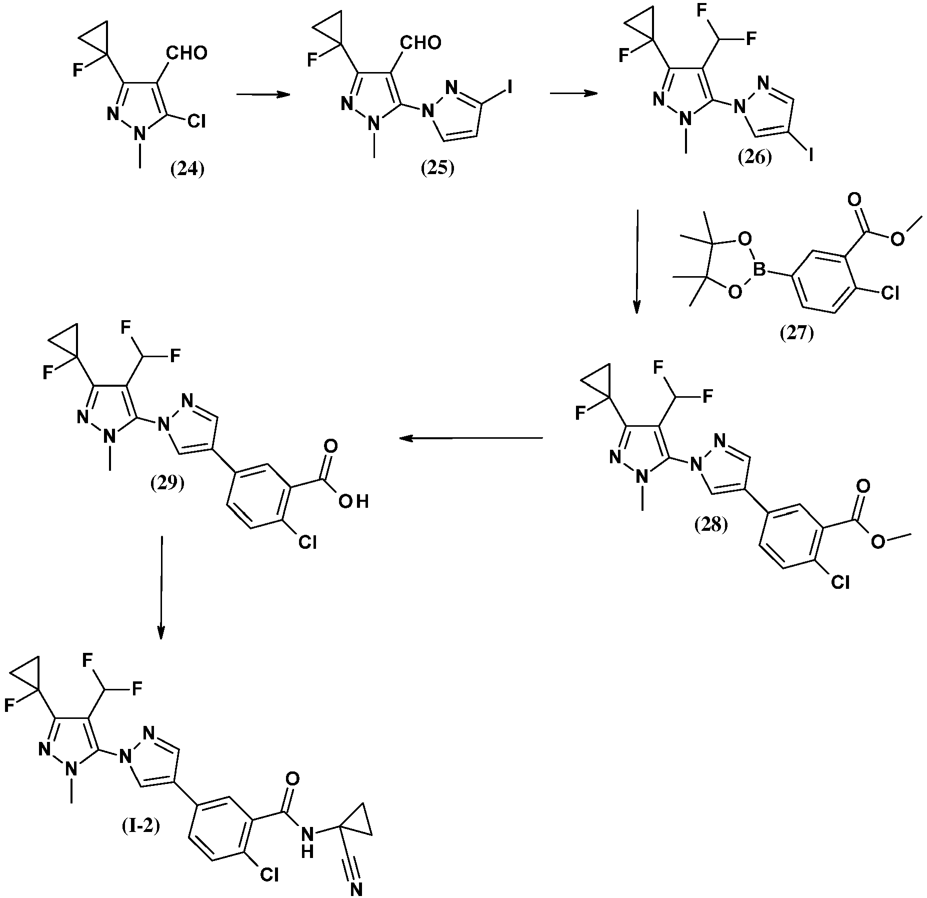

### 5'-(1-fluorocyclopropyl)-4-iodo-2'-methyl-2'H-1,3'-bipyrazole-4'-carbaldehyde (25)

593 mg 5-chloro-3-(1-fluorocyclopropyl)-1-methyl-1H-pyrazole-4-carbaldehyde (**24**) (2,70 mmol) were added to a solution of 840 mg 4-iodo-1H-pyrazole (4,33 mmol) and 2,20 g Cs₂CO₃ (6,76 mmo) in anhydrous DMF. The solution was stirred for 16 h at room temperature. The solution was diluted with EtOAc, washed with NH₄Cl solution and the aqueous layer was extracted with EtOAc. The combined organic layers were whashed with brine, dried over MgSO₄ and the solvent was evaporated. The residue was purified by flash chromatography.

200 mg 5'-(1-fluorocyclopropyl)-4-iodo-2'-methyl-2'H-1,3'-bipyrazole-4'-carbaldehyde (**25**) were isolated as a colorless solid.
HPLC-MS*⁾: logP = 2,86, Masse (m/z) = 376 [M⁺].
¹H-NMR (400 MHz, DMSO-d6): 9,86(s,1H), 8,54(s,1H), 8,06(s,1H), 3,73(s,3H), 1,47-1,46(m,4H)

### 4'-(difluoromethyl)-5'-(1-fluorocyclopropyl)-4-iodo-2'-methyl-2'H-1,3'-bipyrazole (26)

200 mg 5'-(1-fluorocyclopropyl)-4-iodo-2'-methyl-2'H-1,3'-bipyrazole-4'-carbaldehyde (**25**) (0,5 mmol) were dissolved in 10 mL CH₂Cl₂ and 542 mg N,N-diethylaminosulfurtrifluoride (3,0 mmol) dissolved in 20 mL CH₂Cl₂ were added. The solution was stirred at r.t. for 48 h. The reaction was quenched by the addition of aqueous NaHCO₃ solution. The mixture was extracted with EtOAc. The combined organic layers dried over Na₂SO₄ and the solvent was evaporated.

214 mg 4'-(difluoromethyl)-5'-(1-fluorocyclopropyl)-4-iodo-2'-methyl-2'H-1,3'-bipyrazole (**26**) were isolated as yellow oil and used in the next step without further purification.
HPLC-MS*⁾: logP = 3,37, Masse (m/z) = 398 [M⁺].
¹H-NMR (400 MHz, DMSO-d6): 8,38(s,1H), 8,01(s,1H), 6,99(t,1H), 3,62(s,3H), 1,44-1,43(m,4H)

### Methyl 2-chloro-5-[4'-(difluoromethyl)-5'-(1-fluorocyclopropyl)-2'-methyl-2'H-1,3'-bipyrazol-4-yl]benzoate (28)

189 mg 4'-(difluoromethyl)-5'-(1-fluorocyclopropyl)-4-iodo-2'-methyl-2'H-1,3'-bipyrazole (**26**), 170 mg methyl 2-chloro-5-(4,4,5,5-tetramethyl-1,3,2-dioxaborolan-2-yl)benzoate (**27**) and 22,8 mg Pd(PPh₃)₄ were dissolved in 5 mL 1,4-dioxane and 2 mL NaHCO₃ solution was added. The mixture was stirred at 80 °C for 16 h. The solution was cooled down to r.t. and filtered over silica. The filtrate was concentrated and the residue was purified by flash chromatography.

87 mg methyl 2-chloro-5-[4'-(difluoromethyl)-5'-(1-fluorocyclopropyl)-2'-methyl-2'H-1,3'-bipyrazol-4-yl]benzoate (**28**) were isolated as yellow oil.
HPLC-MS*⁾: logP = 3,63, Masse (m/z) = 424 [M+].
1H-NMR (400 MHz, DMSO-d6): 8,77(s,1H), 8,50(s,1H), 8,12(d,1H), 7,93-7,91(m,1H), 7,65(d,1H), 7,00(t,1H), 3,90(s,3H), 3,71(s,3H), 1,49-1,41(m,2H), 1,23-1,16(m,2H)

### 2-chloro-5-[4'-(difluoromethyl)-5'-(1-fluorocyclopropyl)-2'-methyl-2'H-1,3'-bipyrazol-4-yl]benzoic acid (29)

87 mg methyl 2-chloro-5-[4'-(difluoromethyl)-5'-(1-fluorocyclopropyl)-2'-methyl-2'H-1,3'-bipyrazol-4-yl]benzoate (**28**) were dissolved in 2 mL THF and 14.7 mg LiOH dissolved in 2 mL H₂O were added. The solution was stirred at r.t. for 3 h. The mixture was diluted with EtOAc and washed with HCl 1M and brine. The organic layer was dried over MgSO₄ and the solvent was evaporated. The residue was used in the next step without further purification.

82 mg 2-chloro-5-[4'-(difluoromethyl)-5'-(1-fluorocyclopropyl)-2'-methyl-2'H-1,3'-bipyrazol-4-yl]benzoic acid (**29**) were isolated as yellow oil.
HPLC-MS*): logP = 2,71, Masse (m/z) = 410 [M+].

### 2-chloro-N-(1-cyanocyclopropyl)-5-[4'-(difluoromethyl)-5'-(1-fluorocyclopropyl)-2'-methyl-2'H-1,3'-bipyrazol-4-yl]benzamide (I-2)

41 mg 2-chloro-5-[4'-(difluoromethyl)-5'-(1-fluorocyclopropyl)-2'-methyl-2'H-1,3'-bipyrazol-4-yl]benzoic acid (**29**) were 36,5 mg 4-*N,N*-dimethylaminopyridine and 57,4 mg 1-Ethyl-3-(3-dimethylaminopropyl)carbodiimide were dissolved in 9 mL CH₂Cl₂ and 1 mL DMF. The mixture was stirred at r.t. for 12 h. The solution was diluted with CH₂Cl₂ and washed with HCL 1M and brine. The organic layer was dried over MgSO₄ and the solvent was evaporated. The residue was purified by preparative HPLC.

25 mg 2-chloro-*N*-(1-cyanocyclopropyl)-5-[4'-(difluoromethyl)-5'-(1-fluorocyclopropyl)-2'-methyl-2'H-1,3'-bipyrazol-4-yl]benzamide (**I-2**) were isolated as colorless solid.
HPLC-MS*⁾: please refer to Table 1
¹H-NMR data: please refer to peak list.

### Preparation of the starting materials

### Synthesis of 5-chloro-3-(1-fluorocyclopropyl)-1-methyl-1H-pyrazole-4-carbaldehyde (24)

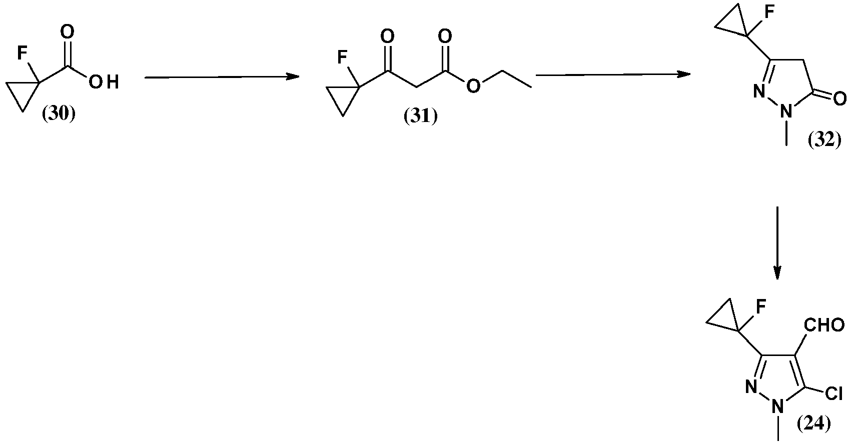

### methyl 3-(1-fluorocyclopropyl)-3-oxopropanoate (31)

10 g 1-fluorocyclopropanecarboxylic acid (**30**) were dissolved in 80 mL CH₂Cl₂ and 10,9 g TMS-CH₂N₂ were added dropwise. The mixture was stirred at r.t. for 1 h. The solvent was evaporated and product was used in the next step without further purification.

11,3 g ethyl acetate were dissolved in 150 mL THF. 122 mL LiHMDS 1M in hexanes was added dropwise at -78 °C. The mixture was stirred at -78 °C for 1 h. 8,0 g of the above methyl ester dissolved in 150 mL THF was added dropwise at -78 °C and the mixture was stirred for another 3 h. The reaction was quenched by the addition of saturated aqueous NH₄Cl solution. The mixture was allowed to warm to r.t. and was extracted with EtOAc. The combined organic layers were washed with brine and dried over MgSO₄. The solvent was evaporated and the product was used in the next without further purification.

10,9 g methyl 3-(1-fluorocyclopropyl)-3-oxopropanoate (**31**) were isolated as yellow oil.
GC-MS**): Index = 1502, Masse (m/z) = 175 [M+H]⁺

### 5-(1-fluorocyclopropyl)-2-methyl-2,4-dihydro-3H-pyrazol-3-one (32)

10,9 g methyl 3-(1-fluorocyclopropyl)-3-oxopropanoate (**31**) were dissolved in 150 mL toluene. 2,31 g methylhydrazine dissolved in 100 mL toluene were added dropwise at 0 °C. The mixture was heated to 100 °C and stirred for 2 h. The solvent was evaporated and the residue was purified by flash chromatography.

1,5 g 5-(1-fluorocyclopropyl)-2-methyl-2,4-dihydro-3H-pyrazol-3-one (**32**) were isolated as yellow solid.
HPLC-MS*): logP = 0,38, Masse (m/z) = 157 [M+H]⁺.
1H-NMR (400 MHz, DMSO-d6): 11,00(s,1H), 5,41(s,1H), 3,46(s,3H), 1,30-1,18(m,2H), 0,99-0,93(m,2H)

### 5-chloro-3-(1-fluorocyclopropyl)-1-methyl-1H-pyrazole-4-carbaldehyde (24)

298 mg 5-(1-fluorocyclopropyl)-2-methyl-2,4-dihydro-3H-pyrazol-3-one (**32**) were dissolved in 5 mL DMF and 732 mg POCl₃ were added. The reaction was stirred at 80 °C for 4 h. The solution was cooled to r.t. and poured on ice. The mixture was extracted with CH₂Cl₂. The combined organic layers were dried over MgSO4 and the solvent was evaporated. The residue was used in the next step without further purification.

795 mg 5-(1-fluorocyclopropyl)-2-methyl-2,4-dihydro-3H-pyrazol-3-one (**32**) were isolated as yellow oil in mixture with DMF.
HPLC-MS*): logP = 1,71, Masse (m/z) = 203 [M+H]⁺.
1H-NMR (400 MHz, DMSO-d6): 3,83 (s,3H), 1,47 - 1,43 (m, 2H) 1,19 - 1,17 (m, 2H)

### Biological Efficacy

### Boophilus microplus - Iniectiontest (BOOPMI Ini)

| | |
|---|---|
| Solvent: | dimethyl sulfoxide |

To produce a suitable preparation of active compound, 10 mg of active compound are dissolved in 0.5 ml solvent, and the concentrate is diluted with solvent to the desired concentration.

Five adult engorged female ticks (*Boophilus microplus*) are injected with 1 µl compound solution into the abdomen. The ticks are transferred into replica plates and incubated in a climate chamber.

After 7 days egg deposition of fertile eggs is monitored. Eggs where fertility is not visible are stored in a climate chamber till hatching after about 42 days. An efficacy of 100 % means all eggs are infertile; 0 % means all eggs are fertile.

In this test, for example, the following compounds from the preparation examples showed good activity of 100 % at an application rate of 20 µg /animal: I-4

### Ctenocephalides felis - oral test (CTECFE)

| | |
|---|---|
| Solvent: | dimethyl sulfoxide |

To produce a suitable preparation of active compound, 10 mg of active compound are dissolved in 0.5 ml solvent, and the concentrate is diluted with cattle blood to the desired concentration.

Approximately 20 adult unfed cat fleas (*Ctenocephalides felis*) are placed in flea chambers. The blood chamber, sealed with parafilm on the bottom, are filled with cattle blood supplied with compound solution and placed on the gauze covered top of the flea chamber, so that the fleas are able to suck the blood. The blood chamber is heated to 37 °C whereas the flea chamber is kept at room temperature.

After 2 days mortality in % is determined. 100 % means all the fleas have been killed; 0 % means none of the fleas have been killed.

In this test, for example, the following compounds from the preparation examples showed good activity of 100 % at an application rate of 100 ppm: 1-4

### Lucilia cuprina - test (LUCICU)

| | |
|---|---|
| Solvent: | dimethyl sulfoxide |

10 mg active compound are dissolved in 0.5 ml Dimethylsulfoxid. Serial dilutions are made to obtain the desired rates.

Approximately 20 1^{st} instar larvae of the Australian sheep blowfly (*Lucilia cuprina*) are transferred into a test tube containing minced horse meat and compound solution of the desired concentration.

After 2 days mortality in % is determined. 100 % means all the larvae have been killed; 0 % means none of the larvae have been killed.

In this test, for example, the following compounds from the preparation examples showed good activity of 100 % at an application rate of 100 ppm: I-4

### Myzus persicae - spray test

| | | |
|---|---|---|
| Solvent: | 78.0 | parts by weight acetone |
| | 1.5 | parts by weight dimethylformamide |
| Emulsifier: | alkylarylpolyglycol ether | |

To produce a suitable preparation of active compound, 1 part by weight of active compound is mixed with the stated amount of solvents and is diluted with water, containing an emulsifier concentration of 1000 ppm, to the desired concentration. Further test concentrations are prepared by dilution with emulsifier containing water.

Chinese cabbage (*Brassica pekinensis*) leaf disks infected with all instars of the green peach aphid (*Myzus persicae*), are sprayed with a preparation of the active ingredient of the desired concentration.

After 6 days mortality in % is determined. 100 % means all aphids have been killed and 0 % means none of the aphids have been killed.

In this test, for example, the following compounds from the preparation examples showed good activity of 100 % at an application rate of 100 g/ha: 1-9

In this test, for example, the following compounds from the preparation examples showed good activity of 90 % at an application rate of 100 g/ha: I-3, I-7, I-10, I-11, I-12

### Phaedon cochleariae - spray test

| | | |
|---|---|---|
| Solvent: | 78.0 | parts by weight of acetone |
| | 1.5parts by weight of dimethylformamide | |
| Emulsifier: | | alkylarylpolyglycol ether |

To produce a suitable preparation of active compound, 1 part by weight of active compound is mixed with the stated amount of solvents and is diluted with water, containing an emulsifier concentration of 1000 ppm, to the desired concentration. Further test concentrations are prepared by dilution with emulsifier containing water.

Chinese cabbage (*Brassica pekinensis*) leaf disks are sprayed with a preparation of the active ingredient of the desired concentration. Once dry, the leaf disks are infested with mustard beetle larvae (*Phaedon cochleariae*).

After 7 days mortality in % is determined. 100 % means all beetle larvae have been killed and 0 % means none of the beetle larvae have been killed.

In this test, for example, the following compounds from the preparation examples showed good activity of 100 % at an application rate of 100 g/ha: 1-2, 1-3, 1-4, 1-6, 1-7, 1-8, 1-9, 1-10, 1-11, 1-12

### Spodoptera frugiperda - spray test

| | | |
|---|---|---|
| Solvent: | 78.0 | parts by weight acetone |
| | 1.5 | parts by weight dimethylformamide |
| Emulsifier: | alkylarylpolyglycol ether | |

To produce a suitable preparation of active compound, 1 part by weight of active compound is mixed with the stated amount of solvents and is diluted with water, containing an emulsifier concentration of 1000 ppm, to the desired concentration. Further test concentrations are prepared by dilution with emulsifier containing water.

Maize (*Zea mays*) leaf sections are sprayed with a preparation of the active ingredient of the desired concentration. Once dry, the leaf sections are infested with fall armyworm larvae (*Spodoptera frugiperda*).

After 7 days mortality in % is determined. 100% means all caterpillars have been killed and 0% means none of the caterpillars have been killed.

In this test, for example, the following compounds from the preparation examples showed good activity of 100 % at an application rate of 100 g/ha: 1-2, 1-7, 1-8

In this test, for example, the following compounds from the preparation examples showed good activity of 83 % at an application rate of 100 g/ha: 1-3

### Tetranvchus urticae - spray test OP-resistant

| | |
|---|---|
| Solvent: | 78.0 parts by weight acetone |
| | 1.5parts by weight dimethylformamide |
| Emulsifier: | alkylarylpolyglycol ether |

To produce a suitable preparation of active compound, 1 part by weight of active compound is mixed with the stated amount of solvents and is diluted with water, containing an emulsifier concentration of 1000 ppm, to the desired concentration. Further test concentrations are prepared by dilution with emulsifier containing water.

French bean (*Phaseolus vulgaris*) leaf disks infected with all instars of the two spotted spidermite (*Tetranychus urticae*), are sprayed with a preparation of the active ingredient of the desired concentration.

After 6 days mortality in % is determined. 100% means all spider mites have been killed and 0% means none of the spider mites have been killed.

In this test, for example, the following compounds from the preparation examples showed good activity of 100 % at an application rate of 100 g/ha: 1-2, 1-3, 1-4, 1-6, 1-7, 1-8, 1-9, 1-11, 1-12

In this test, for example, the following compounds from the preparation examples showed good activity of 90 % at an application rate of 100 g/ha: I-10

## Claims

1. Compounds of the general formula (I), 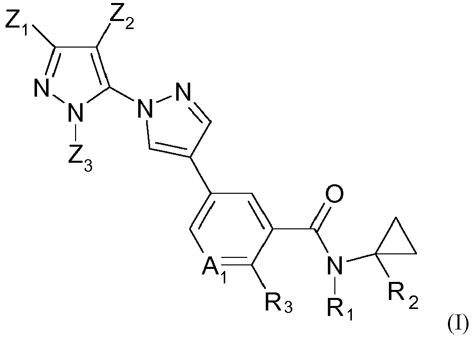 in which
Z₁ represents 1-halogenated cyclopropyl;
Z₂ represents -S(O)₀₋₂-(C₁-C₂)-alkyl, (C₁-C₂)-halogenalkyl, or nitro (NO₂);
Z₃ represents (C₁-C₂)-alkyl;
R₁ represents hydrogen (H) or (C₁-C₂)-alkyl;
R₂ represents H or cyano (CN);
R₃ represents CH₃ or chlorine (CI);
A₁ represents CH or nitrogen (N).

2. Compound according to claim 1, wherein A₁ is CH.

3. Compound according to claim 1 or 2, wherein A₁ is CH and R₃ is Cl.

4. Compound according to any of the preceding claims, wherein
Z₁ is 1-fluoro-cyclopropyl or 1-chloro-cyclopropyl;
Z₂ is -S-CH₃, -SO-CH₃, -SO₂-CH₃, CF₃, CF₂H, Cl, Br, I or NO₂;
Z₃ is CH₃.

5. Compound according to any of the preceding claims, wherein
Z₁ is 1-fluoro-cyclopropyl or 1-chloro-cyclopropyl;
Z₂ is -S-CH₃, Cl, Br, I, CF₃, CF₂H, or NO₂;
Z₃ is CH₃.

6. Compound according to any of the preceding claims, wherein
Z₁ is 1-fluoro-cyclopropyl or 1-chloro-cyclopropyl;
Z₂ is I, CF₃, CF₂H, or NO₂;
Z₃ is CH₃.

7. Compound according to any of the preceding claims, wherein R₁ is H.

8. Compound according to any one of claims 1 or 4 to 7, wherein A₁ is N.

9. Compound according to claim 8, wherein R₃ is Cl.

10. Use of a compound of the general formula (I) according to any of Claims 1 to 8 for controlling insects, arachnids and nematodes, excluded herefrom are methods for the surgical or therapeutic treatment of the human or animal body and diagnostic methods carried out on the human or animal body.

11. Pharmaceutical composition comprising at least one compound according to any of Claims 1 to 9.

12. A Compound according to any of Claims 1 to 8 for use as a medicament.

13. Use of a compound according to any of Claims 1 to 9 for preparing a pharmaceutical composition for controlling parasites on animals.

14. A compound selected from the group consisting of

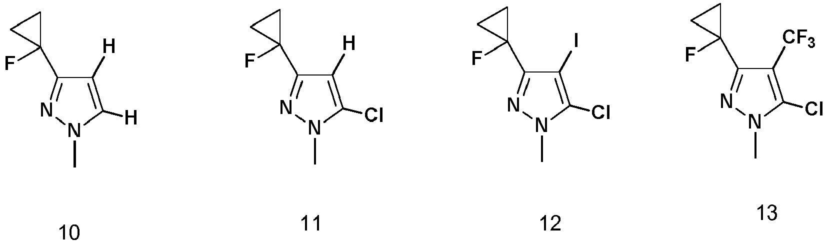

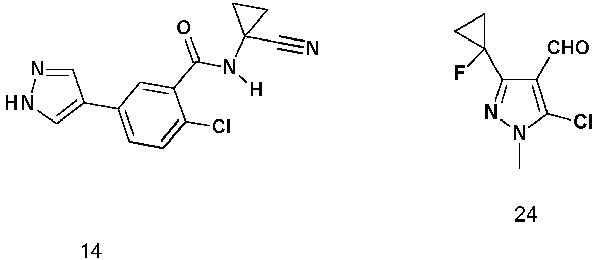

15. Use of a compound according to any of Claims 1 to 9 for protecting the propagation material of plants.

## Patentansprüche

1. Verbindungen der allgemeinen Formel (I), 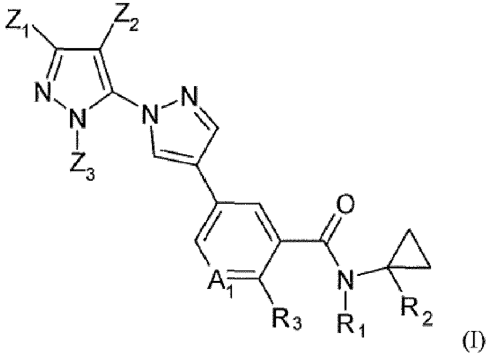 in der
Z₁ 1-halogeniertes Cyclopropyl bedeutet;
Z₂ -S(O)₀₋₂-(C₁-C₂)-Alkyl, (C₁-C₂)-Halogenalkyl oder Nitro (NO₂) bedeutet;
Z₃ (C₁-C₂)-Alkyl bedeutet;
R₁ Wasserstoff (H) oder (C₁-C₂)-Alkyl bedeutet;
R₂ H oder Cyano (CN) bedeutet;
R₃ CH₃ oder Chlor (Cl) bedeutet;
A₁ CH oder Stickstoff (N) bedeutet.

2. Verbindung nach Anspruch 1, wobei A₁ CH bedeutet.

3. Verbindung nach Anspruch 1 oder 2, wobei A₁ CH bedeutet und R₃ Cl bedeutet.

4. Verbindung nach einem der vorhergehenden Ansprüche, wobei
Z₁ 1-Fluorcyclopropyl oder 1-Chlorcyclopropyl bedeutet;
Z₂ -S-CH₃ -SO-CH₃, -SO₂-CH₃, CF₃, CF₂H, Cl, Br, I oder NO₂ bedeutet;
Z₃ CH₃ bedeutet.

5. Verbindung nach einem der vorhergehenden Ansprüche, wobei
Z₁ 1-Fluorcyclopropyl oder 1-Chlorcyclopropyl bedeutet;
Z₂ -S-CH₃, Cl, Br, I, CF₃, CF₂H oder NO₂ bedeutet;
Z₃ CH₃ bedeutet.

6. Verbindung nach einem der vorhergehenden Anspüche, wobei Z, 1-Fluorcyclopropyl oder 1-Chlorcyclopropyl bedeutet;
Z₂ I, CF₃, CF₂H oder NO₂ bedeutet;
Z₃ CH₃ bedeutet.

7. Verbindung nach einem der vorhergehenden Ansprüche, wobei R₁ H bedeutet.

8. Verbindung nach einem der Ansprüche 1 oder 4 bis 7, wobei A₁ N bedeutet.

9. Verbindung nach Anspruch 8, wobei R₃ Cl bedeutet.

10. Verwendung einer Verbindung der allgemeinen Formel (I) nach einem der Ansprüche 1 bis 8 zum Bekämpfen von Insekten, Spinnentieren und Nematoden, wobei Verfahren zur chirurgischen oder therapeutischen Behandlung des menschlichen oder tierischen Körpers und am menschlichen oder tierischen Körper durchgeführte diagnostische Verfahren ausgeschlossen sind.

11. Pharmazeutische Zusammensetzung, umfassend mindestens eine Verbindung nach einem der Ansprüche 1 bis 9.

12. Verbindung nach einem der Ansprüche 1 bis 8 zur Verwendung als Arzneimittel.

13. Verwendung einer Verbindung nach einem der Ansprüche 1 bis 9 zur Herstellung einer pharmazeutischen Zusammensetzung für die Bekämpfung von Parasiten an Tieren.

14. Verbindung, ausgewählt aus der Gruppe bestehend aus

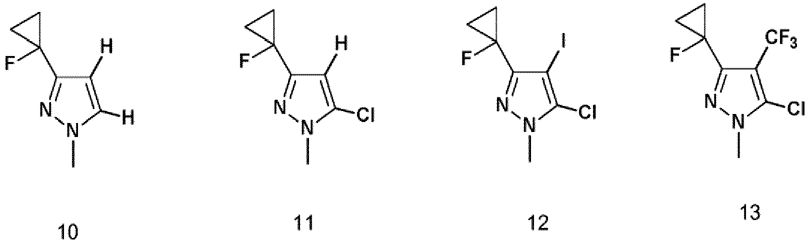

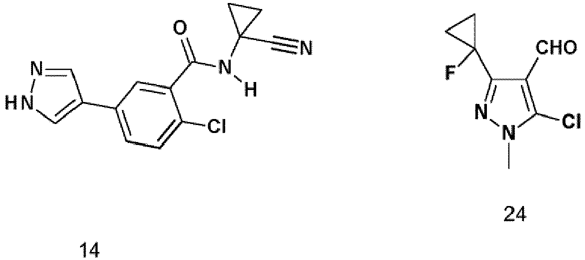

15. Verwendung einer Verbindung nach einem der Ansprüche 1 bis 9 zum Schützen des Vermehrungsguts von Pflanzen.

## Revendications

1. Composés de formule générale (I) 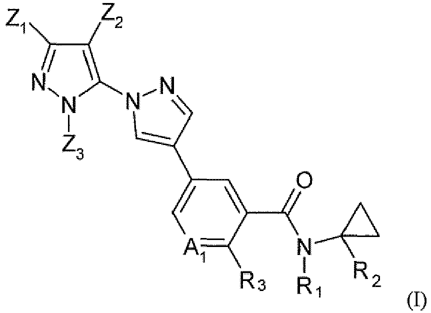 dans lesquels
Z₁ représente cyclopropyle 1-halogéné ;
Z₂ représente -S(O)₀₋₂-(C₁-C₂)-alkyle, (C₁-C₂)-halogénoalkyle, ou nitro (NO₂);
Z₃ représente (C₁-C₂)-alkyle ;
R₁ représente hydrogène (H) ou (C₁-C₂)-alkyle ;
R₂ représente H ou cyano (CN) ;
R₃ représente CH₃ ou chlore (Cl) ;
A₁ représente CH ou azote (N).

2. Composé selon la revendication 1, dans lequel A₁ est CH.

3. Composé selon la revendication 1 ou 2, dans lequel A₁ est CH et R₃ est Cl.

4. Composé selon l'une quelconque des revendications précédentes, dans lequel
Z₁ est 1-fluorocyclopropyle ou 1-chlorocyclopropyle ;
Z₂ est -S-CH₃, -SO-CH₃, SO₂-CH₃, CF₃, CF₂H, Cl, Br, I ou NO₂ ;
Z₃ est CH₃.

5. Composé selon l'une quelconque des revendications précédentes, dans lequel
Z₁ est 1-fluorocyclopropyle ou 1-chlorocyclopropyle;
Z₂ est -S-CH₃, Cl, Br, I, CF₃, CF₂H ou NO₂ ;
Z₃ est CH₃.

6. Composé selon l'une quelconque des revendications précédentes, dans lequel Z, est 1-fluorocyclopropyle ou 1-chlorocyclopropyle;
Z₂ est I, CF₃, CF₂H ou NO₂ ;
Z₃ est CH₃.

7. Composé selon l'une quelconque des revendications précédentes, dans lequel R₁ est H.

8. Composé selon l'une quelconque des revendications 1 ou 4 à 7, dans lequel A₁ est N.

9. Composé selon la revendication 8, dans lequel R₃ est Cl.

10. Utilisation d'un composé de formule générale (I) selon l'une quelconque des revendications 1 à 8, pour le contrôle des insectes, des arachnides et des nématodes, à l'exclusion de méthodes destinées au traitement chirurgical ou thérapeutique du corps humain ou animal et de méthodes de diagnostic pratiquées sur le corps humain ou animal.

11. Composition pharmaceutique comprenant au moins un composé selon l'une quelconque des revendications 1 à 9.

12. Composé selon l'une quelconque des revendications 1 à 8, pour une utilisation comme médicament.

13. Utilisation d'un composé selon l'une quelconque des revendications 1 à 9, pour la préparation d'une composition pharmaceutique destinée au contrôle de parasites chez les animaux.

14. Composé choisi dans le groupe constitué par

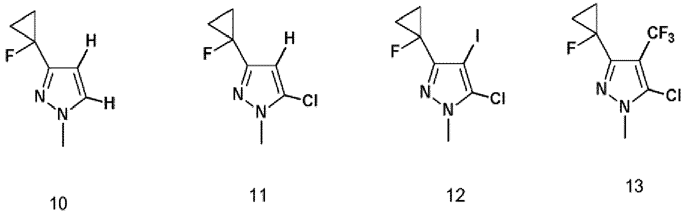

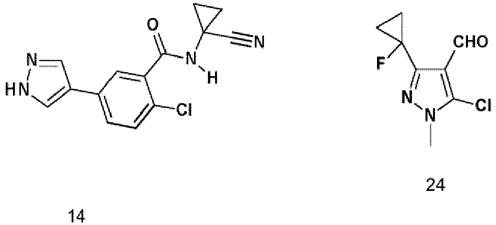

15. Utilisation d'un composé selon l'une quelconque des revendications 1 à 9, pour la protection du matériel de propagation de plantes.
